# EUROPEAN PATENT SPECIFICATION

(11) **EP 2 391 641 B1**
(45) Date of publication and mention of the grant of the patent: **24.12.2014**
(21) Application number: 10703916.6
(22) Date of filing: 02.02.2010
(51) Int. Cl.: C07K 14/335, C12N 1/15, C12N 1/21, C12N 15/31, C07K 16/12, A61K 38/00, A61K 38/16, A61K 39/00, A23L 1/305, A23K 1/16

(54) **Lactobacillus rhamnosus pili and pilus polypeptides**
Lactobacillus rhamnosus Pili und Pilus Polypeptiden
Les pili du Lactobacillus rhamnosus et les polypeptides du pilus

(30) Priority: 02.02.2009 FI 20090031; 02.02.2009 US 364128
(43) Date of publication of application: 07.12.2011
(73) Proprietor: Valio Ltd., 00370 Helsinki (FI)
(72) Inventor: de VOS, Willem Meindert, NL-6717 LM Ede (NL); PALVA, Airi, FI-00780 Helsinki (FI); PALVA, Ilkka, Deceased (FI); REUNANEN, Justus, FI-04230 Kerava (FI); von OSSOWSKI, Ingemar, FI-00790 Helsinki (FI); SATOKARI, Reetta, FI-21610 Kirjala (FI); VESTERLUND, Satu, FI-21110 Naantali (FI); KANKAINEN, Matti, FI-00180 Helsinki (FI); SALUSJÄRVI, Tuomas, FI-02300 Espoo (FI); TYNKKYNEN, Soile, FI-00270 Helsinki (FI)
(74) Representative: Kolster Oy Ab
(86) International application number: PCT/FI2010/050059
(87) International publication number: WO 2010/086512

(56) References cited:
- US-A1- 2004 009 490
- DATABASE UniProt [Online] 14 November 2006 (2006-11-14), "SubName: Full=Uncharacterized protein encoded in toxicity protection region of plasmid R478, contains von Willebrand factor (VWF) domain;" XP002579086 retrieved from EBI accession no. UNIPROT:Q03BW3 Database accession no. Q03BW3
- DATABASE UniProt [Online] 2 September 2008 (2008-09-02), "SubName: Full=Fimbriae subunit;" XP002589126 retrieved from EBI accession no. UNIPROT:B3W7W7 Database accession no. B3W7W7
- DATABASE UniProt [Online] 2 September 2008 (2008-09-02), "SubName: Full=Bee2;" XP002589127 retrieved from EBI accession no. UNIPROT:B3W7W6 Database accession no. B3W7W6
- SCOTT JUNE R ET AL: "Pili with strong attachments: Gram-positive bacteria do it differently" MOLECULAR MICROBIOLOGY, vol. 62, no. 2, October 2006 (2006-10), pages 320-330, XP002579055 ISSN: 0950-382X
- DATABASE UniProt [Online] 4 November 2008 (2008-11-04), "SubName: Full=Sortase (Surface protein transpeptidase);" XP002579056 retrieved from EBI accession no. UNIPROT:B5QRG6 Database accession no. B5QRG6
- MANDLIK ANJALI ET AL: "The molecular switch that activates the cell wall anchoring step of pilus assembly in gram-positive bacteria" PROCEEDINGS OF THE NATIONAL ACADEMY OF SCIENCES OF THE UNITED STATES OF AMERICA, vol. 105, no. 37, September 2008 (2008-09) , pages 14147-14152, XP002579057 ISSN: 0027-8424
- VAN PIJKEREN JAN-PETER ET AL: "Comparative and functional analysis of sortase-dependent proteins in the predicted secretome of Lactobacillus salivarius UCC118." APPLIED AND ENVIRONMENTAL MICROBIOLOGY JUN 2006 LNKD- PUBMED:16751526, vol. 72, no. 6, June 2006 (2006-06), pages 4143-4153, XP002579058 ISSN: 0099-2240
- KANKAINEN MATTI ET AL: "Comparative genomic analysis of Lactobacillus rhamnosus GG reveals pili containing a human- mucus binding protein." PROCEEDINGS OF THE NATIONAL ACADEMY OF SCIENCES OF THE UNITED STATES OF AMERICA 6 OCT 2009 LNKD- PUBMED:19805152, vol. 106, no. 40, 6 October 2009 (2009-10-06), pages 17193-17198 +SUP, XP002579059 ISSN: 1091-6490
- DATABASE UniProt [Online] 13 October 2009 (2009-10-13), "SubName: Full=Pilus specific protein, ancillary protein involved in mucus-adhesion, contains von Willebrand factor (VWF) domain; SubName: Full=Putative cell surface protein;" XP002579087 retrieved from EBI accession no. UNIPROT:C7T9P6 Database accession no. C7T9P6
- DATABASE UniProt [Online] 13 October 2009 (2009-10-13), "SubName: Full=Pilus specific protein, major backbone protein; SubName: Full=Putative uncharacterized protein;" XP002589128 retrieved from EBI accession no. UNIPROT:C7T9P4 Database accession no. C7T9P4
- DATABASE UniProt [Online] 13 October 2009 (2009-10-13), "SubName: Full=Pilus specific protein, minor backbone protein; SubName: Full=Putative uncharacterized protein;" XP002589129 retrieved from EBI accession no. UNIPROT:C7T9P5 Database accession no. C7T9P5
- DATABASE UNIPROT [Online] 01 July 2008 'SubName: Full=Hypothetical cell-surface protein; Flags: Fragment;' Retrieved from EBI, accession no. UNIPROT:B2Y6D7 Database accession no. B2Y6D7

## Description

### Field of the invention

The present invention relates to the fields of life sciences and food, feed or pharmaceutical industry. Specifically, the invention relates to novel peptides, pilus structures, polynucleotides as well as vectors, host cells, products and pharmaceutical compositions comprising the polynucleotides, peptides, or pilus structures. The invention also relates to gene clusters and antibodies. Furthermore, the present invention relates to methods for producing the peptides or pilus structures or producing the products comprising the peptides or pilus structures. Furthermore, the present invention relates to a peptide and/or a pilus structure for reducing or inhibiting the adhesion of pathogenic bacteria, promoting the adhesion of bacterial cells to the mucus and/or epithelium and/or for modifying immune response in a subject.

### Background of the invention

Invasive adherence to host tissues by bacterial pathogens is often facilitated by means of elongated hairlike proteinaceous fibers called pili or fimbriae that protrude outwardly from the microbial cell surface. In Gram-negative pathogenic bacteria the role of pili as colonization agents in pathogenesis is well recognized and the overall mechanism of pilus assembly is clearly defined from over fifty years of research. The most structurally characterized Gram-negative pili are the type I form, found, for example, in the enteropathogenic *E. coli,* and type IV form, found, for example, in species of *Neisseria* and *Pseudomonas* as well as in *E. coli.* Typically, the Gram-negative pili are long (1 to 4 µm in length) and thin (5 to 8 nm in width), and also display both flexible and robust structural properties. These pili are generally comprised of a series of non-covalently linked multiple protein subunits whose assembly is dependent upon specific chaperone proteins, but independent of any enzymatic activity. Frequently, a protein with adhesive properties is positioned at the tip of the pili. It is generally considered that the intervening length of protein subunits from the microbial surface promotes an unhindered contact between the adhesive tip protein and corresponding host cell receptor sites, which are potentially represented by components of the extracellular matrix (ECM) or specific carbohydrate moieties of glycoproteins and glycolipids (Scott J.R. and Zähner D, 2006, Mol Microbiol 62, 320-330; Telford, J.L., et al. 2006, Nat Rev Microbiol 4, 509-519).

The presence of Gram-positive pilus-like structures was actually first observed in the late 1960's by electron microscopy of *Corynebacterium renale* (Yanagawa, R. et al. 1968, Jpn J Vet Res 16, 31-37), and in the subsequent years pili have been found in several other Gram-positive bacterial species, including the very recent discovery of pili in the three main invasive disease-causing streptococcal pathogens in humans, i.e., *Streptococcus pyogenes, Streptococcus agalactiae,* and *Streptococcus pneumoniae* (Telford, J.L., et al. 2006, Nat Rev Microbiol 4, 509-519). The most detailed characterization studies of Gram-positive pili originate from the corynebacteria, streptococci, and bacilli pathogens.

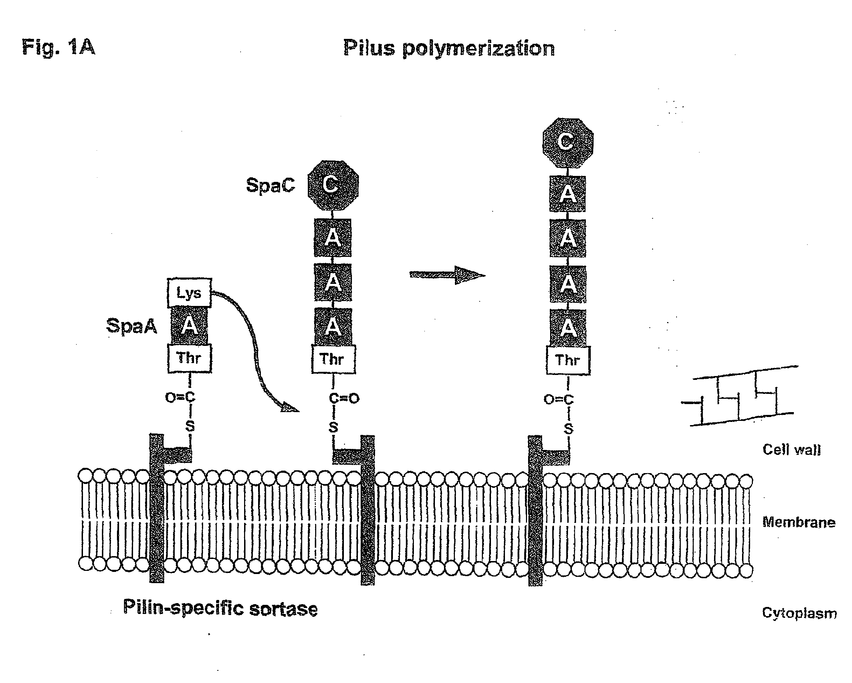

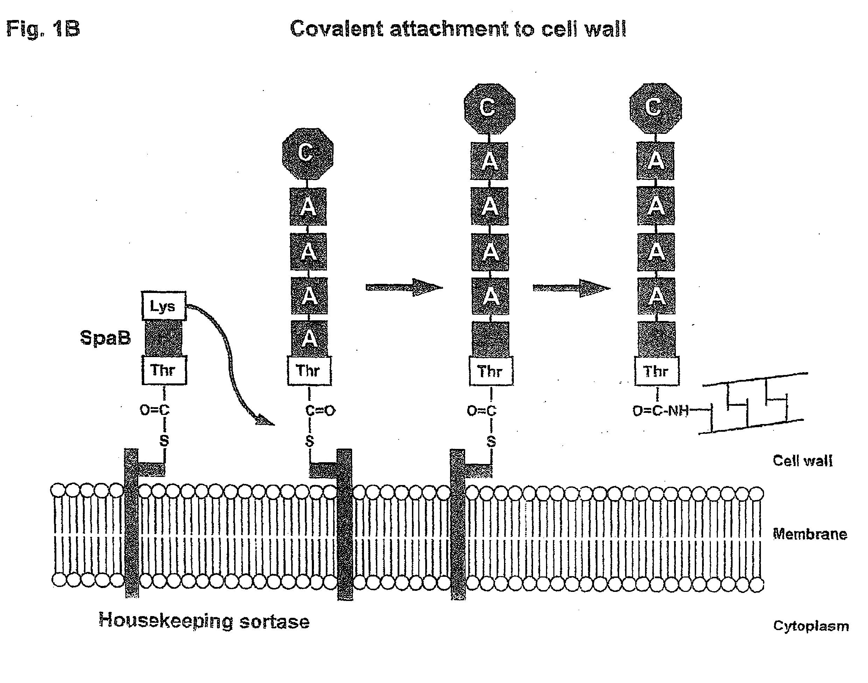

Unlike in the Gram-negative bacteria, the pili in Gram-positive bacteria are much thinner in width (2 to 3 nm) and more difficult to visibly distinguish which also suggests why the presence of these pili may have been overlooked in many species of Gram-positive bacteria (Kang, H.J. et al. 2007, Science 318, 1625-1628). To date, the most thorough description of the pilus-assembly process, that is also generally representative of all Gram-positive pili, has been carried by *in vivo* characterization studies of pili biogenesis in *Corynebacterium diphtheriae* (Ton-That, H. and Schneewind, O. 2004, Trends Microbiol 12, 228-234). Structurally, the prototype pili appear as polymers composed of covalently cross-linked protein subunits (called pilins) that are also covalently anchored at the base to the peptidoglycan component of the cell wall, with both of these covalent bonds being enzymatically dependent upon catalysis by different sortase family membrane-bound transpeptidases, i.e., the pilin-specific and the housekeeping sortases, respectively (Mandlik, A. et al. 2008, Trends Microbiol 16, 33-40). The Gram-positive pilus is typically composed of three pilin subunits and, in the case of C. *diphtheriae*, the proteins (or genes encoding them) are named as SpaA (*spaA*) (sortase-mediated pilin assembly) for the major pilin subunit that exclusively forms the shaft or backbone of the pilus, SpaB (*spaB*) for an ancillary minor pilin subunit, and SpaC (*spaC*) for another minor pilin subunit with adhesive properties located at the tip of the pilus (Figure 1). The genes encoding these three pilin subunits are localized within the same loci as a pilin gene cluster along with at least one gene encoding a pilin-specific sortase in close proximity. As well, the genes within the pilin cluster are frequently flanked on both ends by transposable elements suggesting an origin by horizontal gene transfer. The transcription of all these genes is in the same direction and indicative of operon regulatory control (Scott J.R. and Zähner D, 2006, Mol Microbiol 62, 320-330).

The revised model of the overall Gram-positive pilus assembly process, which is dependent upon several different conserved motifs and domains within the primary sequence of each pilin subunit, includes four basic stages (Mandlik, A. et al. 2008, Proc Natl Acad Sci USA 105, 14147-14152; Telford, J.L., et al. 2006, Nat Rev Microbiol 4, 509-519) (Figure 1). In the first stage, the pilin proteins, each of which contain a N-terminal signal peptide, are secreted through the bacterial cell membrane by the Sec-dependent pathway and then retained in the cell membrane by the presence of a C-terminal membrane-spanning domain consisting of a hydrophobic region of about 20 residues and a positively charged tail.

In the second stage of the assembly process, the cell wall sorting signal (CWSS), preferably the LPXTG-motif, which also immediately precedes the membrane-spanning domain, becomes available for sortase-dependent cleavage of the cell membrane-anchored pilin proteins. The pilin-specific sortase cleaves this five residue motif between the threonine (T) and glycine (G) residues and forms an acyl-enzyme intermediate involving a covalent thioester bond between the carboxyl group of the threonine residue and a cysteinyl thiol found within the catalytic pocket of the sortase.

The third stage represents the polymerization of the pilin subunits by isopeptide bond formation and involves the cleavage of the thioester bond and the release of the sortase from the pilin subunit by the nucleophilic attack of the ε-amino group from the side chain of a lysine (K) residue conserved in the pilin-motif (WXXXVXVYPKN) of a second pilin subunit. An amide bond is thought to form between the C-terminal carboxyl of the threonine residue in the first pilin subunit and the side chain amino group of the pilin-motif lysine from a second pilin subunit still bound as a covalent thioester with an another pilin-specific sortase (Budzik, J.M. et al. 2008, Proc Natl Acad Sci USA 105, 10215-10220). In this model of pilus assembly, the growing polymeric structure is fed by additional pilin subunits at the base of the pilus and the overall length governed by the amount of available pilin subunits associated with pilin-specific sortases. Since the pilin-motif is a characteristic feature of the major (SpaA) and ancillary minor (SpaB) pilin subunits, but missing in the primary sequence of the minor pilin subunits (SpaC) displaying adhesive properties, this pilin subunit is likely located at the tip of the pilus shaft and the first pilin subunit to initiate pilus polymerization.

The attachment of the polymerized pilus to the cell wall represents the fourth stage of the assembly process. Herein, the ancillary minor pilin subunit (SpaB) signals the cessation of pilus polymerization, but only when presented in association with a housekeeping sortase, whose gene is encoded somewhere else on the genome. In this final stage, the growing polymeric structure of major pilin subunits (SpaA) is transferred from a thioester linkage with a pilin-specific sortase to form an amide bond with the side chain of the lysine in the pilin-motif of SpaB minor pilin subunit, which is coupled as a housekeeping sortase acyl-enzyme intermediate. The nucleophilic attack by the amino group of the pentapeptide of the peptidoglycan lipid II precursor then permits the housekeeping sortase to catalyze the attachment of the SpaB pilinlinked pilus polymer to the cell wall. The E-box represents a third and less characterized conserved primary sequence motif (YXLXETXAPXGY) found between the LPXTG- and pilin-motifs of the pilin subunits from many Gram-positive bacteria.

Thus far, three-dimensional (3-D) structure determinations by x-ray crystallography have revealed structural insights into the assembly and function for only two Gram-positive pilin subunit proteins. Krishnan et al. (2007, Structure 15:893-903) had solved the crystal structure for the minor pilin GBS52 of *Streptococcus agalactiae* and revealed the presence of two IgG-like domain folds that share a structural similarity with the *S. aureus* collagen-binding protein Cna which also indicates how this minor pilin subunit could facilitate pilus adherence to a specific host tissue. The crystal structure of the major pilin Spy0128 from *Streptococcus pyogenes,* solved by Kang et al. (2007, Science 318, 1625-1628), had demonstrated how self-generated intramolecular isopeptide bonds between the side chains of lysine and asparagine residues within the pilin subunit could also complement the sortase-catalyzed intermolecular isopeptide bonds for maintaining the overall strength and stability of pili.

The majority of probiotic microbes are members of the Gram-positive lactobacilli and bifidobacteria and have a long tradition of use in fermented foods and dairy products (Goldin, B.R. and Gorbach, S.L. 2008, Clin Infect Dis 46, S96-S100; Ljungh, A. and Wadstrom, T. 2006, Curr Issues Intest Microbiol 7, 73-89; Salminen, S. et al. 1998, Br J Nutr 80, S147-S171). Pilus structures of probiotic lactobacilli or genes encoding these pilus structures have not been described in the literature. The presence of pilus-like structures or polynucleotides has never been shown in *Lactobacillus rhamnosus.*

### Brief description of the invention

The object of the present invention is to provide novel pilus polypeptides as well as polynucleotides encoding them. Furthermore, the object of the invention is to provide novel pilus structures. Still, the object of the invention is to provide novel methods, uses and products related to the above-mentioned peptides, polypeptides, proteins, pilus structures, and polynucleotides.

The present application introduces peptides comprising a sequence having at least 94% sequence identity with SEQ ID NO 1 (GG00441), at least 94% sequence identity with SEQ ID NO 2 (GG00442), at least 84% sequence identity with SEQ ID NO 3 (GG00443), at least 91% sequence identity with SEQ ID NO 4 (GG00444), at least 83% sequence identity with SEQ ID NO 5 (GG02369), at least 94% sequence identity with SEQ ID NO 6 (GG02370), at least 93% sequence identity with SEQ ID NO 7 (GG02371), or at least 93% sequence identity with SEQ ID NO 8 (GG02372), or fragments or variants thereof. The present invention relates to an isolated polypeptide comprising a sequence having at least 95% identity with SEQ ID NO 4 (GG00444).

The present application also introduces polynucleotides encoding for a peptide sequence having at least 94% sequence identity with SEQ ID NO 1 (GG00441), at least 94% sequence identity with SEQ ID NO 2 (GG00442), at least 84% sequence identity with SEQ ID NO 3 (GG00443), at least 91% sequence identity with SEQ ID NO 4 (GG00444), at least 83% sequence identity with SEQ ID NO 5 (GG02369), at least 94% sequence identity with SEQ ID NO 6 (GG02370), at least 93% sequence identity with SEQ ID NO 7 (GG02371) or at least 93% sequence identity with SEQ ID NO 8 (GG02372), or with fragments or variants thereof. The present invention relates to a polynucleotide comprising a sequence of SEQ ID NO 12 or encoding the peptide of the invention.

The present application also introduces a pilus structure comprising at least one of the peptides of the invention, a product comprising at least one peptide or pilus structure of the invention and to a pharmaceutical or nutritional composition comprising at least one peptide or pilus structure of the invention. The present invention relates to an isolated pilus structure comprising the peptide of the invention.

Furthermore, the present invention relates to a product comprising the peptide or the pilus structure of the invention or a product comprising the peptide or the pilus structure of the invention for use as a medicament or for the prevention or treatment of diarrhea, arterial hypertension, vascular diseases, allergies, cancer, atopic diseases, viral diseases, infectious diseases, urinary tract infections, respiratory infections, dental caries, irritable bowel syndrome (IBS), inflammatory bowel disease (IBD), mucosal inflammation, gut permeability disorders, obesity, metabolic syndrome, oxidative stress or abdominal pain.

Furthermore, the peptide or pilus structure of the invention can be used in the manufacture of a medicament for treating or preventing diarrhea, arterial hypertension, vascular diseases, allergies, cancer, atopic diseases, viral diseases, infectious diseases, urinary tract infections, respiratory infections, dental caries, IBS, IBD, mucosal inflammation, gut permeability disorders, obesity, metabolic syndrome, oxidative stress or abdominal pain.

Furthermore, the peptide or pilus structure of the invention can be used for the treatment or prevention of diarrhea, arterial hypertension, vascular diseases, allergies, cancer, atopic diseases, viral diseases, infectious diseases, urinary tract infections, respiratory infections, dental caries, IBS, IBD, mucosal inflammation, gut permeability disorders, obesity, metabolic syndrome, oxidative stress or abdominal pain.

Still, the present application introducer a polynucleotide comprising a sequence of any one of SEQ ID NOs 9-16 or a degenerate thereof, or encoding a peptide of the application. The present invention relates to a vector comprising the polynucleotide of the invention, to a host cell comprising the polynucleotide or the peptide of the invention, and to a gene cluster comprising the polynucleotide of the invention.

Also, the present invention relates to an antibody against the peptide of the invention or against its functional domains.

The present application also introduces a method of treating or preventing diarrhea, arterial hypertension, vascular diseases, allergies, cancer, atopic diseases, viral diseases, infectious diseases, urinary tract infections, respiratory infections, dental caries, IBS, IBD, mucosal inflammation, gut permeability disorders, obesity, metabolic syndrome, oxidative stress or abdominal pain comprising administration of at least one peptide or pilus structure of the invention to a subject.

The present application introduces a method for screening of bacterial strains, which comprise at least one polynucleotide of the invention or a fragment thereof, wherein the method comprises:
i) providing DNA or RNA from bacterial strains;
ii) hybridizing primers or probes specific to the polynucleotide of the invention or a fragment thereof with DNA or RNA from step i) and optionally amplifying the polynucleotide or the fragment thereof;
iii) detecting at least one polynucleotide or a fragment thereof homologous to the polynucleotide of the invention or the fragment thereof.

At least one polynucleotide of the invention or fragment thereof or at least one antibody of the invention can be used for screening of bacterial strains.

The present application introduces a method of screening bacterial strains, which comprise at least one peptide or pilus structure of the invention, using at least one antibody of the invention, wherein the method comprises:
i) providing proteins of bacterial strains;
ii) detecting at least one polypeptide, pilus structure or a fragment thereof using the antibody/antibodies.

The present application introduces a method of reducing or inhibiting the adhesion of pathogenic bacteria to the gastrointestinal tract, to the epithelium or to the mucus of a subject, wherein the method comprises administering at least one peptide and/or pilus structure of the invention to the subject.

At least one peptide and/or pilus structure of the invention can be used for reducing or inhibiting the adhesion of pathogenic bacteria to the gastrointestinal tract, to the epithelium or to the mucus of a subject.

The present invention relates to the peptide or pilus structure of the invention for reducing or inhibiting the adhesion of pathogenic bacteria to the gastrointestinal tract, to the epithelium or to the mucus of a subject.

The present application introduces a method of promoting the adhesion of a bacterial cell or the adhesion of any other agent to the mucus or epithelium, wherein the method comprises:
i) producing at least one peptide or pilus structure of the invention or a fragment thereof;
ii) displaying the peptide, pilus structure and/or fragment thereof on the bacterial cell or on any other agent;
iii) bringing the bacterial cells or any other agent into contact with the mucus or epithelium.

The present application introduces a use of at least one peptide or pilus structure of the invention for promoting the adhesion of a bacterial cells or the adhesion of any other agent to the mucus or epithelium.

The present invention relates to the peptide or pilus structure of the invention for promoting the adhesion of a bacterial cells or the adhesion of any other agent to the mucus or epithelium.

The present application introduces a method of modifying immune response in a subject, wherein the methods comprise:
i) producing at least one peptide or pilus structure of the invention or a fragment thereof;
ii) displaying the peptide, pilus structure and/or fragment thereof on a host cell;
iii) optionally bringing the host cell into contact with the mucus or another host cell.

The present application introduces a use of at least one peptide or pilus structure of the invention for modifying immune responses.

The present invention relates to the peptide or pilus structure of the invention for modifying immune response.

The present invention relates to a method of producing a product of the invention, wherein the method comprises a step of adding the peptide or pilus structure of the invention to the product.

The present invention also relates to a method of producing the peptide of the invention, wherein the method comprises the following steps:
i) providing at least one polynucleotide of the invention;
ii) transforming a host cell with the polynucleotide;
iii) culturing the host cell from step ii) to produce the peptide;
iv) optionally recovering the peptide.

In addition, the present invention relates to a method of producing the peptide or pilus structure of the invention, wherein the method comprises the following steps:
i) disrupting a cell producing or comprising the peptide or pilus structure of the invention;
ii) optionally, recovering the peptide or pilus structure.

Also, the present invention relates to a method of producing the peptide of the invention, wherein the method comprises the following steps:
i) providing amino acids;
ii) manufacturing the peptide of the invention from the amino acids of step i) with synthetizing the peptide.

The present application introduces a method of detecting potential probiotic bacterial strains by using bioinformatic approaches, wherein the method comprises the following steps:
i) providing a sequence of at least one peptide, polynucleotide or fragment thereof;
ii) comparing the sequence of step i) against sequences of sequence collections;
iii) detecting sequences having biologically congruent fragments to sequences of step i) or having high identity to the sequence of step i).

The present application also introduces a method of detecting pathogen strains, against which the peptides or pilus structures of the invention are effective, by using bioinformatic approaches, wherein the method comprises:
i) providing a sequence of at least one peptide, polynucleotide of fragment thereof;
ii) comparing the sequence of step i) against sequences of sequence collections;
iii) detecting sequences having biologically congruent fragments to the sequence of step I) or having high identity to the sequence of step i).

The peptides, pilus structures and polynucleotides of the invention provide tools for further developments in food, feed, cosmetics and pharmaceutical industries. The present invention enables rapid and efficient screening methods and reliable and accurate, either qualitative or quantitative analysis of a multitude of bacterial strains. Therefore, the methods and means of the invention enable the discovery of novel probiotic bacterial strains as well as discoveries of new products (incl. ingredients, supplements, and nutritional products), medicaments and therapeutic methods. Furthermore, by the present invention more effective and specific treatments become available.

There is a continued, evident need to offer the consumers new products having clearly demonstrated effects on health and produced in a form that allows them to be used as such or as a part of another product, such as a pharmaceutical or a food or feed product. In accordance with the present invention, products are also applicable as capsules, pills or tablets that allow the use as convenient part or supplement, for example, of the every-day diet or medication.

### Brief description of the figures

Figures 1 a and 1b show the models of pilus assembly and covalent attachment to the cell wall in Gram-positive Corynebacteria.

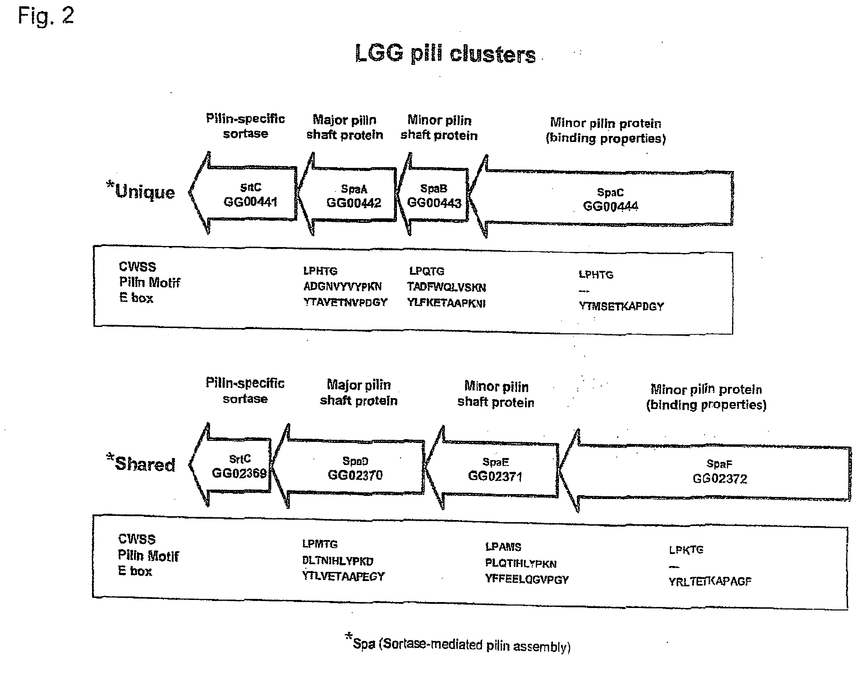

Figure 2 shows the *Lactobacillus rhamnosus* GG (LGG) pili clusters including genes encoding pilin-specific sortases, major pilus shaft protein, minor pilus shaft protein and capping pilus proteins. CWSS indicates a cell wall sorting signal, i.e. a conserved motif found in many Gram-positive bacteria, Pilin Motif and E-box also indicate conserved motifs found in many Gram-positive bacteria.

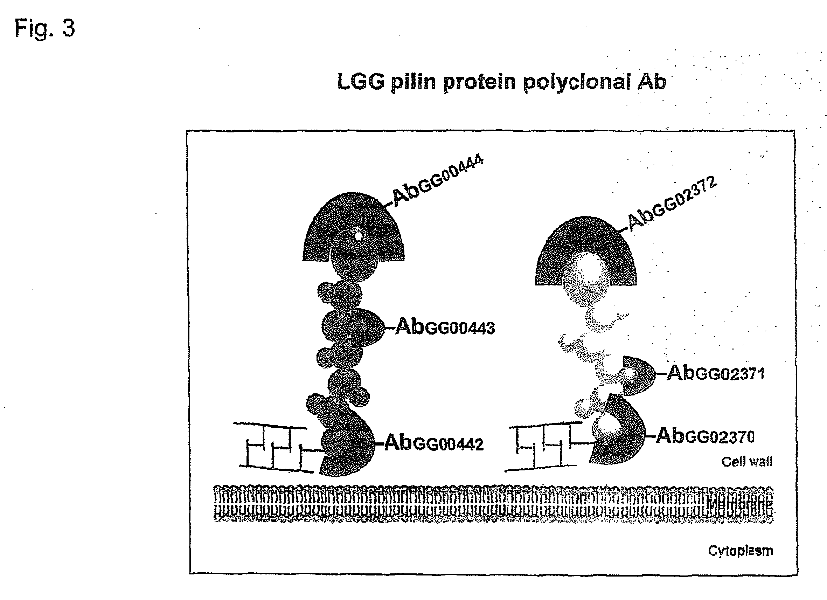

Figure 3 shows examples of polyclonal antibodies binding to peptides GG00442, GG00443, GG00444, GG02370, GG02371 and GG02372 of the LGG pilus structure.

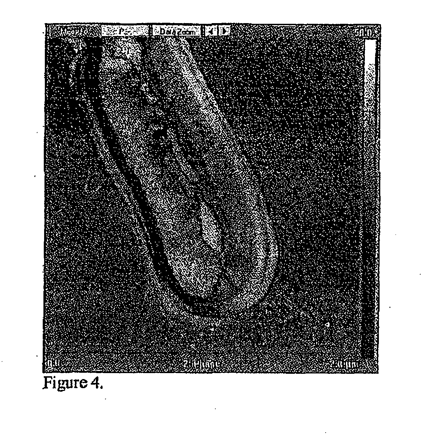

Figure 4 shows a phase contrast Atomic Force Microscope micrograph picture of protruding pili structures of LGG.

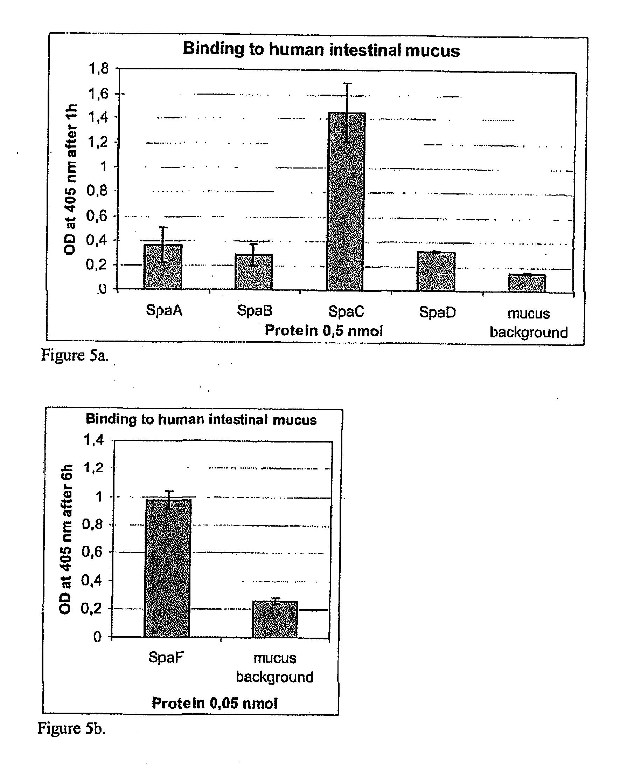

Figures 5a and 5b show the *in vitro* binding of recombinant histidine-tagged LGG proteins, i.e. SpaA, SpaB, SpaC, SpaD and SpaF pilin proteins, to human intestinal mucus. Resected human intestinal tissue was used as a source of mucus on a polystyrene microtiter plate. The bound proteins were detected by enzyme-linked immunosorbent assay.

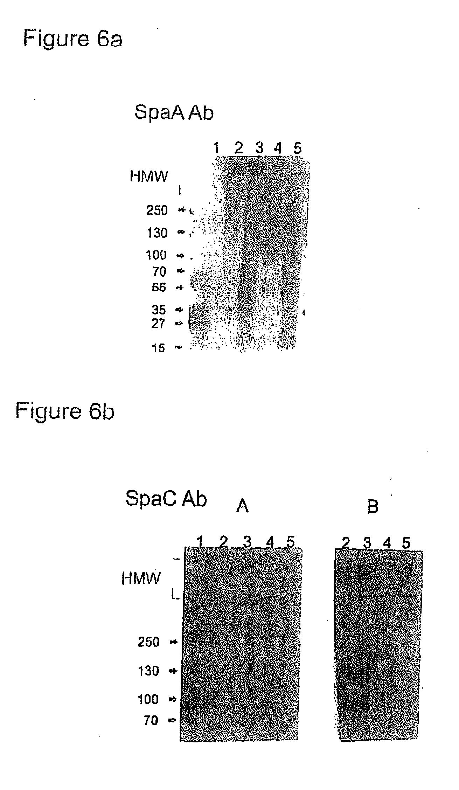

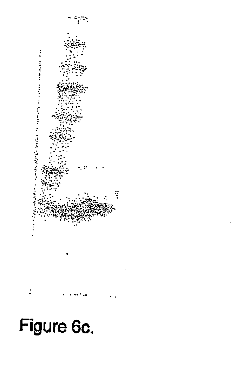

Figures 6a and 6b show Western blots of cell wall fractions of LGG and as a negative control *L. rhamnosus* LC705 (LC705) grown in mTSB-medium or MRS+ 0.6% ox gall bile medium using SpaA and SpaC pilin protein-specific polyclonal antibodies, respectively. Figure 6a shows the presence of SpaA-containing pili and SpaA monomers in LGG and Figure 6b shows the presence of SpaC-containing pili and SpaC monomers in LGG. Lane 1: recombinant SpaA/SpaC pilin protein; Lane 2: LGG grown in mTSB; Lane 3: LGG grown in MRS+ 0.6% ox gall bile; Lane 4: LC705 grown in mTSB, Lane 5: LC705 grown in MRS+0.6% ox gall bile. The antibody used is indicated on a top of each picture. In figure 6b, Panel A: lanes 1 to 5 are exposed for 1 second; Panel B: lanes 2-5 are exposed separately for 60 seconds. The positions of the molecular weight standards are indicated on the left as kilodaltons. HMW indicates high molecular weight ladder. Figure 6c shows the presence of SpaB-containing pili and SpaB monomers in Western blots of cell wall fractions of LGG Lane 1: molecular weight marker; Lane 2: LGG grown in MRS. Detection was performed with SpaB pilin protein-specific polyclonal antibodies, Goat anti-rabbit IgG- AP conjugate (Bio Rad) and BCIP/NBT color reagent.

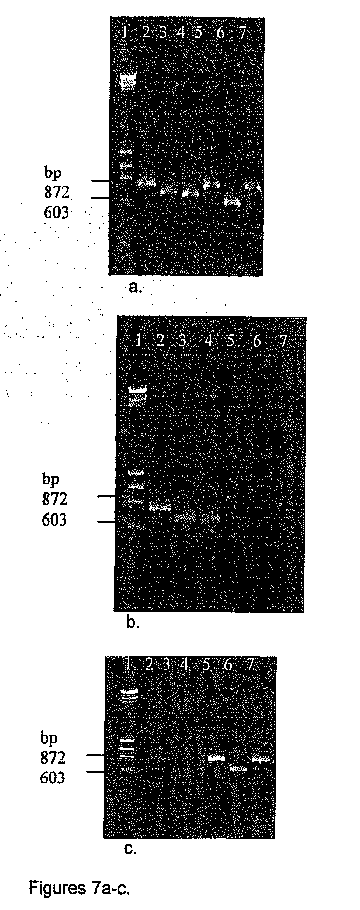

Figures 7a-c show the results of PCR screening of new probiotic strains having pili structures. Figures 7a-c show the amplification products of *Lactobacillus rhamnosus* GG, *Lactobacillus rhamnosus* LC705 and *Lactobacillus casei* ATCC 334, respectively. Lanes 1: molecular weight marker; Lanes 2: amplification product with primers for SpaF; Lanes 3: amplification product with primers for SpaE; Lanes 4. amplification product with primers for SpaD; Lanes 5: amplification product with primers for SpaC; Lanes 6: amplification product with primers for SpaB; Lanes 7: amplification product with primers for SpaA.

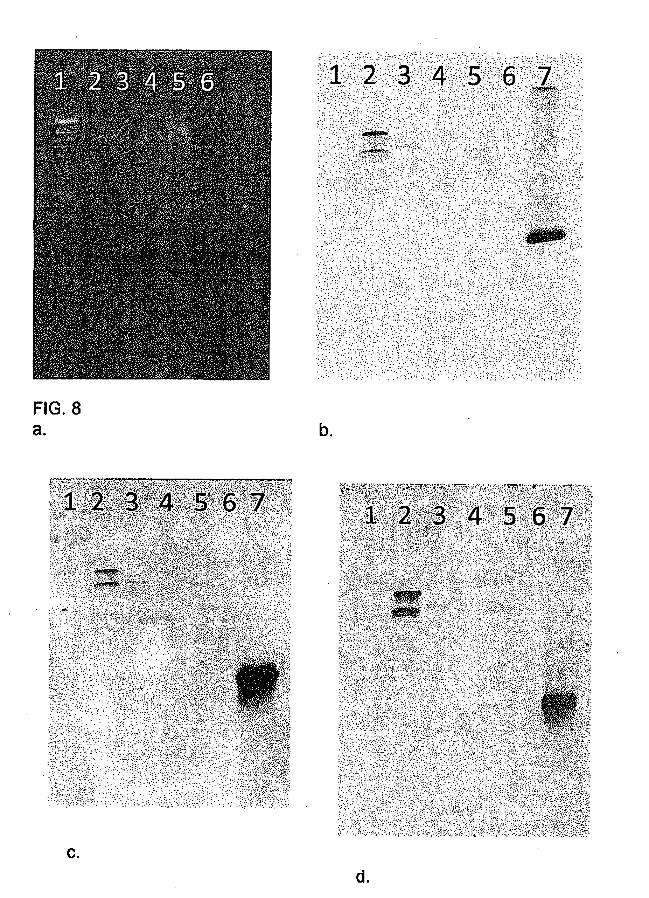

Figures 8a-d show Southern hybridization signals indicating the presence of *spaC, spaB* or *spaA* in *L. casei* ATCC 334, *L. rhamnosus* LC705 and *L. rhamnosus* GG. Figure 8a shows digested genomic DNAs separated by agarose gel electrophoresis and Figures 8b,8c and 8d show Southern hybridization of the same DNAs using DIG-labeled 801-bp PCR amplification product of *Lactobacillus rhamnosus* GG *spaC* gene, 612-bp PCR amplification product of *Lactobacillus rhamnosus* GG *spaB* gene or 780-bp PCR amplification product of *Lactobacillus rhamnosus* GG *spaA* gene as a probe, respectively. Lane 1: Molecular weight marker I *Hind*III- φX174 *Hae*III mix; Lane 2: DIG labeled molecular weight marker II (Roche); Lane 3: *Lactobacillus casei* ATCC 334 digested with *Hind*III; Lane 4: *Lactobacillus rhamnosus* LC705 digested with *Hind*III; Lane 5: *Lactobacillus rhamnosus* GG digested with *Hind*III; Lane 6: - ; Lane 7: Unlabeled probe.

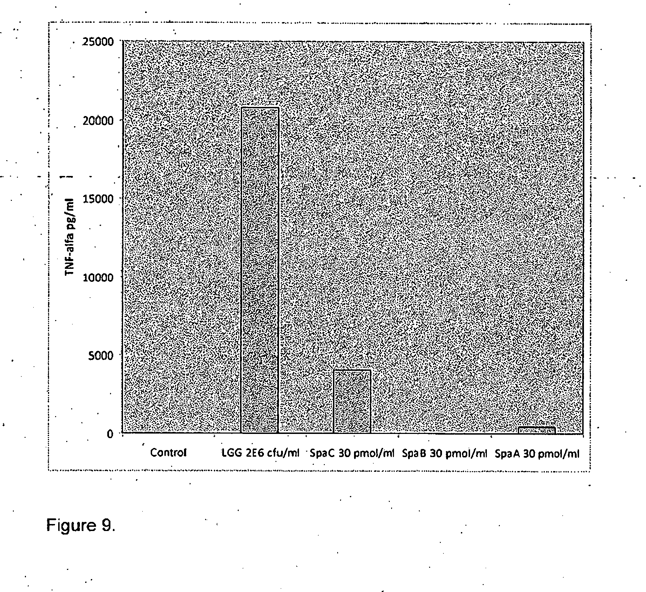

Figure 9 shows TNF-α levels during macrophage stimulation with live *Lactobacillus rhamnosus* GG 2x10⁶ cfu/ml or with purified His-Tag labelled *Lactobacillus rhamnosus* GG proteins SpaA, SpaB and SpaC approximately 30 pmol/ml.

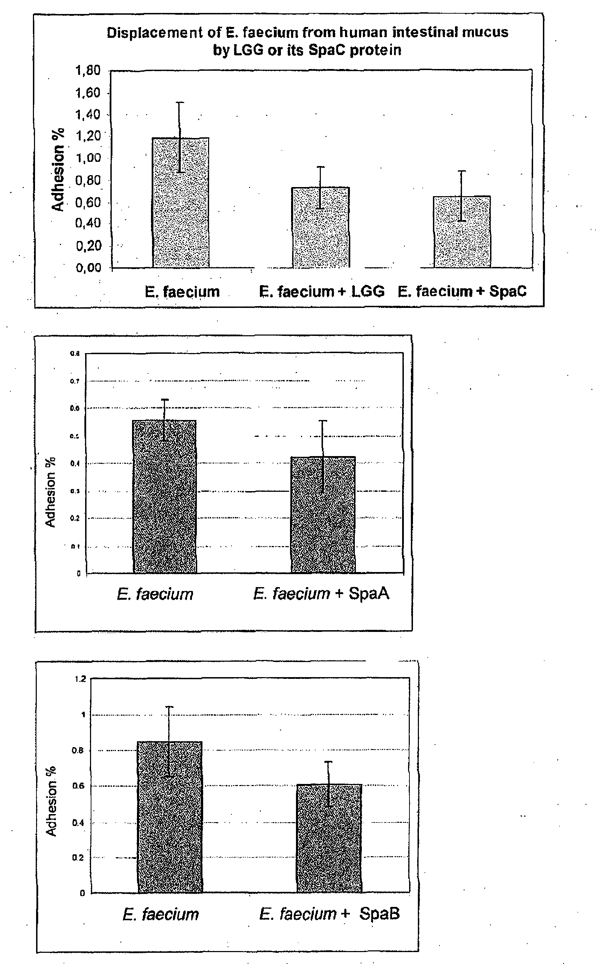

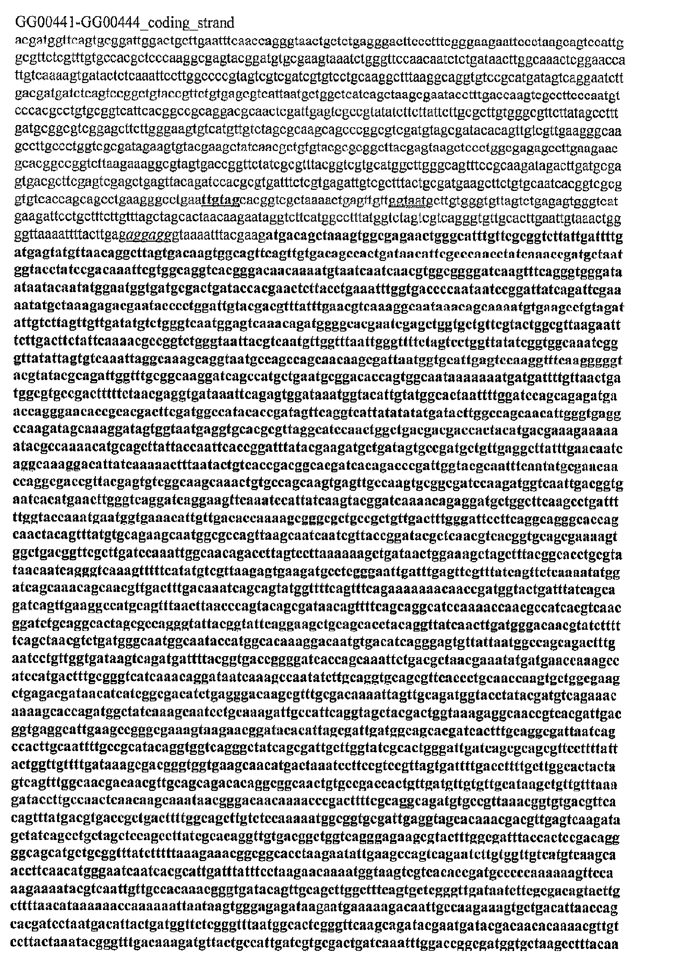

Figure 10 shows displacement of pathogenic bacterium *Enterococcus faecium* from the human intestinal mucus by *Lactobacillus rhamnosus* GG or SpaC, SpaB and SpaA pilin proteins. Adhesion (%) is demonstrated as a mean ± S.D. of five parallel experiments. * refers to significantly reduced adhesion of *E. faecium* (P<0.05).

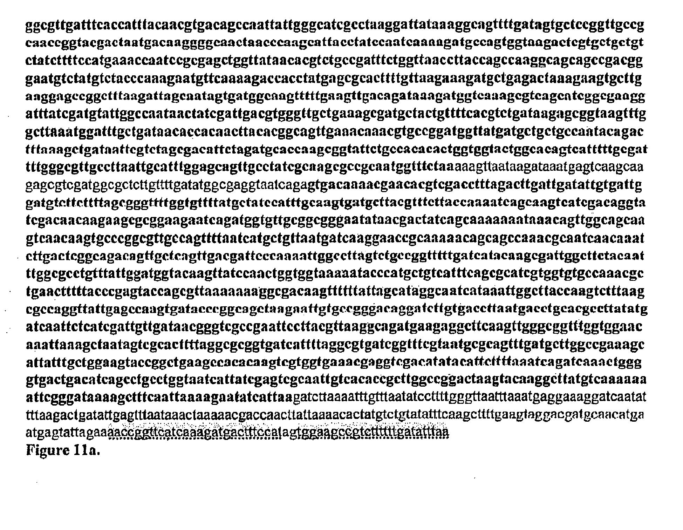

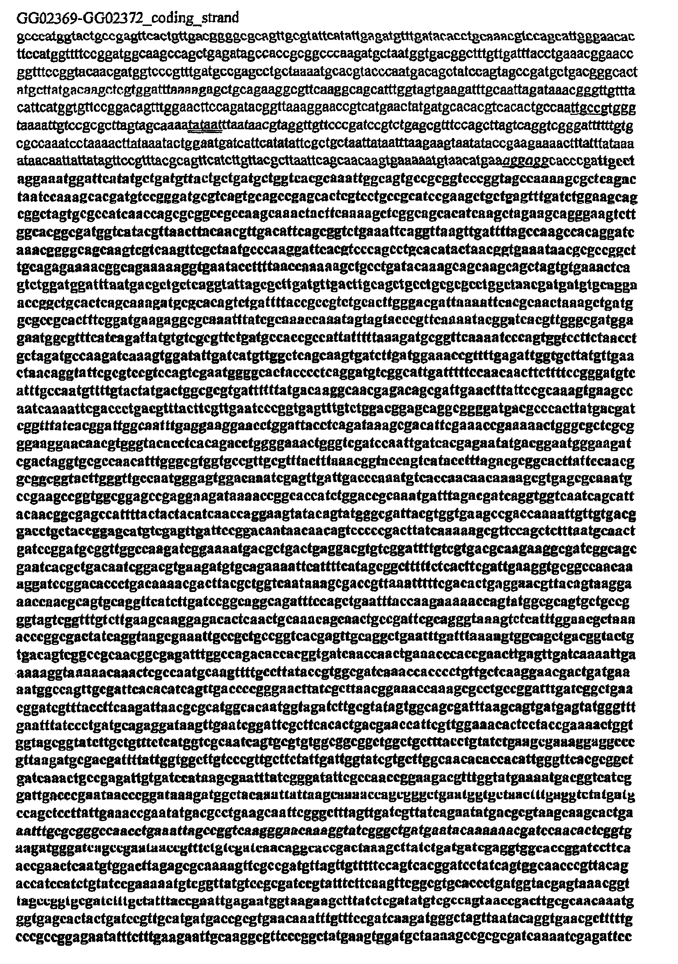

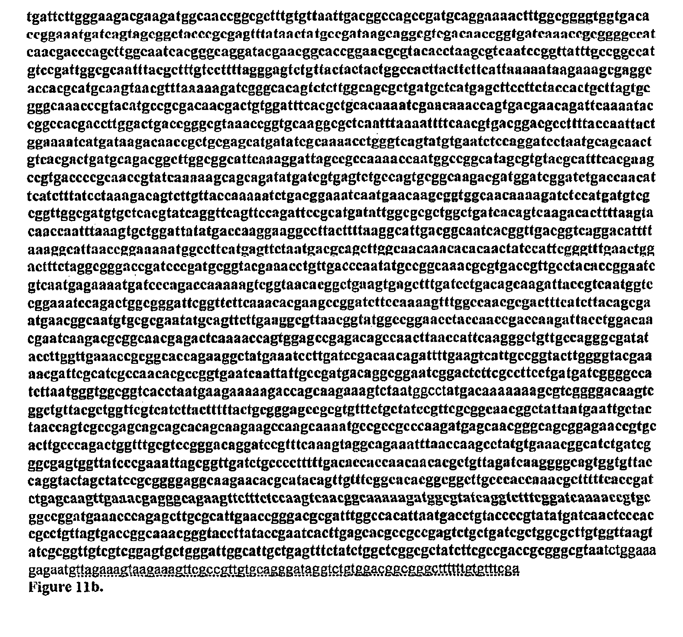

Figures 11 a and 11 b show nucleotide sequences encoding the pili operons presented in Figure 2. Figure 11 a shows the operon encoding GG00441 - GG00444 genes (bold). The putative conserved elements -35 sequence (underlined), -10 sequence (double underlined), ribosomal binding site (underlined italics) and rho terminator (dotted underline). Figure 11b shows the operon encoding GG02369 - GG02372 genes (bold). The putative conserved elements -35 sequence (underlined), -10 sequence (double underlined), ribosomal binding site (underlined italics) and rho terminator (dotted underline).

### Detailed description of the invention

Lactic acid bacteria have been utilized in food industry for a long time and today they are used in various food supplies such as milk products. For example lactobacilli and bifidobacteria are known to have probiotic effects, but the ways by which probiotic bacteria affect the health are not fully understood. Therefore, further investigations of probiotics are warranted.

This invention resides in the finding that also Gram-positive bacteria have pilus structures. Furthermore, the invention resides in the finding of novel pilus peptides and structures in Gram-positive bacteria, specifically in lactobacilli, more specifically in *Lactobacillus rhamnosus.*

### Peptides of the pilus structure

Generally a Gram-positive bacterial pilus extends out from the outer membrane of the bacteria, usually being 1-4 µm long and 2-8 nm wide and appearing in low numbers. Pili are considered to promote adherence of the bacteria to target surfaces. Indeed, as used herein, the expression "pilus structure" refers to an elongated hair or hairlike proteinaceous fiber, comprising multiple protein subunits (preferably more than one subunits). The assembly of these proteins may be dependent on specific proteins, i.e. sortases. A protein having adhesive properties is usually located at the top of the pili. Also the other proteins of the heteromeric pilus structure may be adhesive. As used herein, the expression "part of a pilus structure" refers to any component of a pilus, preferably any protein or any fragment or any variant of the pilus. In a preferred embodiment of the invention, the pilus structure is located on the surface of a microorganism or originates therefrom.

As used herein, the expression "peptide" refers to any peptide, such as a dipeptide, polypeptide, protein and/or pilin protein.

In a specific embodiment of the invention, characteristic features of the pilin are major (SpaA), ancillary minor (SpaB) and capping (SpaC) pilin subunits.

Pilin specific sortases act by transferring SpaA to SpaC in a growing polymeric structure of pilin (Figure 1). In a preferred embodiment of the invention, the peptide comprising a sequence having at least 94% sequence identity with SEQ ID NO 1 (GG00441) or a sequence having at least 83% sequence identity with SEQ ID NO 5 (GG002369) is a pilin specific sortase (Figure 2, see also Figures 11 for nucleotide sequences encoding the pili operons presented in Figure 2).

SpaA likely forms a back-bone of the pilus structure. The length of the different pilus structures depends on the amount of SpaA in the back-bone (Figure 1). In a preferred embodiment of the invention, the peptide comprising a sequence having at least 94% sequence identity with SEQ ID NO 2 (GG00442) or a sequence having at least 94% sequence identity with SEQ ID NO 6 (GG002370) is a major pilus shaft protein, i.e. a major pilin subunit (Figure 2, see also Figures 11 for nucleotide sequences encoding the pili operons presented in Figure 2). GG00442 and GG02370 contain the sortase-recognition site, thus being substrates of the sortases.

SpaB is likely added to the pilus structure at the latest state (terminal stage) of the pilus formation and it forms a link of the pilus to the cell wall (Figure 1). In a preferred embodiment of the invention, the peptide comprising a sequence having at least 84% sequence identity with SEQ ID NO 3 (GG00443) or a sequence having at least 93% sequence identity with SEQ ID NO 7 (GG002371) is a minor pilus shaft protein (Figure 2, see also Figures 11 for nucleotide sequences encoding the pili operons presented in Figure 2). GG00443 and GG002371 contain the sortase-recognition site, thus being substrates of the sortases.

SpaC is likely located at the tip of the pilus shaft and the first pilin subunit to initiate pilus polymerization (Figure 1). In a preferred embodiment of the invention, the peptide comprising a sequence having at least 91% sequence identity with SEQ ID NO 4 (GG00444) or a sequence having at least 93% sequence identity with SEQ ID NO 8 (GG002372) is a binding pilus protein (Figure 2, see also Figures 11 for nucleotide sequences encoding the pili operons presented in Figure 2). GG00444 protein contains a von Willebrand factor (vWF) domain, and GG00444 and GG02372 contain the sortase-recognition sites, thus being substrates of the sortases.

In a specific embodiment of the disclosure, the peptide or polypeptide of the disclosure comprises a sequence having at least 60, 65, 70, 75, 80, 81, 82, 83, 84, 85, 86, 87, 88, 89, 90, 91, 92, 93, 94, 95, 96, 97, 98, 99, 99.5, 99.8, 99.9 or 100% identity to amino acid sequence of SEQ ID NO 1, 2, 3, 4, 5, 6, 7 or 8, or to fragments or variants thereof.

According to a specific embodiment of the disclosure, the peptide has at least 60, 65, 70, 75, 80, 81, 82, 83, 84, 85, 86, 87, 88, 89, 90, 91, 92, 93, 94, 95, 96, 97, 98, 99, 99.5, 99.8, 99.9 or 100% identity to any one of the amino acid sequences of SEQ ID NO 1, 2, 3, 4, 5, 6, 7 or 8, or to fragments or variants thereof.

In another specific embodiment of the disclosure the peptide has a sequence shown in any one of the sequences SEQ ID NO 1, 2, 3; 4, 5, 6, 7 or 8, or fragments or variants thereof.

Identity of any sequence or fragments thereof compared to the sequence of this invention refers to the identity of any sequence compared to the entire sequence of the present invention. Sequence identity may be determined for example by using BLAST (Basic Local Alignment Search Tools) or FASTA (FAST-All). In the searches, setting parameters "gap penalties" and "matrix" are typically selected as default.

As used herein, a fragment or variant of a peptide refers to any part or variant of a peptide, which may have the biological function. A variant refers to a peptide having small alterations in the peptide sequence, e.g. small deletions, mutations or insertions. As used herein "a functional fragment or variant of a peptide" refers for example to a fragment or variant capable of forming the isopeptide bonds between the different pilin subunits (e.g., the transpeptidase activity of a pilin specific sortase), being a substrate of sortases (e.g., function of SpaA, SpaB, SpaC, SpaD, SpaE and SpaF) or binding to the cell wall (e.g., function of SpaC and SpaF) or to other proteins or fragments (e.g., function of SpaC). Also, "a functional fragment or variant of a peptide" may refer to any fragment or variant comprising a biological domain.

Today, domains within polypeptides are usually defined as spatially distinct structures that could conceivably fold and function in isolation (Ponting C. P. and Russell R. R. 2002, Annu Rev Biophys Biomol Struct. 31:45-71).

Based on the analysis of polypeptide sequence of, for example, SEQ ID NO 4 using the tool of the popular domain- database the PFAM (Finn, R. D. et al. 2009, Nucleic Acids Res. Nov 17. [Epub ahead of print]), the polypeptide sequence of SEQ ID NO 4 was characterized to have three domains with a significant E-value, less than or equal to 0.1, in this study.

Amino acids from 137 to 271 of SEQ ID NO 4 form the first domain (PF00092 - von Willebrand factor type A domain) that is found also in many proteins mediating and involved in protein-protein interactions (Colombatti, A. et al. 1993, Matrix. 13:297-306; Whittaker C. A. and Hynes R. O. 2002, Mol Biol Cell. 13:3369-87; Konto-Ghiorghi, Y et al. 2009, PLoS Pathog. 5:e1000422). The von Willebrand factor type A domain -containing proteins function often in multiprotein complexes and have been documented to participate in numerous biological events in eukaryotes including membrane transport, the proteasome, transcription, DNA repair, cell adhesion, migration, homing, pattern formation, and signal transduction (Colombatti, A. et al. Matrix. 13:297-306). More recently, the von Willebrand factor type A domain of the pilin of the pathogenic *Streptococcus agalactiae* strain NEM316 was shown to be essential for the adhesive function of this protein to human epithelial cells (Konto-Ghiorghi, Y. et al. 2009, PLoS Pathog. 5:e1000422).

Amino acids from 617 to 691 form the second domain (PF05738 - Cna protein B-type domain) and amino acids from 749 to 821 form the third domain (PF05738 - Cna protein B-type domain) of SEQ ID NO 4. The Cna protein B-type domain has been documented to share an important role in *Staphylococcus aureus* collagen-binding surface protein. However, this region does not mediate collage binding, but in stead, serves as a 'stalk' that projects the other regions of the protein from the bacterial surface and thus facilitates bacterial adherence to collagen (Deivanayagam, C. C. et al. 2000, Structure. 8:67-78). Recently, another property was suggested for Cna protein B-type domain as it was noted to contain isopeptide bond-forming residues, shown to be important for pilus-assembly and for linking the different pilin-subunits together (Kang, H. J. et al. 2007, Science 318, 1625-1628). The sequence identity percentage of a fragment can be measured as is done for the entire polypeptide, using identical tools and settings.

Any polypeptide sequence of the invention can be analysed similar to SEQ ID NO 4 as described above. Cna protein B-type domains are found in SEQ ID NO 2 (amino acids 73-155 and 193-295), in SEQ ID NO 3 (amino acids 72-164), in SEQ ID NO 6 (amino acids 374-470 and 69-168), in SEQ ID NO 7 (amino acids 238-328) and in SEQ ID NO 8 (amino acids 743-805 and 839-906).

Based on current knowledge, polypeptides with a high enough identity percentage share similar spatial structures and are likely to be involved in similar, although not necessarily in identical, functions. The sequence level identity percentage for functional and structural equivalence is a function of alignment length (Sander C. and Schneider R. 1991, Proteins. 9:56-68). For example, polypeptides having at least 50 amino acids have a good structural similarity when their sequence level identity percentage exceeds 35%. Thus, current knowledge says that polypeptide sequences with sequence identity percentage over 35% and length over 50 amino acids can be involved in similar function, have similar spatial structure and be detected using the same antibody-related screening method.

In a preferred embodiment of the invention, a peptide having SEQ ID NO 2-4 or 6-8 is a part of a pilus structure. In another preferred embodiment of the invention, the pilus structure of the invention comprises at least one of the peptides of the invention, more preferably at least two, or at least three peptides of the invention. Furthermore, in a preferred embodiment of the invention, the pilus structure comprises peptides GG00442 (SEQ ID NO 2), GG00443 (SEQ ID NO 3) and GG00444 (SEQ ID NO 4) and/or peptides GG02370 (SEQ ID NO 6), GG02371 (SEQ ID NO 7) and GG02372 (SEQ ID NO 8).

### Gram-positive and probiotic bacteria

The peptides or pilus structures of the invention can be from any bacteria, such as Gram-positive or Gram-negative bacteria. However, in a preferred embodiment of the invention, the peptides or pilus structures are from gram-positive bacteria. Gram-positive bacteria, which may comprise the peptides or pilus structures of the invention, include but are not limited to lactobacilli, lactococci, bifidobacteria, propionibacteria, leuconostoc, streptococci, corynebacteria, actinomyces and mycobacteria.

In a preferred embodiment of the disclosure, the peptide or pilus structure is from probiotic bacteria, such as probiotic lactobacilli, lactococci, bifidobacteria, enterococci, propionibacteria, leuconostoc, and streptococci, or yeast. Probiotics are live micro-organisms, preferably non-pathogenic microbes which, when administered in adequate amounts to man or animal, promote the well being of the host (Fuller, R. 1989, J. Appl. Microbiol. 66:365-378). Probiotics will result in a beneficial health advantage to the host, when consumed as a food or a food supplement in adequate amounts.

Health claims of probiotics in humans or animals include the possible prevention and treatment of many ailments. The health-promoting effects of probiotics include for example the balancing and maintenance of intestinal flora, stimulation of the immune system and anti-carcinogenic activity. The useful effects of probiotics in human intestines are based on several independent factors caused by live bacterial cells, their cell structures and metabolic products.

A bacterium may be referred to as a probiotic, if it essentially meets the following requirements (Lee, Y-K and Salminen, S. 1995 Trend Food Sci Technol, 6:241-245): it remains viable in the demanding conditions prevailing in the digestive tract (low pH of the stomach, acids of the digestive system, etc.); attaches to the walls of the intestine; colonizes the GIT; metabolizes in the intestine; is technologically applicable (endures processing); exhibits clinically tested and reported health effects; and is safe to consume.

There are huge differences in microbial content between the different parts of the gastrointestinal tract, about 95% of all the intestinal bacteria appearing in the colon. Over 400 bacterial species have been estimated to thrive in the colon in addition to transient microbes. The dominating species are the following: *Bacteroides, Bifidobacterium, Coprococcus, Peptostreptococcus, Eubacterium* and *Ruminococcus.* The number of species *Lactobacillus, Streptococcus, Fusobacterium, Veillonella, Propionibacterium and Enterobacteriaceae* is slightly less. Some of the species represent useful microbes, whereas others may even be harmful (Tannock, G.W. 1998, Int. Dairy J. 8:527-533). Changes in the composition of the intestinal flora or a sudden reduction in the amount of it (due to severe diarrhea, antibiotics treatment, etc.) increase the infectivity of potentially pathogenic species, which may have serious consequences (outbreak of allergies, intestinal diseases, cancer).

In a preferred embodiment of the disclosure, the peptide or pilus structure binds to the gastrointestinal tract (GIT), most preferably to the epithelium of the gastrointestinal tract. In another preferred embodiment of the disclosure, the peptide or pilus structure binds to the mucus. Mucus is a slippery secretory product, a viscous colloid, from mucus-producing cells. Mucus protects epithelial cells for example in the GIT. In addition to antiseptic enzymes and immunoglobulins mucus also contains mucins and inorganic salts. As used herein, gastrointestinal tract refers to a tube from the mouth to the anus, which participates in digesting food. The GIT comprises the mouth, esophagus, stomach, duodenum, jejunum, ileum, small intestine, large intestine (colon), cecum, rectum and anus.

The best-documented probiotics include *L. rhamnosus* GG, *L. johnsonii* LA1, *L. casei* Shirota and *Bifidobacterium lactis* Bb12. In addition, a number of other probiotics have been described in the literature of the art. In a preferred embodiment of the invention, the peptide or pilus structure is from *Lactobacillus rhamnosus*, most preferably from *Lactobacillus rhamnosus* GG (LGG, LGG^{®}) strain, which is a non-pathogenic Gram-positive isolate originally from the USA (US4839281 A). LGG strain is isolated from human feces, it is able to grow well in pH 3 and survives even lower pH values as well as high bile acid contents. The strain exhibits excellent adhesion to both mucus and epithelial cells, and colonizes GIT. Lactic acid yield from glucose is good: when grown in MRS broth, the strain produces 1.5-2% of lactic acid: The strain does not ferment lactose and thus it does not produce lactic acid from lactose. The strain ferments following carbohydrates: D-arabinose, ribose, galactose, D-glucose, D-fructose, D-mannose, rhamnose, dulcitol, inositol, mannitol, sorbitol, N-acetylglucosamine, amygdalin, arbutin, esculin, salicin, cellobiose, maltose, saccharose, trehalose, melezitose, gentibiose, D-tagatose, L-fucose, and gluconate. The strain grows well at 15-45°C, the optimum temperature being 3-37°C. LGG has been deposited with the depository authority American Type Culture Collection under accession number ATCC 53103.

### Pilus genes

The genes encoding the pilin proteins of a pilus structure are clustered on the same loci in the LGG genome. Altogether two different gene clusters encoding the pilus peptides were found by bioinformatic methods in the LGG genome (Figure 2, see also Figures 11 for nucleotide sequences encoding the pili operons presented in Figure 2).

In one preferred embodiment of the disclosure, the polynucleotide has a sequence of any one of SEQ ID NOs 9-16 or a degenerate or fragment thereof, or it encodes the peptide of the invention or a fragment thereof. A polynucleotide that has a degenerate of a sequence shown in any one of SEQ ID NOs 9-16 means that it contains one or more different nucleotides, but still encodes for a same amino acids, A "polynucleotide" as used herein is a sequence of nucleotides, such as a DNA or RNA sequence, and may be a single or double stranded polynucleic acid. The term polynucleotide encompasses genomic DNA, cDNA and mRNA. Also, the polynucleotide may be an isolated DNA.

In another preferred embodiment of the invention, the gene cluster comprises at least one polynucleotide of the invention. In another preferred embodiment of the invention, the gene cluster comprises at least two, at least three or at least four polynucleotides of the invention. Most preferably the gene cluster comprises polynucleotides shown in SEQ ID NOs 9-12 or SEQ ID NOs 13-16. As used herein, "a gene cluster" refers to a group of at least two genes that encode for peptides/proteins needed for a joint function (concerted action), here e.g. for the pilus structure. The genes of the same cluster are usually grouped together on the same genetic locus.

According to a specific embodiment of the disclosure, the polynucleotide has at least 30, 35, 40, 45, 50, 55, 60, 65, 70, 75, 80, 81, 82, 83, 84, 85, 86, 87, 88, 89, 90, 91, 92, 93, 94, 95, 96, 97, 98, 99, 99.5, 99.8, 99.9 or 100% identity to any one of the nucleotide sequences of SEQ ID NO. 9, 10, 11, 12, 13, 14, 15 or 16, or fragments thereof.

In another specific embodiment of the disclosure the polynucleotide has a sequence shown in any one of the sequences SEQ ID NO 9, 10, 11, 12, 13, 14, 15 or 16.

### Products and pharmaceutical compositions

In one preferred embodiment of the invention, the product comprises at least one peptide or pilus structure of the invention. The product may also comprise at least two or at least three peptides of the invention. In one preferred embodiment, a product comprises at least one fragment of the peptide of the invention. The products of the invention may be selected from but are not limited to the group consisting of food products, animal feed, nutritional products, food supplements, food ingredients, health food, pharmaceutical products and cosmetics. In one preferred embodiment of the invention, the product is a food or feed product. In another embodiment of the invention the product is functional food, i.e. food having any health promoting and/or disease preventing or treating properties. Preferably a food product of the invention is selected from the group consisting of dairy products, bakery product, chocolate and confectionary, sugar and gum confectionary, cereal products, snacks, berry or fruit based products and drinks/beverages. Dairy products include but are not limited to milk, sour milk, yogurts and other fermented milk products such as cheeses and spreads, milk powders, children's food, baby food, toddler's food, infant formula, juices and soups. In addition to the peptides or pilus structures of the invention, the product may also contain other starters, probiotics etc.

In a preferred embodiment of the invention the product is a pharmaceutical composition. In one preferred embodiment of the invention, the pharmaceutical composition comprises at least one peptide or pilus structure of the invention, and in another embodiment, at least two, or at least three peptides of the invention. The pharmaceutical compositions may be used for example in solid, semisolid or liquid form, such as in the form of tablets, pellets, capsules, solutions, emulsions, suspensions, vaginal gels and ointments, vaginal suppositories and like,. Preferably the composition is for oral administration or for enteral applications.

In addition to at least one peptide or pilus structure of the invention, the pharmaceutical composition may comprise prebiotics, pharmaceutically acceptable carrier(s) (e.g. water, glucose or lactose), adjuvant(s), excipient(s), auxiliary excipient(s), antiseptic(s), stabilizing, thickening or coloring agent(s), perfume(s), binding agent(s), filling agent(s), lubricating agent(s), suspending agent(s), sweetener(s), flavoring agent(s), gelatinizer(s), anti-oxidant(s), preservative(s), buffer(s), pH regulator(s), wetting agent(s) or components normally found in corresponding products.

The product or pharmaceutical composition of the invention comprises the peptide or pilus structure in an amount sufficient to produce the desired effect. Other ingredients as well as other specific components of the products or pharmaceutical compositions are either obtained commercially or prepared by conventional techniques known in the art.

The products or pharmaceutical compositions may be manufactured by any conventional processes known in the art. Generating the peptide or pilus structure to a product means that the peptide or pilus structure may for example be added to any products or mixed with any agents. The peptide or pilus structure may also be generated in a product, for example, by expression in appropriate conditions. The peptide or pilus structure may be added or mixed either in connection with the preparation or thereafter, during the finishing of the end product. In a preferred embodiment of the invention, the peptide or pilus structure of the invention is added to a product.

### Production methods

The peptide or pilus structure of the invention can be produced for example by synthetic methods, e.g., peptide synthesis or by recombinant production with a genetically modified organism. In a preferred embodiment of the invention, the peptide or pilus structure is recombinant. As used herein, "recombinant" genetic material refers to a material, which typically is a combination of one or more genetic material, e.g. DNA strands of various origin, and it has been produced by combining or inserting the sequences. Recombinant production enables achieving specific and/or special traits into a gene or gene product or, for example, into expression of a gene (e.g. over- or under-expression). The polynucleotide of the invention may for example be put under the control of any endogenous or exogenous regulators, such as promoters. A recombinant protein is derived from a recombinant DNA.

At least one polynucleotide of interest may be isolated from a cell or produced synthetically. This nucleotide can be transformed to a host cell. A suitable host cell for producing any peptide of the invention may be any eukaryotic cell or micro-organism, preferably bacteria, most preferably lactic acid bacteria, such as lactobacilli, lactococci, bifidobacteria, enterococci, leuconostoc, and streptococci, or propionibacteria, or yeast.

As used herein, "transformation" refers to a genetic alteration of a cell by foreign genetic material, preferably DNA, resulting in expression of this genetic material. The foreign genetic material can be introduced as such or as incorporated into any other genetic material, such as vectors, plasmids etc. Any method of genetic engineering or any molecular cloning methods can be used for transforming a host cell with the polynucleotide of the invention. There are various methods of introducing foreign material into a eukaryotic cell. Materials, such as polymers (e.g., DEAE-dextran or polyethylenimine), liposomes and nanoparticles (e.g., gold), have been used as carriers for transformation. Genetic material can also be introduced into cells by using for example viruses or vectors as carriers. Other methods for introducing foreign material into a cell include but are not limited to nucleofection, electroporation, conjucation, transfection, sonoporation, heat shock and magnetofection. The use of various transfection reagents, such as calcium phosphate or lipofectamine, is well known in the art. A preferable method for introducing foreign material into a bacterial cell is electroporation.

The peptide or pilus structure of the invention may also be produced by cells expressing the peptides or pilus structures naturally.

After a natural cell or transformed host cell has produced the peptide of the invention in appropriate conditions, the peptide can for example be purified from the cell or a secreted form of the peptide can be recovered, e.g., from culture media. In order to purify the peptide, the cell may be disrupted for example by sonication, radiation, heating, lysis, mechanical agitation (sharing), enzymatic methods, 'cell pomb' or chemical agents (hypotonic shock, detergents, and solvents) or mixtures thereof. The peptide or pilus structure is obtainable from growing or metabolically active, i.e. live and/or lyophilized, or non-viable, e.g., heat-killed, irradiated or lysed organisms. The peptide or pilus structure is obtainable from a dead cell or a living cell.

The peptide or pilus structure can be produced in one cell and then displayed on the same cell, or the peptide or pilus structure may be produced in another cell than on which it is displayed.

Any known methods such as immunization can be used for producing antibodies against the peptides of the invention. Antibodies can be generated against any epitopes or functional domains of the peptides and they can be either monoclonal or polyclonal. In a preferred embodiment of the invention, the antibodies are polyclonal (Figure 3). As used herein, "a functional domain of a peptide" refers to any part of the peptide, which has a biological function.

### Treatments

Bacteria, a large group of unicellular micro-organisms, cause various diseases in eukaryotes, such as human beings, animals and plants. However, it is only within recent years that the presence of pili on the surface of important pathogens has gained interest among researches. Because GIT and its microbiota affect the well being of the subjects, the utility of the pili of the bacteria potentiates novel treatments. The peptides, pilus structures or polynucleotides of the invention can be utilized in a method of treating or preventing diseases either caused by micro-organisms, such as bacteria or virus, or caused by any other reason, such as unbalanced nutrition, stressed life style or genetic predisposition. Diseases or ailments, which can be prevented or treated with the peptides, pilus structures, polynucleotides or with the pharmaceutical products of the invention include but are not limited to diarrhea, such as traveler's diarrhea, arterial hypertension, vascular diseases, allergy, atopic diseases, urinary tract infections, respiratory infections, dental caries, irritable bowel syndrome, inflammatory bowel disease as well as remedying minor bowel discomfort and enhancing/promoting one's overall well-being. The composition of the invention is also useful for the prevention and treatment of gastrointestinal disorders and diseases, and for promoting general health. The disorders or diseases are preferably selected from the group consisting of mucosal inflammation, gut permeability disorders, IBD, IBS, and other gastrointestinal disorders. In a special embodiment of the invention peptides or pilus structures are used as vaccines (immunological response).

The method of reducing or inhibiting the adhesion of pathogenic bacteria to the GIT of a subject results in preventing or alleviating the symptoms caused by the pathogen. The pathogen is displaced from the epithelia or surface of the GIT by competition with the peptide or pilus structure of the invention. Example 11 of the application describes the competition assay displacing the harmful pathogenic bacteria with LGG pilus proteins. The preferred pathogens to be displaced include but are not limited to *Escherichia coli,* salmonella, bacilli, bacteroides, listeria, staphylococci, enterococci, clostridia and streptococci. As used herein, "pathogenic bacteria" refers to any bacteria causing any disease or any harmful effect. As used herein "adhesion" refers to anchoring of at least two molecules or structures to each other by chemical or physical bonds/forces or without them. Different types of adhesion, such as mechanical adhesion, chemical adhesion, dispersive adhesion, electrostatic adhesion and diffusive adhesion, are known. Adhesion can be a reversible or irreversible event, but in a biochemical system, adhesion is usually reversible.

*Enterococcus faecalis* and *Enterococcus faecium* are intestinal bacteria that are emerging nosocomial pathogens, including vancomycin-resistant enterococci (VRE) that are highly resistant to the important clinical antibiotic vancomycin (de Regt, M.J. et al., 2008, J Antimicrob Chemother. 62(6):1401-1406). These species are ranked among the top three nosocomial bacterial pathogens causing bloodstream, surgical site and urinary tract infections in hospitalised patients (Richards, M.J. et al., 2000, Infect Control Hosp Epidemiol 21:510-515). Enterococci can cause a wide variety of diseases: urinary tract infection, bacteremia, sepsis, endocarditis, wound and tissue infections, intra-abdominal and pelvic infections (Kayser, F.H. et al. 2003, Int J Food Microbiol 88(2-3):255-262), and infect surgical sites especially in the presence of implanted devices (Baldassarri L et al., 2005, Int J Artif Organs 28 (11):1101-1109) as well as cause meningitis (Pintado V et al., 2003, Medicine (Baltimore). 82(5):346-64), chronic apical periodontitis (Hancock H.H et al., 2001. Oral Surg Oral Med Oral pathol 91:579 -586) and periapical lesions (Sunde P.T. et al., 2002, J Endod 28:304 -310).

Recently, it has been described that *E. faecium* isolates contain surface located pili and remarkably, the vast majority (71 %) of the hospital-acquired and an important fraction (43 %) of the non-hospital strains of *E*. *faecium* contain pilus genes (Hendricks A.P. et al., 2008, Microbiology 154:3212-3223). In a double-blind and placebo-controlled study it has been described that consumption of *Lactobacillus rhamnosus* GG effectively cleared enterococci from infection in VRE-positive patients (Manley K.J. et al., 2007 Med J Aust. 186(9):454-457). The molecular support for the competition between pili-containing *Lactobacillus rhamnosus* GG and VRE originates from binding studies that showed that *Lactobacillus rhamnosus* has a 20-130 fold higher binding to human gastrointestinal mucus than vancomycin-resistant *E*. *faecium* (Pultz N.J. et al., 2006 Curr Microbiol. 52(3):221-224). Surprisingly, in a binding assay of this invention, the purified His-Tag labelled LGG proteins SpaA, SpaB and SpaC inhibit pathogens, e.g., vancomycin-resistant *E. faecium* from binding to the mucus.

The method of reducing or inhibiting the adhesion of pathogenic bacteria to the gastrointestinal tract, to the epithelium or to the mucus of a subject may comprise the following steps: i) producing at least one peptide of the invention or fragment thereof or pilus structure; ii) displaying the peptide, fragment and/or pilus structure on the cell or mucus.

In addition to reducing adherence of harmful or pathogenic bacteria, the present invention also offers the possibility to promote the adhesion of beneficial cells or other agents, such as enzyme(s), recombinant cells, microcapsule, nanocapsule or medicament(s), to the GIT. The method of promoting the adhesion of a bacterial cell to the mucus and to the GIT or a use of a peptide or a pilus structure of the invention for promoting the adhesion of a bacterial cell to the gastrointestinal mucus relates to a surprising ability of the novel peptides or pilus structures to adhere to the GIT *in vivo, ex vivo* or *in vitro.* The pilus peptide or pilus structure function as a tool for linking a cell or any other agent, such as medicaments, enzyme(s), micro-organism(s), recombinant cells, microcapsules or nanocapsules to the GIT.

The method of modifying the immune response in a subject and the use of the peptides or the pilus structure for modifying the immune response are based on a surprising finding that the peptides or the pilus structure of the invention cause changes in the immune response. An immune response refers to a response to an antigen in the body, in an *ex vivo* or *in vitro* system or to a response to another modulator. This response can be mediated by lymphocytes and/or the recognition of antigens by specific antibodies. One goal of the immune response is to destroy the antigen, which usually is of foreign origin, or to neutralize it. As used herein, "modifying" refers to any alteration of the immune response, such as an increase or decrease. Alterations of an immune response can be monitored by any suitable medical, physiological or biological test including but not limited to those, which are based on detecting activation of signalling pathways as well as detecting a transcription or translation level of marker genes or the amount of proteins, e.g., antibodies or receptors. Currently, there is no single marker available for determining the immune response in a cell or organism. However, preferable markers include but are not limited to tumor necrosis factor alpha (TNF-α), interleukin 12 (IL-12), IL-10, IL-1 β, and interferon alpha (IFN-α). Other possible markers are IL-1α, IL-6, IL-18, IFN-γ, IL-4, TGF-β, IL-I Ra and IL-18BP. In a preferred embodiment of the invention, the marker(s) is/are selected from a group consisting of TNF-α, Th1 cytokines, IL-10 and IL-12.

TNF-α is an inflammatory cytokine (Bertazza L and Mocellin S. 2008, Front Biosci. 13:2736-43), which activates immune system and initiates an inflammatory response to counteract infection. TNF- α also mediates along with IL-6 and INF-γ, the systemic effects of inflammation, such as fever and synthesis of acute phase proteins. The production of appropriate amounts of TNF- α as well as other proinflammatory cytokines is important in response to resolution of infection. However, inappropriate or excessive amounts of proinflammatory cytokines, such as TNF-α, has been linked to pathophysiological conditions such as rheumatoid arthritis, spondyloarthritis, uveitis, psoriasis and inflammatory bowel disease. TNF-α along with IFN-γ is also one of the Th1 type cytokines and therefore it activates macrophages and inhibits B cells and thereby promotes Th1 type immunity. TNF-α is also involved in the mast cell activation therefore takes part in the allergic reactions. The Th1 type response leads to cell-mediated immunity. The Th1 type responses coordinate the host response to intracellular pathogens and have a central role in activating phagocytes and in the promotion of microbial killing, which is important in different infections, like respiratory infections and gastrointestinal infections, such as diarrhea. Th1 type responses are also important in the balancing the immune response in allergy - in allergy the immune response is skewed towards a Th2 type response leading to hypersensitivity, and agents stimulating the Th1 type immune response can balance the situation.

Alterations of the immune response can be checked by *in vitro, ex vivo* or *in vivo* tests from any biological sample or subject. The properties of probiotic strains may be investigated in cell cultures (*in vitro*) utilizing for example peripheral blood mononuclear cells (PBMC), human monocytes, macrophages and dendrite cells. Examples of *ex vivo* experiments include determination of phagocytosis of neutrophils and monocytes, oxidative burst i.e. superoxide generation of neutrophils and monocytes, NK cell activity, lymphocyte proliferation and production of cytokines by peripheral blood mononuclear cells, monocytes or lymphocytes. *In vivo* experiments include but are not limited to the determination of a response to vaccines (e.g. vaccine specific antibodies or vaccine-specific antibody forming cells), delayed type hypersensitivity and a response to attenuated pathogens.

As an alternative to probiotic effects, the peptides or pilus structures of the invention may cause any other effects in a cell or a subject. These other effects may also occur alone or in addition to probiotic effects. A probiotic effect may be a combination of other immunomodulator(s) and peptides or pilus structures.

In the present invention, the subject for treatments or preventions can be any eukaryotic organism, preferably a human being or an animal, especially pets and production animals. The animal may be selected from a group consisting of production animals and pets, such as cows, horses, pigs, goats, sheep, poultry, dogs, cats, rabbits, reptiles and snakes.

### Screening methods

Any polynucleotide of the invention or any fragment thereof can be used for screening bacterial strains having similar pilus structures. In the method of screening bacterial strains, at least one polynucleotide or fragments thereof encoding pilus peptides or fragments thereof can be determined for example by PCR based methods, such as conventional PCR and sequencing or minisequencing; hybridisation methods, such as Southern or Northern hybridizations; any bioinformatic methods utilizing different programs and parameters; and any antibody based methods by using antibodies against peptides of the invention, flow cytometry, immunoprecipitation co-immunoprecipitation, immunohistochemistry, immunofluorescence, ELISA and ELIS-POT techniques. Therefore, in a preferred embodiment of the disclosure new bacterial strains having pilus structures are screened by PCR using primers designed on LGG pilus-genes. In another preferred embodiment of the disclosure, new bacterial strains having pilus structures are screened by Southern hybridization using amplification products of LGG genes of the invention as probes.

Stringent hybridisation conditions for primers or probes are preferred in the methods for screening homologous sequences or fragments to the polynucleotide of the invention. As used herein "homologous sequence" or "sequence having high identity" refers to a sequence, which may be identical but does not have to be identical to the other sequence. However, the sequences are similar and they have high identity %.

"Biologically congruent fragments" refers to similar sequences or sequences having identity percentage of over 35% and length over 50 amino acids.

In another preferred embodiment of the disclosure, new bacterial strains and meta-populations having pilus structures are screened by computational approaches from existing or newly created sequence listings or databases.

The sample to be screened can be taken from any organism or any matter, and may be, e.g., a bacterial culture, a tissue sample, a blood sample (serum or plasma sample), a food sample or an environmental sample. In a preferred embodiment of the invention the bacterial strain to be screened is a potential probiotic bacterial strain.

With the screening methods of the disclosure, it is possible to detect strains related to pathogenic bacterial strains or bacterial strains comprising known pathogenic components. Such strains may comprise sequences having functionally corresponding fragments to sequences of the present invention, such as to SEQ ID NO 4 (GG00444), and an at least 35 to 100% sequence identity, or comprise a polynucleotide of the present disclosure, such as a polynucleotide comprising SEQ ID NO 12 or a degenerate form thereof, or encodes a peptide of the present disclosure.

In the present invention, screening can be carried out *in vivo, in vitro, in silico* or *ex vivo* conditions.

The present invention is illustrated by the following examples, which are not intended to be limiting in any way.

### Example 1. Cloning, expression, and purification of recombinant LGG pilin proteins

The coding sequences for SpaA (GG00442), SpaB (GG00443), SpaC (GG00444), SpaD (GG02370), SpaE (GG02371), and SpaF (GG02372), excluding the region encoding the N-terminal signal peptide and the C-terminal cell wall sorting signal (CWSS), were PCR amplified from LGG genomic DNA using pairs of flanking 5'- and 3'-end oligonucleotide primers, one containing an EcoRI site (a Sacl site for GG02372) and another with a Xhol site (see Table 1). The amplified PCR fragments were cleaved with EcoRI (or SacI for GG02372) and Xhol restriction endonucleases, then ligated into the corresponding sites in the T7-regulated expression vector pET28b+, and the resulting recombinant plasmids (pKTH5319 for GG00442, pKTH5320 for GG00443, pKTH5321 for GG00444, pKTH5324 for GG02370, pKTH5379 for GG02371, and pKTH5341 for GG02372) propagated in the *E. coli* strain BL21 (DE3) pLysS for the expression of intracellular C-terminal hexahistidine-tagged proteins. Established procedures were employed in all DNA manipulations using standard protocols. For protein production, *E. coli* was grown at 37°C to midlog phase in Luria-Bertani medium supplemented with 50 µg/ml kanamycin, protein expression induced for three hours by 1 mM IPTG, the cells harvested by centrifugation, and the cell pellet resuspended in lysis buffer [50 mM NaH₂PO₄ (pH 8.0), 300 mM NaCl, 10 mM imidazole]. The cells were disrupted by sonication, clarified by centrifugation, and the cell-free lysates passed through a 0.45 µm filter. The hexahistidine-tagged pilin proteins were then purified by Ni²⁺-chelating affinity chromatography. Briefly, the cell-free lysates were each applied to a column of Ni-NTA agarose (Qiagen), washed with wash buffer [50 mM NaH₂PO₄ (pH 8.0), 300 mM NaCI, 20 mM imidazole], and the proteins eluted from the column with elution buffer [50 mM NaH₂PO₄ (pH 8.0), 300 mM NaCI, 250 mM imidazole]. Column fractions containing purified proteins were pooled, buffer-exchanged to 10 mM Tris-HCI (pH 8.0) for the SpaA (GG00442), SpaC (GG00444), SpaD (GG02370), SpaE (GG02371), and SpaF (GG02372) proteins and to 50 mM sodium acetate (pH 5.1) for the SpaB (GG00443) protein using a BioRad EconoPac 10 DG desalting column, and concentrated using a 30 kDa Microsep filter (Pall Life Sciences). The purity of the recombinant pilin proteins were monitored by SDS-PAGE and the protein concentrations estimated by A₂₈₀ measurements.

### Example 2. Generation of recombinant LGG pilin protein-specific polyclonal antibodies

Rabbit polyclonal antibodies specific for the SpaA (GG00442), SpaB (GG00443), SpaC (GG00444), SpaD (GG02370), SpaE (GG02371), and SpaF (GG02372) pilin proteins were produced according to the immunization protocol described by Johnston B.A. et al. (1991, Laboratory of Animal Science 41: 15-21). In brief, a subcutaneous (SC) injection (1 ml) of a 1:1 mix of 400 µg purified recombinant pilin protein in Freud's complete adjuvant was initially administered, followed by three sets of booster injections (SC) of 1:1 mixes of 200 µg protein in Freud's incomplete adjuvant at three-week intervals. The final blood collection was made two weeks after the last booster injection. The preparation of anti-sera from the blood was carried out using standard protocols.

**Table 1**

| **Gene** | **Forward oligonucleotide primer*** | **Reverse oligonucleotide primer**** |
|---|---|---|
| **SpaA (GG00442)** | 5'-TCGGGTTCA**GAATTC**TACGAATGATACGAC (SEQ NO 19) | 5'-TGCCAGTACCACC**CTCGAG**TGGCAGAATAC (SEQ NO 20) |
| **SpaB (GG00443)** | 5'-GCAGACACA**GAATTC**AACTGTGCCGACC (SEQ NO 21) | 5'-CAACTGTATCACC**CTCGAG**TGGCAACAATTGACG (SEQ NO 22) |
| **SpaC (GG00444)** | 5'-CAGTTCAGTTGT**GAATTC**CACTGATAACATTCG (SEQ NO 23) | 5'-AGCCCTGACCACC**CTCGAG**CGGCAAAATTGC (SEQ NO 24) |
| **SpaD (GG02370)** | 5'-ACCCGTACA**GAATTC**GACAACGACTGTG (SEQ NO 25) | 5'-GTCCGATTCCGCC**CTCGAG**CGGCAATAATTG (SEQ NO 26) |
| **SpaE (GG02371)** | 5'-CCACATTGGGTTCA**GAATTC**TGATCAAACTG (SEQ NO 27) | 5'-TGCGCCAATCGGA**CTCGAG**CGGCAAATAAC (SEQ NO 28) |
| **SpaF (GG02372)** | 5'-GCAAATTGGCAG**GAGCTC**GGTCCCGGTAG (SEQ NO 29) | 5'-CCGCTACCACC**CTCGAG**CGGTAGGAGTG (SEQ NO 30) |

| | | |
|---|---|---|
| * and ** Restriction endonucleases, EcoRI and Sacl in the forward and Xhol in the reverse oligonucleotide primers, are underlined and in boldfaced type | | |

### Example 3. Prediction of protein-encoding sequences by bioinformatic methods

Prediction of protein-encoding sequences was accomplished using Glimmer3 (Delcher A.L. et al. 2007, Bioinformatics. 23:673-679) and analyzing the completed genome sequence of LGG. Glimmer3 was applied using the iteration-mode script (g3-iterated.csh) with following modifications to default parameters: minimum gene length (150 bp) and maximum over lap (50 bp). Start sites of the initial predictions were rectified using BLAST (Altschul S.F. et al. 1997, Nucleic Acids Res. 25(17):3389-3402) and searching for putative ribosomal binding sites. The Glimmer3 predictions for GG00441, GG00442, GG00443, GG00444, GG02369, GG02370 and GG02371 were accepted as such, whereas the prediction of GG02372 was manually rectified to start 21 bp more downstream. Rho-dependent stops sites were predicted using TransTermHP (Kingsford C.L. et al. 2007, Genome Biol. 8:R22.) which showed that GG00441, GG00442, GG00443 and GG00444; GG02369, GG02370, GG02371 and GG02372 are transcribed as single transcript and thus form own operons.

Annotations were obtained by converting the predicted protein-encoding sequences to protein sequences and by performing a homology search against the public sequence database (Wheeler D.L. et al. 2008, Nucleic Acids Res. 36: D13-21). Annotations were accepted only from those sequences of which local alignments between the query had >=35% amino acid identity and covered >= 80% of the sequence of the subject. Based on this search, GG00441 and GG02369 were annotated as sortase-enzymes; GG00444 as a von Willebrand factor domain containing protein; GG02370 and GG02371 as a conserved hypothetical protein and GG02372 as an outer membrane protein. No annotations were obtained for GG00442 and GG00443.

Further annotation and information about the sequences were obtained by integrating information of InterPro and COG analyses (Mulder N.J. et al. 2007, Nucleic Acids Res. 35:D224-D228; Tatusov R.L. et al. 2000, Nucleic Acids Res. 28:33-36) and doing specific domain analyses. The specific domain searches were performed using Hmmsearch tool of the Hmmer-package and using sortase associated domain models, obtained from public databases of PFAM and TIGRFAM (Finn R.D. et al. 2008, Nucleic Acids Res. 36:D281-288; Haft D.H. et al. 2003, Nucleic Acids Res. 31:371-373). Following models were used to search for sortase-recognition sites: TIGR01167, TIGR03063, TIGR03065, TIGR03068 and PF00746 and the following models to search for sortases: TIGR01076, TIGR03064, PF04203 and PF07170. Both fs- and Is-models of the PFAM models were searched and the full length models of the TIGR models. Both search-types, the sequence and the domain search, were used. Matches scoring higher than the recorded trusted cut-off given by the database were considered significant. In cases, where the sequence-model was significant, every domain hit was accepted. These searches indicated that GG00441 and GG02369 are sortase-enzymes and that GG00442, GG00443, GG02370 and GG02372 contain the sortase-recognition site, thus being their likely substrates. Sortase-recognition sites were also searched for using regular expression searches (with the patterns LPXTG and LVNTG (Ton-That H et al. 2004, Mol Microbiol. 53:251-261), where X denotes any amino acid) revealing following matches: GG00442 and GG00443, GG00444, GG02370, GG02371 and GG02372. E-boxes were searched using YXXXETXXPX(G/N)X as the regular expression that was derived from the original YXLXETXAPXGY-pattern (Ton-That, H et al. 2004, Mol Microbiol. 53:251-261). The E-box search revealed hits on GG00442, GG00443, GG00444, GG02370 and GG02372 verifying the likeliness of these sequences to be sortase-substrates. The existence of possible secretion signals was tested using SignalP3-tool using both the hidden Markov model and the neural network methods. In all cases both methods predicted that the peptide sequences of GG00441, GG00442, GG00443, GG02370, GG02371 and GG02372 contained a signal suitable for the secretion.

### Example 4. Bioinformatic screens on public databases

Peptide sequences, fragments thereof, variants thereof, polynucleotide sequences, fragments thereof or variants thereof according to the present invention can be used for performing computational searches against public and private sequence collections and thereby for detecting bacterial strains comprising similar peptide sequences, polynucleotide sequences or pilus structures. Another preferred use of bioinformatic screening methods is for selecting bacterial communities enriched by the peptide sequences, polynucleotide sequences or pilus structures. Bioinformatic searches offer a plausible method for the detection of strains having sequences, which are in public sequence collections but have never been annotated or curated by an expert.

Bioinformatic searches are performed using algorithms such as BLAST (Altschul, S.F. et al. 1997, Nucleic Acids Res. 25(17): 3389-3402) or FASTA (Pearson, W.R. 1990, Methods Enzymol 183:63-98) (preferably default parameters are used). BLAST and FASTA algorithms are used to compare the selected sequences against a set of other sequences and to report statistically significant hits. Peptide sequences, polynucleotide sequences or pilus structures are searched from, for example, the following public sequence collections offered by the National Center for Biotechnology Information (NCBI): non-redundant protein sequences, environmental samples, whole-genome shotgun read and Genomic survey sequences; or preferably from a private sequence collection generated, for example, using high-throughput sequencing methods.

The peptide sequences of SEQ ID NO 1-8 or fragments thereof are used to screen for significant matches of peptides by performing a standard Blast search against the non-redundant protein sequence collection of NCBI. When a significant peptide match is found, a bacterium encoding this peptide of interest is classified as a putative probiotic strain or as a putative pathogen, against which the peptide is effective.

### Example 5. Atomic force microscopy showing LGG pili

An LGG strain was grown on a MRS (LabM) agar plate at 37°C for 20 hours anaerobically. Bacterial cells were diluted in sterile water, fixed to Mica slide and air dried. Both topographic and phase contrast figures of bacteria were obtained by Nanoscope IIIa Multimode AFM (Atomic force microscope, Digital Instruments, Santa Barbara) -microscope and J scanner (Figure 4).

### Example 6. Binding of recombinant LGG proteins to human intestinal mucus as assessed by non-quantitative ELISA-assay

The binding of recombinant hexahistidine-tagged SpaA, SpaB, SpaC, SpaD, SpaF pilin proteins to human intestinal mucus was assessed *in vitro.* Resected human intestinal tissue was used as a source of mucus. The use of resected human intestinal tissue was approved by the joint ethical committee of the University of Turku and Turku University Central Hospital (both in Turku, Finland) and informed written consent was obtained from the patients. The mucus was isolated from the healthy part of tissue obtained from patients undergoing colonic surgery e.g. due to colorectal cancer. The processing of intestinal tissue and the isolation of mucus was done as described previously (Vesterlund, S. et al 2005; Res Microbiol. 156(2):238-244; J Microbiol Methods 2005, 60(2):225-233). Mucus was passively immobilized on a polystyrene microtiter plate (Maxisorp, Nunc, Denmark) by overnight incubation at 4°C. The wells were washed three times with phosphate-buffered saline (PBS; pH 7.2) and blocked with 0.5% (w/v) bovine serum albumin (Sigma A7030) in PBS for 1 h at room temperature. The blocking solution was removed and 0.5 or 0.05 nmol of the hexahistidine-tagged pilin proteins in BSA-PBS was added followed by 1h incubation at 37°C. After incubation and washes the bound proteins was detected by enzyme-linked immunosorbent assay. The pilin proteins was detected by a mouse Tetra-His antibody (Qiagen, 34670) and a goat anti-mouse IgG Fab specific alkaline phosphatase conjugate (Sigma, A1293) as the secondary antibody. Dilutions 1:2000 and 1:5000 (v/v) were used for the primary and secondary antibodies, respectively. The substrate 4-nitrophenyl phosphate disodium salt (pNPP, Sigma, A7030) in di-ethanolamine-MgCI-buffer (Reagena, 170057, Finland) was added in concentration of 2 mg/ml and the color development was measured after 1 h at 405 nm. Results are average ± stdev from three parallel measurements (Figures 5a-b).

### Example 7. Extraction of cell wall-associated pilus proteins and western blot

Fresh 10 h cultures of LGG and LC705 (negative control) cells in MRS (LabM) were inoculated (1%) in mTSB medium (15 g/l TSB medium, BD Biosciences) enriched with 20g/l Bacto peptone (Difco), or MRS medium supplemented with 0.6% ox gall bile (Sigma) and cultivated at 37°C. Growth was monitored by measuring optical density (OD₆₀₀) and cells in stationary growth phase were collected by centrifugation.

The fractionation of the bacterial cells was done essentially as described elsewhere (Åvall-Jääskeläinen, S. et al. 2003, Appl Environ Microbiol 69:2230-2236). Briefly, the bacteria (10⁹ cfu) were washed once with PBS and homogenized by beating three times for two minutes with glass beads in a cell mill (Bühler Vibrogen-Zellmühle). The bacterial homogenates were resuspended in 500 µl PBS and centrifuged five minutes at 1.000 g. The supernatant was centrifuged at 16.000 g for 30 minutes at +4°C to collect the cell walls. The resulting pellets were resuspended in 50 µl of 50 mM Tris-Cl (pH 8..0) supplemented with 5 mM MgCl₂, 5 mM CaCl₂, 10 mg/ml lysozyme, and 42 U/ml mutanolysin. The resuspended cell wall pellets were incubated 3 hours at 37°C to release the cell wall associated polypeptides.

The enzymatically treated cell wall fractions were run on a 4-15% gradient gel (Bio-Rad) and transferred to an Immobilon-P PVDF membrane (Millipore). The membrane was subjected to Western analysis with the ECL Advance™ Western Blotting Detection Kit (Amersham) according to manufacturer's instructions. The SpaA and SpaC pilin protein-specific polyclonal primary antibodies (see Example 2) were diluted 1:25.000, and the Goat Anti-Rabbit IgG (H+L)-HRP-Conjugate (Bio-Rad) secondary antibody was diluted 1:100.000. SpaB pilin protein was detected with a SpaB pilin protein-specific polyclonal primary antibody, Goat Anti-rabbit IgG - AP-Conjugate (Bio Rad) and BCIP/NBT color reagent.

The pili in gram-positive bacteria are composed of pilin subunits covalently linked to one another. The monomeric pilin subunits are added to the growing pili one by one by the action of sortases, and as a consequence, at a given time point each individual cell carries pili of different lengths on its surface (Scott J.R. and Zahner D. 2006, Mol Microbiol 62:320-330). Thus, a classical way to show the existence of pili is to subject a mutanolysin/lysozyme treated cell wall fraction to Western analysis: if pili are present, a high molecular weight ladder (HMW) will be detected on the blot, and in many instances also a pilin monomers will be observed (Scott, J.R. and Zahner, D. 2006, Mol Microbiol 62:320-330). The presence of SpaA, Spa B and SpaC containing pili in LGG is clearly evident from Figures 6a, 6b and 6c, since the monomeric SpaA, Spa B and SpaC pilin subunits and HMWs can be identified from the LGG cell wall extracts using SpaA, SpaB and SpaC -specific antibodies, whereas LC705 cells are deficient of SpaA, SpaB and SpaC moieties. The exposure time needed to record chemiluminescent signal from the SpaC blot was 60 seconds, whereas exposure time of 1 second was sufficient for the SpaA blot, implying the SpaA to be present at higher numbers in the pili as SpaC. This difference in relative numbers might suggest the SpaA to be the shaft forming pilin subunit, whereas SpaC could serve as a pilus tip adhesin. Also of notice is that pili are found in LGG cells grown in a medium supplemented with bile, indicating that pili might be expressed in the human gastrointestinal tract.

### Example 8. Screening of new probiotic strains having pili structures by PCR

Lactobacilli were grown anaerobically in MRS broth at +37°C for 10 hours. The genomic DNA was isolated as follows. 1 ml of the culture is centrifuged at 14.000 g for 2 min. The collected cells were resuspended in 480 µl of 50 mM EDTA, 100 µl of 50 mg / ml lysozyme (Amresco, Solon, OH, USA) and 20 µl of 50 U / µl mutanolysine (Sigma) was added and the mixture was incubated at 37°C for 1 h. The mixture was centrifuged for 2 min at 14 000 g, the supernatant was discarded and the bacterial pellet was extracted with a Wizard® Genomic DNA Purification Kit (Promega) according to the manufacturer's instructions. The purified DNA was suspended in 200 µl of Tris-EDTA (TE) buffer. About 200 ng of genomic DNA was used as a template in PCR reaction. PCR was performed using Dynazyme polymerase (Finnzymes, Espoo, Finland) and oligonucleotide primers based on sequences GG00442, GG00443, GG00444 and GG02370, GG02371, GG02372 genes shown in Table 2. The PCR reaction was performed with a PCT-200 apparatus (MJ Research, Waltham, MA, USA) and contains 10 mM Tris-HCl, 1.5 mM MgCl₂, 50 mM KCl and 0.1% Triton-X 100 (pH 8.8). The primers were used at 1-µM and the deoxynucleotides at 200-µM concentrations. Initial denaturation was at 94° for 2 min. The first cycle was 1 min each at 95°C, 65°C and 72°C, the next five cycles were 1 min each at 95°C, 60°C and 72°C, and the last 25 cycles were 1 min each at 95°C, 55°C and 72°C. To terminate cycling the reaction mixture was maintained at 72°C for 5 min and at 4°C for 15 min. The amplified DNA bands were separated in 0.7% agarose gel by gel electrophoresis (see Figures 7a-c).

All strains LGG, LC705 and *Lactobacillus casei* ATCC 334 showed pili structures of the invention (see Figures 7a-c).

**Table 2**

| **Gene** | **Forward oligonucleotide primer** | **Reverse oligonucleotide primer** |
|---|---|---|
| SpaA (GG00442) | 5'-TCTCGGGTTTAATGGCACTC (SEQ NO 31) | 5'-TCTGTATTGGCAGCAGCATC (SEQ NO 32) |
| SpaB (GG00443) | 5'-TCCTTCCGTCCGTTAGTGAT (SEQ NO 33) | 5'-CGTTTGTGGCAACAATTGAC (SEQ NO 34) |
| SpaC (GG00444) | 5'-CCAAATTGGCAACAGACCTT (SEQ NO 35) | 5'-GCCATCTGGTGCTTTTGTTT (SEQ NO 36) |
| SpaD (GG02370) | 5'-CGGACGCCTTTTACCAATTA (SEQ NO 37) | 5'-AACAGGTTTCGTACCGCATC (SEQ NO 38) |
| SpaE (GG02371) | 5'-TATGACGCGTAAGCAAGCAC (SEQ NO 39) | 5'-TGGCCGTCAATTAACACAAA (SEQ NO 40) |
| SpaF (GG02372) | 5'-CTACCGGAGCATGTCGAGTT (SEQ NO 41) | 5'-GGCCATTTTCATCAGTCGTT (SEQ NO 42) |

Example of the primers for amplification of pili genes are shown in Table 2, but are not limited to those. The sizes of the amplified PCR-products using *L. rhamnosus* GG DNA as a template and Table 2 primers are 780bp, 612 bp and 801 bp for SpaA, SpaB, SpaC, respectively, and for SpaD, SpaE and SpaF 688bp, 705bp and 799 bp.

### Example 9. Screening of new probiotics having pili genes by Southern hybridization

New probiotic strains having pili structures were screened by Southern hybridization using LGG amplification products from Example 8 as probes. Hybridization conditions were adjusted to stringent, enabling probe hybridization only to identical sequences, or to low stringent, allowing some amount of sequence discrepancy. The PCR amplification products of SpaA, B, C, D, E and F were purified in NuSieve low melt agarose (FMC Bioproducts, Rockland, ME, USA) and labelled with DIG-system (Roche Diagnostics). The total DNA of the bacterial strains was digested with HindIII, and the resulting fragments were separated in 0.7% agarose gel. The DNA fragments in agarose were blotted onto nylon membranes and hybridized according to standard procedure of DIG-system. Stringent hybridization was performed at 68°C, washes were twice in 2 x SSC - 0.1 % SDS at room temperature, and twice in 0.1 x SSC-0.1% SDS for 15 minutes at 68°C. Hybridization with lower stringency was performed at 60°C, and the last two washes were in 0.5 x SSC-0.1% SDS at 50°C for 15 minutes. Hybridization was detected with alkaline-phosphatase-conjugated antibody and NBT/BCIP color reaction (DIG-system, Roche).

Figure 8 shows digested genomic DNAs separated by agarose gel electrophoresis (Figure 8a) and Southern hybridizations of the same DNAs using DIG-labeled PCR amplification product of *Lactobacillus rhamnosus* GG *spaC* gene (801 bp), *spaB* (612 bp) or *spaA* (780 bp) as a probe (Figures 8b-8d). The PCR reaction was performed using SpaC, SpaB or SpaA primers shown in Table 2. Hybridization was done at +68°C.

Hybridization signals indicated the presence of *spaC, spaB* and *spaA* in *L*. *casei* ATCC 334 (lane 3) and *L. rhamnosus* GG (lane 5) but not in *L*. *rhamnosus* LC705 (lane 4).

### Example 10. Immunomodulation by purified LGG pilus proteins

Human macrophages were isolated from blood of healthy volunteers (buffy coat fraction) as documented previously (Miettinen, M. et al. 2000, J Immunol 164:3733-3740; Miettinen, M. et al. 2008, J Leuk Biol 84:1092-1100). Essentially, this was done using freshly collected, leukocyte-rich buffy coats from 4 healthy blood donors (supplied by the Finnish Red Cross Blood Transfusion Service, Helsinki FI) and isolating peripheral blood monocuclear cells (PBMCs) by Ficoll-Paque (Amersham Pharmacia Biotech, Uppsala SE) gradient centrifugation. Monocytes were purified from PBMCs by adherence on six-well plastic plates (Falcon Becton Dickinson, Franklin Lakes NJ, US) and cultured for 7 days in macrophage -serum-free medium (Gibco Invitrogen, Grand Island NY, US) in the presence of 10 ng/ml recombinant human (rh) GM-CSF (Leucomax, Schering-Plough, Innishannon, IRL) to obtain macrophages. Macrophages were incubated at a concentration of approximately 4 million cells per well in a 6-well microtiter plate and stimulated with an equivalent number of live bacteria (LGG and *Streptococcus pyogenes* T1M1) or approximately 3, 100, 3000 or 10000, etc., fmol of purified His-Tag labelled LGG proteins SpaA and SpaC. After incubation for 6 h and 24 h, the modulation of the amounts of immune markers or activation of signalling pathways or receptor expression was determined as described previously (Miettinen, M. et al. 1996, Infec immunol 64:5403-5405; Miettinen, M. et al. 2000, J Immunol 164:3733-3740; Miettinen, M. et al. 2008, J Leuk Biol 84:1092-1100).

Figure 9 shows TNF-α levels during macrophage stimulation with live LGG bacteria (2x10⁶ cfu/ml) or with purified His-Tag labelled LGG proteins SpaA, SpaB and SpaC (approximately 30 pmol/ml) TNF-α levels increased in stimulation with SpaA and SpaC.

Typically, cells of the probiotic LGG and the pathogen *S*. *pyogenes* T1M1 show immunomodulatory activities and induce a specific Th1-like response in PBMCs or macrophages (Miettinen, M. et al. 2000, J Immunol 164: 3733-3740; Veckman, V. et al. 2003, J Leuk Biol 74:395-402). Remarkably, the purified LGG pili proteins also induce a response in macrophages, demonstrating their functionality in immunomodulation. Moreover, these experiments show that the LGG pili proteins signal to human host cells.

### Example 11. Competition assay with LGG pilus proteins

The processing of intestinal tissue and the isolation of mucus was done as described in Example 6.

The competition assay was carried out according to Vesterlund, S. et al. 2006 (Microbiology 152(6):1819-1826). Mucus (Sigma), at a concentration 0.5 mg/ml, was passively immobilized on a polystyrene microtiter plate (Maxisorp, Nunc, Denmark) by overnight incubation at 4°C. The wells were washed two times with phosphate-buffered saline (PBS; pH 7.2). *Enterococcus faecium* was cultured in brain-heart-infusion broth and LGG in MRS broth overnight in anaerobic conditions at 37°C. To the *E*. *faecium* culture 10 µl ml⁻¹ of [5'-3H]thymidine (16.7 Ci mmol⁻¹; 618 GBq mmol⁻¹) was added to metabolically radiolabel the bacteria. The bacterial cells were harvested by centrifugation and washed twice with PBS buffer. The OD₆₀₀ of the bacterial suspensions were adjusted with PBS to 0.25.

A concentration adjusted *E. faecium* cell suspension was added to the wells in a volume of 100 µl, five parallel wells were used in each experiment. The bacteria were allowed to adhere for 1 h at 37°C and the wells were washed two times with 200 µl PBS to remove the non-adherent bacteria. 100 µl of the concentration adjusted LGG cell suspension or 0.5 nmol of the SpaC protein in 100 µl of PBS buffer were added and followed by incubation for 1 h at 37°C. The wells were washed two times with 200 µl PBS and the bacteria bound to mucus were released and lysed with 1% SDS - 0.1 M NaOH by incubation at 60°C for 1h, followed by the measurement of radioactivity by liquid scintillation. The adhesion ratio (%) of bacteria was calculated by comparing the number of adhered bacteria to that of added bacteria. Pair-wise student's t test was used to determine the significance (P< 0.05) of differences between the control and the samples.

Figure 10 shows that LGG and LGG pilin proteins SpaA, SpaB and SpaC replaced the adhered pathogenic bacterium (*E*. *faecium*) from the human intestinal mucus.

## Claims

1. An isolated peptide comprising a sequence having at least 95% sequence identity with SEQ ID NO 4 (GG00444).

2. An isolated pilus structure comprising the peptide according to claim 1.

3. A peptide or a pilus structure according to claim 1 or 2, which is recombinant.

4. A peptide or a pilus structure according to any one of the previous claims, which is from bacteria, gram positive bacteria, *Lactobacillus rhamnosus* or *Lactobacillus rhamnosus GG* (LGG) strain.

5. A peptide or a pilus structure according to any one of the previous claims, which binds to the gastrointestinal tract and/or to the mucus.

6. A product comprising the isolated peptide or the isolated pilus structure according to any one of claims 1-5.

7. A product according to claim 6, which is a food product, a feed product or a pharmaceutical composition.

8. A product according to claim 7, wherein the product is a food product, which is selected from the group consisting of dairy products, bakery product, chocolate and confectionary, sugar and gum confectionary, cereal products, snacks, berry or fruit based products and drinks/beverages, including milk, sour milk, yogurts, cheeses and spreads, milk powders, children's food, baby food, toddler's food, infant formula, juices and soups.

9. A product comprising the isolated peptide or the isolated pilus structure according to any one of claims 1- 5 for use as a medicament.

10. A product comprising the peptide or the pilus structure according to any one of claims 1-5 for the prevention or treatment of diarrhea, arterial hypertension, vascular diseases, allergies, cancer, atopic diseases, viral diseases, infectious diseases, urinary tract infections, respiratory infections, dental caries, irritable bowel syndrome, inflammatory bowel disease, mucosal inflammation, gut permeability disorders, obesity, metabolic syndrome, oxidative stress or abdominal pain.

11. A polynucleotide comprising the sequence of SEQ ID NO 12 or encoding the peptide according to any one of claims 1 and 3-5.

12. A vector comprising the polynucleotide according to claim 11.

13. A host cell transformed with the vector according to claim 12.

14. A gene cluster encoding peptides of a pilus structure and comprising the polynucleotide according to claim 11.

15. An antibody specifically binding the peptide according to any one of claims 1 and 3-5, or specifically binding the functional domains of said peptide.

16. A peptide and/or a pilus structure according to any one of claims 1-5 for reducing or inhibiting the adhesion of pathogenic bacteria to the gastrointestinal tract, to the epithelium and/or to the mucus of a subject, for promoting the adhesion of a bacterial cell or any other agent to the mucus or epithelium, and/or for modifying immune response in a subject.

17. A method of producing a product according to any one of claims 6-8, wherein the method comprises a step of adding the peptide or the pilus structure according to any one of claims 1-5 to the product.

18. A method of producing the peptide according to any one of claims 1 and 3-5, wherein the method comprises the following steps:
A)
i) providing the polynucleotide according to claim 11;
ii) transforming a host cell with the polynucleotide;
iii) culturing the host cell from step ii) to produce the peptide;
iv) recovering the peptide, or
B)
i) providing amino acids;
ii) manufacturing the peptide according to any one of claims 1 and 3-5 from the amino acids of step i) with synthetizing the peptide.

19. A method of producing the peptide or the pilus structure according to any one of claims 1-5, wherein the method comprises the following steps:
i) disrupting a cell producing or comprising the peptide or the pilus structure according to any one of claims 1-5;
ii) recovering the peptide or the pilus structure.

## Patentansprüche

1. Isoliertes Polypeptid, umfassend eine Sequenz mit mindestens 95 % Sequenz-Identität mit SEQ ID NO: 4 (GG00444).

2. Isolierte Pilusstruktur, umfassend das Peptid gemäß Anspruch 1.

3. Peptid oder Pilusstruktur gemäß Anspruch 1 oder 2, die rekombinant ist.

4. Peptid oder Pilusstruktur nach einem beliebigen der vorhergehenden Ansprüche, die aus Bakterien, gram-positiven Bakterien, Lactobacillus rhamnosus oder Lactobacillus rhamnosus gg (Igg)-Stamm ist.

5. Peptid oder Pilusstruktur nach einem beliebigen der vorhergehenden Ansprüche, welches/welche an den gastrointestinalen Trakt und/oder die Schleimhaut bindet.

6. Produkt, umfassend das isolierte Peptid oder die isolierte Pilusstruktur gemäß einem beliebigen der Ansprüche 1-5.

7. Produkt nach Anspruch 6, das ein Lebensmittelprodukt, ein Futtermittelprodukt oder eine pharmazeutische Zusammensetzung ist.

8. Produkt nach Anspruch 7, wobei das Produkt ein Lebensmittelprodukt ist, das ausgewählt ist aus der Gruppe, bestehend aus Milchprodukten, Backware, Schokolade und Konfekt, Zucker- und Gummikonditorware, Getreideprodukten, Snacks, Beeren- oder Frucht-basierten Produkten und Getränken, einschließlich Milch, Sauermilch, Jogurt, Käse und Brotaufstrichen, Milchpulvern, Kindernahrung, Babynahrung, Kleinkindnahrung, Säuglingsfertignahrung, Säften und Suppen.

9. Produkt, umfassend das isolierte Peptid oder die isolierte Pilusstruktur gemäß einem beliebigen der Ansprüche 1-5 zur Verwendung als ein Medikament.

10. Produkt, umfassend das Peptid oder die Pilusstruktur gemäß einem beliebigen der Ansprüche 1-5 für die Prävention oder Behandlung von Diarrhöe, arterielle Hypertonie, Gefäßerkrankungen, Allergien, Krebs, atopischen Erkrankungen, Viruserkrankungen, Infektionserkrankungen, Harnwegsinfektionen, Atemwegsinfektionen, Zahnkaries, Reizdarmsyndrom, entzündliche Darmerkrankung, Schleimhautentzündung, Störungen der Darmdurchgängigkeit, Obesitas, metabolisches Syndrom, oxidativer Stress oder abdominaler Schmerz.

11. Polynukleotid, umfassend die Sequenz von SEQ ID NO: 12 oder kodierend das Peptid gemäß einem beliebigen der Ansprüche 1 und 3-5.

12. Vektor, umfassend das Polynukleotid gemäß Anspruch 11.

13. Wirtszelle, transformiert mit dem Vektor gemäß Anspruch 12.

14. Gen-Cluster, kodierend Peptide einer Pilusstruktur und umfassend das Polynukleotid gemäß Anspruch 11.

15. Antikörper, der spezifisch das Peptid gemäß einem beliebigen der Ansprüche 1 und 3-5 bindet, oder spezifisch die funktionellen Domänen dieses Peptids bindet.

16. Peptid und/oder Pilusstruktur gemäß einem beliebigen der Ansprüche 1-5 zum Reduzieren oder Inhibieren der Adhäsion von pathogenen Bakterien an den gastrointestinalen Trakt, das Epithel und/oder die Schleimhaut eines Subjekts zum Fördern der Adhäsion einer Bakterienzelle oder eines beliebigen anderen Mittels an die Schleimhaut oder das Epithel und/oder zum Modifizieren einer Immunantwort in einem Subjekt.

17. Verfahren zum Herstellen eines Produkts gemäß eines beliebigen der Ansprüche 6-8, wobei das Verfahren einen Schritt des Hinzufügens des Peptids oder der Pilusstruktur gemäß einem beliebigen der Ansprüche 1-5 zu dem Produkt umfasst.

18. Verfahren zum Herstellen des Peptids gemäß einem beliebigen der Ansprüche 1 und 3-5, wobei das Verfahren die folgenden Schritte umfasst:
A)
i) Bereitstellen des Polynukleotids gemäß Anspruch 11;
ii) Transformieren einer Wirtszelle mit dem Polynukleotid;
iii) Kultivieren der Wirtszelle aus Schritt ii), um das Peptid herzustellen;
iv) Gewinnen des Peptids, oder
B)
i) Bereitstellen von Aminosäuren;
ii) Erzeugen des Peptids gemäß eines beliebigen der Ansprüche 1 und 3-5 aus den Aminosäuren von Schritt i) unter Synthetisieren des Peptids.

19. Verfahren zum Herstellen des Peptids oder der Pilusstruktur gemäß eines beliebigen der Ansprüche 1-5, wobei das Verfahren die folgenden Schritte umfasst:
i) Aufschließen einer Zelle, die das Peptid oder die Pilusstruktur gemäß eines beliebigen der Ansprüche 1-5 herstellt oder umfasst;
ii) Gewinnen des Peptids oder der Pilusstruktur.

## Revendications

1. Peptide isolé comprenant une séquence ayant au moins 95% d'identité de séquence avec SEQ ID NO 4 (GG00444).

2. Structure de pilus isolée comprenant le peptide selon la revendication 1.

3. Peptide ou structure de pilus selon la revendication 1 ou la revendication 2, qui est recombinant(e).

4. Peptide ou structure de pilus selon l'une quelconque des revendications précédentes, qui provient de bactéries, de bactéries gram positives, de *Lactobacillus rhamnosus* ou de souche de *Lactobacillus rhamnosus GG* (LGG).

5. Peptide ou structure de pilus selon l'une quelconque des revendications précédentes, qui se lie à l'appareil gastro-intestinal et/ou au mucus.

6. Produit comprenant le peptide isolé ou la structure de pilus isolée selon l'une quelconque des revendications 1 à 5.

7. Produit selon la revendication 6, qui est une denrée alimentaire, un aliment ou une composition pharmaceutique.

8. Produit selon la revendication 7, dans lequel le produit est une denrée alimentaire, qui est choisie dans le groupe constitué par des produits laitiers, des produits de boulangerie, du chocolat et de la confiserie, du sucre et de la gomme de confiserie, des produits de céréales, des collations, produits et boissons à base de baies ou fruits, y compris du lait, du lait caillé, des yaourts, des fromages et pâtes à tartiner, des poudres de lait, de la nourriture pour enfants, de la nourriture pour bébés, de la nourriture pour tout-petits, des préparations pour nourrissons, des jus et soupes.

9. Produit comprenant le peptide isolé ou la structure de pilus isolée selon l'une quelconque des revendications de 1 à 5 pour une utilisation en tant que médicament.

10. Produit comprenant le peptide ou la structure de pilus selon l'une quelconque des revendications 1 à 5 pour la prévention ou le traitement de la diarrhée, de l'hypertension artérielle, de maladies vasculaires, d'allergies, de cancers, de maladies atopiques, de maladies virales, de maladies infectieuses, d'infections des voies urinaires, d'infections respiratoires, de caries dentaires, du syndrome du côlon irritable, de maladies inflammatoire de l'intestin, d'inflammations des muqueuses, de troubles de la perméabilité intestinale, d'obésité, de syndromes métaboliques, de stress oxydatifs ou de douleurs abdominales.

11. Polynucléotide comprenant la séquence de SEQ ID N ° 12 ou codant pour le peptide selon l'une quelconque des revendications 1 et 3-5.

12. Vecteur comprenant le polynucléotide selon la revendication 11.

13. Cellule hôte transformée avec le vecteur selon la revendication 12.

14. Groupe de gènes codant pour des peptides d'une structure de pilus et comprenant le polynucléotide selon la revendication 11.

15. Anticorps se liant spécifiquement au peptide selon l'une quelconque des revendications 1 et 3-5, ou se liant spécifiquement à des domaines fonctionnels dudit peptide.

16. Peptide et/ou structure de pilus selon l'une quelconque des revendications 1 à 5 pour réduire ou inhiber l'adhérence de bactéries pathogènes à l'appareil gastro-intestinal, à l'épithélium et/ou au mucus d'un sujet, pour la promotion de l'adhérence d'une cellule bactérienne ou de tout autre agent au mucus ou à l'épithélium, et/ou pour la modification de la réponse immunitaire chez un sujet.

17. Procédé de production d'un produit selon l'une quelconque des revendications 6-8, dans lequel le procédé comprend une étape d'addition du peptide ou de la structure de pilus selon l'une quelconque des revendications 1 à 5 au produit.

18. Procédé de production du peptide selon l'une quelconque des revendications 1 et 3 à 5, dans lequel le procédé comprend les étapes suivantes:
A)
i) la fourniture d'un polynucléotide selon la revendication 11;
ii) la transformation d'une cellule hôte avec le polynucléotide;
iii) la culture de la cellule hôte de l'étape ii) pour produire le peptide;
iv) la récupération du peptide, ou
B)
i) la fourniture d'acides aminés;
ii) la fabrication du peptide selon l'une quelconque des revendications 1 et 3 à 5 à partir des acides aminés de l'étape i) avec la synthèse du peptide.

19. Procédé de production du peptide ou de la structure de pilus selon l'une quelconque des revendications 1 à 5, dans lequel le procédé comprend les étapes suivantes:
i) la rupture d'une cellule produisant ou comprenant le peptide ou la structure de pilus selon l'une quelconque des revendications 1 à 5;
ii) la récupération du peptide ou de la structure de pilus.


### SEQUENCE LISTING

<110> Valio Ltd.
<120> Novel peptides and methods for producing them
<130> 2081707PC
<160> 42
<170> PatentIn version 3.3
<210> 1
   <211> 359
   <212> PRT
   <213> Lactobacillus rhamnosus
<400> 1
   Met Thr Lys Arg Thr Arg Arg Pro Leu Asp Leu Ile Asp Ile Val Ile
   1               5                   10                  15
   Gly Cys Leu Leu Leu Ala Gly Phe Gly Val Leu Cys Tyr Pro Phe Ala
               20                  25                  30
   Ser Asp Ala Tyr Val Ser Tyr Gln Asn Gln Gln Val Ile Asp Arg Tyr
           35                  40                  45
   Arg Gln Gln Glu Ala Arg Lys Asn Gln Met Val Leu Arg Arg Glu Tyr
       50                  55                  60
   Asn Asp Tyr Gln Gln Lys Asn Lys Gln Leu Ala Ala Ser Gln Gln Val
   65                  70                  75                  80
   Pro Gly Val Ala Ser Phe Asn His Ala Val Asn Asp Gln Gly Thr Ala
                   85                  90                  95
   Lys Thr Ala Ala Lys Arg Asn Gln Gln Ile Leu Thr Arg Gln Thr Val
               100                 105                 110
   Ala Gln Leu Thr Ile Pro Lys Ile Gly Leu Ser Leu Pro Val Phe Asp
           115                 120                 125
   His Thr Ser Asp Trp Leu Leu Gln Phe Gly Ala Cys Leu Leu Asp Gly
       130                 135                 140
   Thr Ser Tyr Pro Thr Gly Gly Lys Asn Thr His Ala Val Ile Ser Ala
   145                 150                 155                 160
   His Arg Gly Val Pro Asn Ala Glu Leu Phe Thr Arg Val Pro Ala Leu
                   165                 170                 175
   Lys Lys Gly Asp Lys Phe Phe Ile Ser Ile Gly Asn His Lys Leu Ala
               180                 185                 190
   Tyr Gln Val Phe Lys Arg Gln Val Ile Glu Pro Ser Asp Thr Arg Gln
   195 200 205
   Leu Arg Ile Val Pro Gly Gln Asp Leu Val Thr Leu Met Thr Cys Thr
   210 215 220
   Pro Tyr Met Ile Asn Ser His Arg Leu Leu Ile Thr Gly Arg Arg Ile
   225 230 235 240
   Pro Tyr Val Lys Ala Asp Glu Glu Ala Ser Ser Trp Ala Val Trp Trp
   245 250 255
   Asn Lys Leu Lys Leu Ile Val Ala Leu Leu Gly Ala Val Ile Ile Leu
   260 265 270
   Gly Val Ile Gly Phe Val Met Arg Ser Leu Met Leu Gly Arg Lys His
   275 280 285
   Tyr Leu Leu Glu Val Pro Ala Glu Ala Thr Gln Val Val Val Lys Arg
   290 295 300
   Gly Arg His Ile His Ser Phe Lys Ser Asp Gln Thr Gly Val Thr Asp
   305 310 315 320
   Ile Ser Leu Pro Gly Asn His Tyr Arg Val Ala Ile Val Thr Pro Leu
   325 330 335
   Gly Arg Thr Lys Tyr Lys Ala Tyr Val Lys Lys Ile Arg Asp Lys Ser
   340 345 350
   Phe Gln Leu Lys Glu Tyr His
   355
<210> 2
   <211> 334
   <212> PRT
   <213> Lactobacillus rhamnosus
<400> 2
   Met Lys Lys Thr Ile Ala Lys Lys Val Leu Thr Leu Thr Ser Thr Ile
   1 5 10 15
   Leu Met Thr Leu Leu Met Val Leu Gly Phe Asn Gly Thr Arg Val Gln
   20 25 30
   Ala Asp Thr Asn Asp Thr Thr Thr Gln Asn Val Val Leu Thr Lys Tyr
   35 40 45
   Gly Phe Asp Lys Asp Val Thr Ala Ile Asp Arg Ala Thr Asp Gln Ile
       50                  55                  60
   Trp Thr Gly Asp Gly Ala Lys Pro Leu Gln Gly Val Asp Phe Thr Ile
   65                  70                  75                  80
   Tyr Asn Val Thr Ala Asn Tyr Trp Ala Ser Pro Lys Asp Tyr Lys Gly
                   85                  90                  95
   Ser Phe Asp Ser Ala Pro Val Ala Ala Thr Gly Thr Thr Asn Asp Lys
               100                 105                 110
   Gly Gln Leu Thr Gln Ala Leu Pro Ile Gln Ser Lys Asp Ala Ser Gly
           115                 120                 125
   Lys Thr Arg Ala Ala Val Tyr Leu Phe His Glu Thr Asn Pro Arg Ala
       130                 135                 140
   Gly Tyr Asn Thr Ser Ala Asp Phe Trp Leu Thr Leu Pro Ala Lys Ala
   145                 150                 155                 160
   Ala Ala Asp Gly Asn Val Tyr Val Tyr Pro Lys Asn Val Gln Lys Thr
                   165                 170                 175
   Thr Tyr Glu Arg Thr Phe Val Lys Lys Asp Ala Glu Thr Lys Glu Val
               180                 185                 190
   Leu Glu Gly Ala Gly Phe Lys Ile Ser Asn Ser Asp Gly Lys Phe Leu
           195                 200                 205
   Lys Leu Thr Asp Lys Asp Gly Gln Ser Val Ser Ile Gly Glu Gly Phe
       210                 215                 220
   Ile Asp Val Leu Ala Asn Asn Tyr Arg Leu Thr Trp Val Ala Glu Ser
   225                 230                 235                 240
   Asp Ala Thr Val Phe Thr Ser Asp Lys Ser Gly Lys Phe Gly Leu Asn
                   245                 250                 255
   Gly Phe Ala Asp Asn Thr Thr Thr Tyr Thr Ala Val Glu Thr Asn Val
               260                 265                 270
   Pro Asp Gly Tyr Asp Ala Ala Ala Asn Thr Asp Phe Lys Ala Asp Asn
           275                 280                 285
   Ser Ser Ser Asp Ile Leu Asp Ala Pro Ser Gly Ile Leu Pro His Thr
       290                 295                 300
   Gly Gly Thr Gly Thr Val Ile Phe Ala Ile Leu Gly Val Ala Leu Ile
   305 310 315 320
   Ala Phe Gly Ala Val Ala Tyr Arg Lys Arg Arg Asn Gly Phe
   325 330
<210> 3
   <211> 241
   <212> PRT
   <213> Lactobacillus rhamnosus
<400> 3
   Met Thr Lys Ser Phe Arg Pro Leu Val Ile Leu Thr Phe Cys Leu Ala
   1               5                   10                  15
   Leu Leu Val Ser Leu Ala Thr Thr Thr Leu Gln Gln Thr Gln Ala Ala
               20                  25                  30
   Thr Val Pro Thr Thr Val Asp Val Val Leu His Lys Leu Leu Phe Lys
           35                  40                  45
   Asp Thr Leu Pro Thr Gln Gln Ala Asn Asn Gly Thr Thr Lys Pro Asp
       50                  55                  60
   Phe Ser Gln Ala Asp Val Pro Leu Asn Gly Val Thr Phe Thr Val Tyr
   65                  70                  75                  80
   Asp Val Thr Ala Asp Phe Trp Gln Leu Val Ser Lys Asn Gly Gly Ala
                   85                  90                  95
   Ile Glu Val Ala Gln Thr Thr Leu Ser Gln Asp Ser Tyr Gln Pro Ala
               100                 105                 110
   Ser Ser Ser Leu Ile Ala Gln Val Val Thr Ala Gly Gln Gly Glu Ala
           115                 120                 125
   Tyr Phe Gly Asp Leu Pro Leu Arg Gln Gly Gln His Ala Ala Val Tyr
       130                 135                 140
   Leu Phe Lys Glu Thr Ala Ala Pro Lys Asn Ile Glu Ala Ser Gln Asn
   145                 150                 155                 160
   Leu Val Val Val Met Ser Ser Asn Leu Gln His Gly Asn Gln Ser Arg
                   165                 170                 175
   Ile Asp Leu Phe Pro Lys Asn Lys Met Val Ser Arg His Thr Asp Ala
               180                 185                 190
   Pro Lys Lys Val Pro Lys Lys Ile Arg Gln Leu Leu Pro Gln Thr Gly
           195                 200                 205
   Asp Thr Val Ala Ala Trp Leu Ser Val Leu Gly Leu Ile Ile Phe Ala
       210                 215                 220
   Thr Val Leu Ala Phe Asn Ile Lys Asn Gln Lys Ile Asn Lys Trp Glu
   225                 230                 235                 240
   Arg
<210> 4
   <211> 895
   <212> PRT
   <213> Lactobacillus rhamnosus
<400> 4
   Met Thr Ala Lys Val Ala Arg Thr Gly His Leu Phe Ala Val Leu Leu
   1               5                   10                  15
   Ile Leu Met Ser Met Leu Thr Gly Leu Val Thr Ser Gly Ser Ser Val
               20                  25                  30
   Val Thr Ala Thr Asp Asn Ile Arg Pro Thr Tyr Gln Thr Asp Ala Asn
           35                  40                  45
   Gly Thr Tyr Pro Thr Asn Ser Trp Gln Val Thr Gly Gln Gln Asn Val
       50                  55                  60
   Ile Asn Gln Arg Gly Gly Asp Gln Val Ser Gly Trp Asp Asn Asn Thr
   65                  70                  75                  80
   Ile Trp Asn Gly Asp Ala Thr Asp Thr Thr Asn Ser Tyr Leu Lys Phe
                   85                  90                  95
   Gly Asp Pro Asn Asn Pro Asp Tyr Gln Ile Arg Lys Tyr Ala Lys Glu
               100                 105                 110
   Thr Asn Thr Pro Gly Leu Tyr Asp Val Tyr Leu Asn Val Lys Gly Asn
           115                 120                 125
   Lys Gln Gln Asn Val Lys Pro Val Asp Ile Val Leu Val Val Asp Met
       130                 135                 140
   Ser Gly Ser Met Glu Ser Asn Arg Trp Gly Thr Asn Arg Ala Gly Ala
   145                 150                 155                 160
   Val Arg Thr Gly Val Lys Asn Phe Leu Thr Ser Ile Gln Asn Ala Gly
                   165                 170                 175
   Leu Gly Asn Tyr Val Asn Val Gly Leu Ile Gly Phe Ser Ser Pro Gly
   180 185 190
   Tyr Ile Gly Gly Lys Ser Gly Tyr Ile Ser Val Lys Leu Gly Lys Ala
   195 200 205
   Gly Asn Ala Ser Gln Gln Gln Ala Ile Asn Gly Ala Leu Ser Pro Arg
   210 215 220
   Phe Gln Gly Gly Thr Tyr Thr Gln Ile Gly Leu Arg Gln Gly Ser Ala
   225 230 235 240
   Met Leu Asn Ala Asp Thr Ser Gly Asn Lys Lys Met Met Ile Leu Leu
   245 250 255
   Thr Asp Gly Val Pro Thr Phe Ser Asn Glu Val Ile Asn Ser Glu Trp
   260 265 270
   Ile Asn Gly Thr Leu Tyr Gly Thr Asn Phe Gly Ser Ser Arg Asp Glu
   275 280 285
   Pro Gly Asn Thr Ala Arg Leu Arg Trp Pro Tyr Thr Asp Ser Ser Gly
   290 295 300
   His Tyr Ile Tyr Asp Thr Trp Pro Ala Thr Leu Gly Glu Ala Lys Ile
   305 310 315 320
   Ala Lys Asp Ser Gly Asn Glu Val His Ala Leu Gly Ile Gln Leu Ala
   325 330 335
   Asp Asp Asp His Tyr Met Thr Lys Glu Lys Ile Arg Gln Asn Met Gln
   340 345 350
   Leu Ile Thr Asn Ser Pro Asp Leu Tyr Glu Asp Ala Asp Ser Ala Asp
   355 360 365
   Ala Val Glu Ala Tyr Leu Asn Asn Gln Ala Lys Asp Ile Ile Lys Asn
   370 375 380
   Phe Asn Thr Val Thr Asp Gly Thr Ile Thr Asp Pro Ile Gly Thr Gln
   385 390 395 400
   Phe Gln Tyr Ala Asn Asn Gln Ala Thr Val Thr Ser Val Gly Lys Gln
   405 410 415
   Thr Val Pro Ala Ser Glu Leu Pro Ser Ala Ala Ile Gln Asp Gly Gln
   420 425 430
   Leu Thr Val Asn His Met Asn Leu Gly Gln Asp Gln Glu Val Gln Ile
   435 440 445
   His Tyr Gln Val Arg Ile Lys Thr Glu Asp Ala Gly Phe Lys Pro Asp
       450                 455                 460
   Phe Trp Tyr Gln Met Asn Gly Glu Thr Leu Leu Thr Pro Lys Ala Gly
   465                 470                 475                 480
   Ala Ala Ala Val Asp Phe Gly Ile Pro Ser Gly Arg Ala Pro Ala Thr
                   485                 490                 495
   Thr Val Tyr Val Gln Lys Gln Trp Arg Gln Leu Ser Asn Gln Ser Leu
               500                 505                 510
   Pro Asp Thr Leu Asn Val Thr Val Gln Arg Lys Val Ala Asp Gly Ser
           515                 520                 525
   Leu Asp Pro Asn Trp Gln Gln Thr Leu Val Leu Lys Lys Ala Asp Asn
       530                 535                 540
   Trp Lys Ala Ser Phe Thr Ala Pro Ala Tyr Asn Asn Gln Gly Gln Ser
   545                 550                 555                 560
   Phe Ser Tyr Val Val Lys Ser Glu Asp Ala Ser Gly Ile Asp Leu Ser
                   565                 570                 575
   Ser Phe Ile Ser Ser Gln Asn Met Asp Gln Gln Thr Ala Thr Leu Thr
               580                 585                 590
   Leu Thr Asn Gln Gln Tyr Gly Phe Gln Phe Gln Lys Lys Thr Thr Asp
           595                 600                 605
   Gly Thr Asp Leu Ser Ala Asp Gln Leu Lys Ala Met Gln Phe Asn Leu
       610                 615                 620
   Thr Gln Tyr Ser Asp Asn Ser Phe Gln Gln Ala Ser Lys Thr Asn Ala
   625                 630                 635                 640
   Ile Thr Ser Thr Asp Leu Gln Ala Leu Ala Pro Gly Tyr Tyr Gly Ile
                   645                 650                 655
   Gln Glu Ala Ala Ala Pro Thr Gly Tyr Gln Leu Asp Gly Thr Thr Tyr
               660                 665                 670
   Leu Phe Gln Leu Thr Ser Asp Gly Gln Trp Gln Tyr His Gly Thr Lys
           675                 680                 685
   Asp Asn Val Thr Ser Gly Ser Val Ile Asn Gly Gln Gln Thr Leu Asn
       690                 695                 700
   Pro Val Gly Asp Lys Ser Asp Asp Phe Thr Val Thr Gly Asp His Gln
   705                 710                 715                 720
   Gln Ile Leu Thr Leu Thr Lys Tyr Asp Glu Pro Lys Pro Ser Met Thr
                   725                 730                 735
   Leu Arg Val Ile Lys Gln Asp Asn Gln Ser Gln Tyr Leu Ala Gly Ala
               740                 745                 750
   Ala Phe Thr Leu Gln Pro Ser Ala Gly Glu Ala Glu Thr Ile Thr Ser
           755                 760                 765
   Ser Ala Thr Ser Glu Gly Gln Ala Phe Ala Thr Lys Leu Val Ala Asp
       770                 775                 780
   Gly Thr Tyr Thr Met Ser Glu Thr Lys Ala Pro Asp Gly Tyr Gln Ser
   785                 790                 795                 800
   Asn Pro Ala Lys Ile Ala Ile Gln Val Ala Thr Thr Gly Lys Glu Ala
                   805                 810                 815
   Thr Val Thr Ile Asp Gly Glu Ala Leu Lys Pro Gly Glu Ser Lys Asn
               820                 825                 830
   Gly Tyr Thr Leu Ala Ile Asp Gly Ser Thr Ile Thr Leu Gln Ala Ile
           835                 840                 845
   Asn Gln Pro Leu Ala Ile Leu Pro His Thr Gly Gly Gln Gly Tyr Gln
       850                 855                 860
   Arg Leu Leu Gly Ile Ala Leu Gly Leu Ile Ser Ala Ala Phe Leu Leu
   865                 870                 875                 880
   Leu Leu Val Val Leu Ile Lys Arg Arg Val Val Lys Gln His Asp
                   885                 890                 895
<210> 5
   <211> 274
   <212> PRT
   <213> Lactobacillus rhamnosus
<400> 5
   Met Thr Lys Lys Ala Ser Gly Thr Ser Arg Leu Leu Arg Trp Phe Val
   1               5                   10                  15
   Ile Leu Leu Phe Thr Ala Gly Ala Ala Cys Phe Cys Tyr Pro Phe Ala
               20                  25                  30
   Ala Thr Ala Ile Asn Glu Leu Leu Leu Thr Ser Arg Arg Ala Ala Ala
   35 40 45
   Gln Gln Glu Ala Lys Gln Asn Ala Ala Ala Gln Asp Glu Gln Arg Ala
   50 55 60
   Ala Glu Asn Arg Ala Leu Ala Gln Thr Gly Leu Arg Pro Gly Gln Asp
   65 70 75 80
   Pro Phe Gln Ser Arg Gln Lys Phe Asn Gln Ala Tyr Val Lys Arg His
   85 90 95
   Leu Ile Gly Arg Val Val Ile Pro Lys Leu Ala Val Asp Leu Pro Leu
   100 105 110
   Phe Asp Thr Thr Asn Asn Thr Leu Leu Asp Gln Gly Ala Val Val Leu
   115 120 125
   Pro Gly Thr Ser Tyr Pro Arg Gly Gly Lys Asn Thr His Thr Val Val
   130 135 140
   Ser Ala His Gly Gly Leu Pro Thr Lys Arg Phe Phe Thr Asp Leu Ser
   145 150 155 160
   Lys Leu Lys Arg Gly Gln Lys Phe Phe Leu Gln Val Asn Gly Lys Lys
   165 170 175
   Met Ala Tyr Gln Val Phe Arg Ile Lys Thr Val Arg Pro Asp Glu Thr
   180 185 190
   Gln Ser Leu Arg Ile Glu Pro Gly Arg Asp Leu Ala Thr Leu Met Thr
   195 200 205
   Cys Thr Pro Tyr Met Ile Asn Ser His Arg Leu Leu Val Thr Gly Lys
   210 215 220
   Arg Val Pro Tyr Thr Glu Ser Leu Glu His Ala Ala Glu Ser Ala Asp
   225 230 235 240
   Arg Trp Arg Leu Trp Leu Ser Ile Ala Val Val Val Gly Val Leu Gly
   245 250 255
   Leu Ala Leu Leu Ser Phe Tyr Leu Ala Arg Arg Tyr Leu Arg Arg Pro
   260 265 270
   Arg Ala
<210> 6
   <211> 517
   <212> PRT
   <213> Lactobacillus rhamnosus
<400> 6
   Met Gln Val Thr Phe Lys Lys Ile Gly His Ser Leu Leu Ala Ala Leu
   1               5                   10                  15
   Met Leu Met Ser Phe Leu Leu Pro Leu Leu Ser Ala Gly Lys Pro Val
               20                  25                  30
   His Ala Ala Thr Thr Thr Val Asp Phe Thr Leu His Lys Ile Glu Gln
           35                  40                  45
   Thr Ser Asp Glu Gln Ile Gln Asn Thr Gly His Asp Leu Gly Leu Thr
       50                  55                  60
   Gly Arg Lys Pro Val Gln Gly Ala Gln Phe Lys Ile Phe Asn Val Thr
   65                  70                  75                  80
   Asp Ala Phe Tyr Gln Leu Leu Glu Asn His Asp Lys Thr Thr Ala Ala
                   85                  90                  95
   Ser Met Ile Ser Gln Asn Leu Gly Gln Tyr Val Asn Leu Gln Asp Pro
               100                 105                 110
   Asn Ala Ala Thr Val Thr Thr Asp Ala Asp Gly Leu Ala Ala Phe Lys
           115                 120                 125
   Gly Leu Ala Ala Lys Thr Asn Gly Arg His Ser Val Tyr Ala Phe His
       130                 135                 140
   Glu Ala Val Thr Pro Gln Pro Tyr Gln Lys Ala Ala Asp Met Ile Val
   145                 150                 155                 160
   Ser Leu Pro Val Arg Gln Asp Asp Gly Ser Asp Leu Thr Asn Ile His
                   165                 170                 175
   Leu Tyr Pro Lys Asp Ser Leu Val Thr Lys Asn Leu Thr Glu Ile Asn
               180                 185                 190
   Glu Gln Ala Val Ala Thr Lys Asp Leu His Asp Val Ala Val Gly Asp
           195                 200                 205
   Val Leu Thr Tyr Gln Val Gln Phe Gln Ile Pro His Asp Ile Gly Ala
       210                 215                 220
   Leu Ala Asp His Ser Gln Asp Thr Phe Lys Tyr Asn Gln Phe Lys Val
   225                 230                 235                 240
   Leu Asp Tyr Met Thr Lys Glu Gly Leu Thr Phe Lys Ala Leu Thr Ala
   245 250 255
   Ile Thr Val Asp Gly Gln Asp Ile Leu Lys Ala Leu Thr Gly Lys Met
   260 265 270
   Ala Phe Met Ser Ser Asn Asp Ala Ala Trp Gln Gln Thr His Asn Tyr
   275 280 285
   Pro Phe Gly Phe Glu Leu Asp Phe Leu Gly Gly Thr Asp Pro Asp Ala
   290 295 300
   Val Arg Asn Leu Leu Thr Gln Tyr Ala Gly Lys Arg Val Thr Val Ala
   305 310 315 320
   Tyr Thr Gly Ile Val Asn Glu Lys Met Ile Pro Asp Gln Lys Val Gly
   325 330 335
   Asn Thr Ala Glu Val Ser Phe Asp Pro Asp Ser Lys Ile Thr Val Asn
   340 345 350
   Gly Pro Glu Ile Gln Thr Gly Gly Ile Arg Phe Phe Lys His Glu Ala
   355 360 365
   Gly Ser Ser Lys Ser Leu Ala Asn Ala Thr Phe Ile Leu Gln Arg Met
   370 375 380
   Asn Gly Asn Val Arg Glu Tyr Ala Val Leu Glu Gly Val Asn Gly Met
   385 390 395 400
   Ala Gly Thr Tyr Gln Pro Thr Lys Ile Thr Trp Thr Thr Asn Gln Asp
   405 410 415
   Ala Ala Thr Arg Leu Lys Thr Ser Gly Ala Glu Thr Ala Asn Leu Thr
   420 425 430
   Ile Gln Gly Leu Leu Pro Gly Arg Tyr Thr Leu Val Glu Thr Ala Ala
   435 440 445
   Pro Glu Gly Tyr Glu Ile Leu Asp Pro Thr Thr Asp Phe Glu Val Ile
   450 455 460
   Ala Gly Thr Trp Gly Thr Lys Thr Ile Arg Ile Ala Asn Thr Pro Val
   465 470 475 480
   Asn Gln Leu Leu Pro Met Thr Gly Gly Ile Gly Leu Phe Ala Phe Leu
   485 490 495
   Met Ile Gly Ala Ile Leu Met Gly Gly Gly His Leu Met Lys Lys Lys
   500 505 510
   Thr Ser Lys Lys Val
   515
<210> 7
   <211> 451
   <212> PRT
   <213> Lactobacillus rhamnosus
<400> 7
   Met Arg Arg Phe Tyr Trp Trp Leu Val Pro Leu Leu Leu Leu Ile Gly
   1               5                   10                  15
   Ile Val Leu Gly Asn Thr Pro His Trp Val His Ala Ala Asp Gln Thr
               20                  25                  30
   Ala Glu Ile Val Ile His Lys Arg Ile Tyr Arg Asp Ile Arg Gln Pro
           35                  40                  45
   Glu Asp Val Trp Tyr Glu Asn Asp Gly His Arg Ile Asp Pro Asn Asn
       50                  55                  60
   Pro Asp Lys Asp Gly Tyr Lys Leu Leu Ser Lys Thr Ser Gly Leu Asn
   65                  70                  75                  80
   Gly Ala Asn Phe Glu Val Tyr Asp Ala Ser Ser Leu Leu Lys Pro Asn
                   85                  90                  95
   Met Thr Pro Glu Ala Ile Arg Ala Leu Val Asp Arg Tyr Gln Asn Met
               100                 105                 110
   Thr Arg Lys Gln Ala Leu Lys Phe Ala Arg Ala Asn Leu Lys Leu Ala
           115                 120                 125
   Gly Gln Gly Asn Lys Gly Ile Gly Leu Met Asn Thr Lys Asn Asp Pro
       130                 135                 140
   Thr Leu Gly Glu Asp Gly Ile Ser Arg Ile Thr Val Ser Val Asp Gln
   145                 150                 155                 160
   Gln Ala Pro Thr Lys Ala Tyr Leu Met Ile Glu Val Ala Pro Asp Pro
                   165                 170                 175
   Ser Thr Glu Leu Asn Val Asp Leu Glu Arg Lys Ser Ser Pro Met Leu
               180                 185                 190
   Val Val Phe Pro Val Thr Asp Pro Ile Ser Gly Asn Pro Leu Gln Thr
           195                 200                 205
   Ile His Leu Tyr Pro Lys Asn Val Gly Tyr Val Arg Asp Pro Tyr Phe
   210 215 220
   Phe Lys Phe Gly Val His Pro Asp Gly Thr Ser Lys Arg Leu Ala Gly
   225 230 235 240
   Ala Ile Phe Ala Ile Tyr Arg Ile Glu Asn Gly Lys Lys Leu Tyr Leu
   245 250 255
   Asp Met Ser Pro Val Thr Asp Leu Arg Asn Lys Trp Val Ser Thr Thr
   260 265 270
   Asp Pro Leu His Asp Asp Arg Val Asn Lys Phe Val Ser Asp Gln Asp
   275 280 285
   Gly Leu Val Asn Thr Gly Glu Arg Phe Leu Pro Ala Gly Glu Tyr Phe
   290 295 300
   Phe Glu Glu Leu Gln Gly Val Pro Gly Tyr Glu Val Asp Ala Lys Ser
   305 310 315 320
   Arg Ala Ile Lys Ile Glu Ile Pro Asp Ser Trp Glu Asp Glu Asp Gly
   325 330 335
   Asn Arg Arg Phe Val Leu Ile Asp Gly Gln Pro Met Gln Glu Asn Phe
   340 345 350
   Gly Gly Val Val Thr Pro Glu Met Ile Ser Ser Gly Tyr Pro Arg Val
   355 360 365
   Tyr Asn Tyr Ala Asp Lys Gln Ala Ser Thr Thr Gly Asp Gln Thr Ala
   370 375 380
   Gly Pro Ser Thr Thr Gln Leu Gly Asn His Gly Gln Asp Thr Asn Gly
   385 390 395 400
   Thr Gly Thr Arg Thr Pro Lys Arg Gln Ser Gly Tyr Leu Pro Ala Met
   405 410 415
   Ser Asp Trp Arg Asn Leu Arg Phe Val Leu Leu Gly Ser Leu Leu Leu
   420 425 430
   Leu Leu Ala Thr Tyr Phe Phe Ile Lys Asn Lys Lys Ala Arg His His
   435 440 445
   Ala Cys Lys
   450
<210> 8
   <211> 983
   <212> PRT
   <213> Lactobacillus rhamnosus
<400> 8
   Met Pro Arg Lys Trp Ile His Met Leu Met Leu Leu Leu Met Leu Val
   1               5                   10                  15
   Thr Gln Ile Gly Ser Ala Ala Val Pro Val Ala Lys Ser Ala Gln Thr
               20                  25                  30
   Asn Pro Lys His Asp Val Arg Asp Ala Ser Val Gln Pro Ser Thr Arg
           35                  40                  45
   Pro Ala Ala Ser Glu Ala Ala Glu Phe Asp Leu Glu Ala Ala Ala Ser
       50                  55                  60
   Ala Pro Ser Thr Ser Ala Ala Ala Lys Gln Thr Thr Ser Lys Ala Arg
   65                  70                  75                  80
   Gln His Ile Lys Leu Glu Ala Gly Lys Ser Trp His Gly Asp Gly His
                   85                  90                  95
   Thr Leu Thr Tyr Asn Val Asp Ile Gln Arg Ser Glu Ile Gln Val Lys
               100                 105                 110
   Leu Ile Leu Ala Lys Pro Gln Asp Gln Thr Gly Gln Gln Val Val Lys
           115                 120                 125
   Phe Ala Asn Ala Gln Gly Phe Thr Ser Gln Pro Ala His Thr Asn Gly
       130                 135                 140
   Glu Ile Thr Arg Arg Leu Ala Glu Lys Thr Ala Glu Lys Gly Glu Tyr
   145                 150                 155                 160
   Leu Leu Thr Lys Lys Leu Pro Asp Thr Lys Gln Gln Ala Ala Ser Val
                   165                 170                 175
   Lys Leu Ser Leu Asp Gly Phe Asn Asp Ala Ala Gln Val Leu Ala Leu
               180                 185                 190
   Asp Val Asp Leu Gln Leu Pro Ala Arg Leu Ala Asn Asp Asp Val Gln
           195                 200                 205
   Glu Pro Ala Ala Leu Ser Lys Asp Ala His Ser Leu Ile Leu Pro Pro
       210                 215                 220
   Ser Ala Leu Gly Thr Ile Lys Ile His Ala Thr Lys Ala Asp Gly Ala
   225                 230                 235                 240
   Ala Leu Ser Asp Glu Glu Ala Gln Ile Tyr Arg Lys Pro Asn Ser Ser
                   245                 250                 255
   Thr Arg Ser Lys Tyr Gly Ser Arg Trp Ala Met Glu Asn Gly Val Ser
               260                 265                 270
   Ser Asp Tyr Val Ser Arg Ser Asp Ala Thr Ala Ile Ile Phe Lys Asp
           275                 280                 285
   Ala Val Gln Asn Pro Ser Gly Pro Ser Asn Leu Leu Asp Ala Lys Ile
       290                 295                 300
   Lys Val Asp Ile Asp His Val Gly Ser Ala Ser Asp Leu Asp Gly Asn
   305                 310                 315                 320
   Arg Phe Glu Ile Gly Ala Tyr Val Glu Leu Thr Gly Ile Arg Val Arg
                   325                 330                 335
   Pro Val Glu Trp Gly Thr Thr Pro Gln Asp Val Gly Ile Asp Phe Ser
               340                 345                 350
   Asn Asn Phe Phe Ser Gly Met Ser Phe Ala Asn Val Leu Tyr Tyr Asp
           355                 360                 365
   Trp Arg Val Ile Phe Tyr Asp Lys Ala Thr Arg Gln Arg Leu Asn Phe
       370                 375                 380
   Ile Pro Gln Ser Glu Ala Asn Gln Asn Ser Thr Leu Thr Phe Thr Ser
   385                 390                 395                 400
   Leu Asn Pro Gly Glu Phe Val Trp Thr Glu Gln Ala Gly Met Thr Pro
                   405                 410                 415
   Thr Tyr Asp Asp Arg Phe Ile Thr Asp Trp Gln Phe Glu Glu Gly Thr
               420                 425                 430
   Trp Ile Thr Ser Asp Lys Ala Thr Phe Glu Thr Glu Lys Leu Gly Ala
           435                 440                 445
   Arg Gly Lys Glu Gln Arg Gly Tyr Thr Ser Gln Thr Trp Gly Asn Trp
       450                 455                 460
   Val Asp Pro Ile Asp His Glu Asn Met Thr Glu Trp Glu Asp Arg Leu
   465                 470                 475                 480
   Gly Ala Pro Thr Phe Gly Arg Gly Ala Val Ala Phe Thr Leu Asn Gly
                   485                 490                 495
   Thr Ser His Thr Phe Arg Arg Gly Thr Tyr Ser Asn Gly Gly Gly Thr
   500 505 510
   Trp Val Ala Asn Gly Ser Gly Gln Ile Glu Leu Ile Asp Pro Asn Val
   515 520 525
   Thr Asn Asn Lys Ser Val Ser Ala Asn Ala Glu Ala Gly Gly Gly Ala
   530 535 540
   Glu Glu Asp Lys Thr Gly Thr Ile Trp Thr Ala Asn Asp Leu Asp Asp
   545 550 555 560
   Gln Val Val Asn Gln His Tyr Asn Gly Glu Pro Phe Tyr Tyr Tyr Ile
   565 570 575
   Asn Gln Glu Val Tyr Ser Met Gly Asp Tyr Val Val Lys Pro Thr Lys
   580 585 590
   Ile Val Val Thr Asp Leu Leu Pro Glu His Val Glu Leu Ile Pro Asp
   595 600 605
   Asn Asn Asn Ser Pro Pro Thr Tyr Gln Lys Ala Phe Gln Leu Phe Asn
   610 615 620
   Ala Thr Asp Pro Asp Ala Val Gly Gln Asp Arg Lys Met Thr Leu Thr
   625 630 635 640
   Glu Asp Val Ser Asp Phe Val Val Thr Gln Glu Gly Asp Arg Gln Arg
   645 650 655
   Ile Thr Leu Thr Ile Gly Arg Glu Asp Val Gln Lys Ile His Phe His
   660 665 670
   Ser Gly Phe Phe Ser Leu Arg Leu Lys Val Arg Pro Thr Lys Asp Pro
   675 680 685
   Asp Thr Leu Thr Lys Arg Leu Thr Leu Val Asn Lys Ala Thr Val Lys
   690 695 700
   Phe Phe Asp Thr Glu Glu Arg Tyr Ser Lys Glu Thr Asn Ala Val Gln
   705 710 715 720
   Val His Leu Asp Pro Ala Gly Arg Phe Pro Ala Glu Phe Thr Lys Lys
   725 730 735
   Asn Gln Tyr Gly Ala Val Leu Pro Gly Ser Arg Phe Val Leu Lys Gln
   740 745 750
   Gly Asp Thr Gln Leu Gln Thr Ala Thr Ala Asp Ser Gln Gly Lys Val
   755 760 765
   Ser Phe Gly Thr Leu Lys Pro Gly Asp Tyr Gln Val Ser Glu Ile Ala
   770 775 780
   Ala Ala Gly His Glu Leu Gln Ala Glu Phe Asp Leu Lys Val Ala Ala
   785 790 795 800
   Asp Gly Thr Val Thr Val Gly Arg Asn Gly Glu Ile Trp Pro Asp Thr
   805 810 815
   Thr Val Ile Asn Gln Leu Lys Pro Thr Glu Leu Glu Leu Ile Lys Ile
   820 825 830
   Glu Lys Gly Lys Asn Lys Leu Ala Asn Ala Ser Phe Ala Leu Tyr Arg
   835 840 845
   Gly Asp Gln Thr Thr Pro Val Ala Gln Gly Thr Thr Asp Glu Asn Gly
   850 855 860
   Gln Leu Arg Phe Thr His Gln Leu Thr Pro Gly Thr Tyr Arg Leu Thr
   865 870 875 880
   Glu Thr Lys Ala Pro Ala Gly Phe Asp Arg Leu Asn Gly Ser Phe Thr
   885 890 895
   Phe Lys Ile Asn Ala His Gly Thr Met Val Asp Leu Ala Tyr Ser Gly
   900 905 910
   Ser Asp Leu Ser Ser Asp Glu Tyr Gly Phe Glu Phe Ile Pro Asp Ala
   915 920 925
   Glu Asp Lys Leu Asn Arg Ile Arg Phe Thr Leu Thr Asn His Ser Leu
   930 935 940
   Glu Thr Leu Leu Pro Lys Thr Gly Gly Ser Gly Ile Leu Leu Phe Leu
   945 950 955 960
   Met Val Ala Ile Ser Ala Cys Gly Gly Gly Trp Leu Leu Tyr Leu Tyr
   965 970 975
   Leu Lys Arg Lys Glu Ala Arg
   980
<210> 9
   <211> 1080
   <212> DNA
   <213> Lactobacillus rhamnosus
<400> 9
   gtgacaaaac gaacacgtcg acctttagac ttgattgata ttgtgattgg atgtcttctt      60
   ttagcgggtt ttggtgtttt atgctatcca tttgcaagtg atgcttacgt ttcttaccaa     120
   aatcagcaag tcatcgacag gtatcgacaa caagaagcgc ggaagaatca gatggtgttg     180
   cggcgggaat ataacgacta tcagcaaaaa aataaacagt tggcagcaag tcaacaagtg     240
   cccggcgttg ccagttttaa tcatgctgtt aatgatcaag gaaccgcaaa aacagcagcc     300
   aaacgcaatc aacaaatctt gactcggcag acagttgctc agttgacgat tcccaaaatt     360
   ggccttagtc tgccggtttt tgatcataca agcgattggc ttctacaatt tggcgcctgt     420
   ttattggatg gtacaagtta tccaactggt ggtaaaaata cccatgctgt catttcagcg     480
   catcgtggtg tgccaaacgc tgaacttttt acccgagtac cagcgttaaa aaaaggcgac     540
   aagtttttta ttagcatagg caatcataaa ttggcttacc aagtctttaa gcgccaggtt     600
   attgagccaa gtgatacccg gcagctaaga attgtgccgg gacaggatct tgtgacctta     660
   atgacctgca cgccttatat gatcaattct catcgattgt tgataacggg tcgccgaatt     720
   ccttacgtta aggcagatga agaggcttca agttgggcgg tttggtggaa caaattaaag     780
   ctaatagtcg cacttttagg cgcggtgatc attttaggcg tgatcggttt cgtaatgcgc     840
   agtttgatgc ttggccgaaa gcattatttg ctggaagtac cggctgaagc cacacaagtc     900
   gtggtgaaac gaggtcgaca tatacattct tttaaatcag atcaaactgg ggtgactgac     960
   atcagcctgc ctggtaatca ttatcgagtc gcaattgtca caccgcttgg ccggactaag    1020
   tacaaggctt atgtcaaaaa aattcgggat aaaagctttc aattaaaaga atatcattaa    1080
<210> 10
   <211> 1005
   <212> DNA
   <213> Lactobacillus rhamnosus
<400> 10
   atgaaaaaga caattgccaa gaaagtgctg acattaacca gcacgatcct aatgacatta      60
   ctgatggttc tcgggtttaa tggcactcgg gttcaagcag atacgaatga tacgacaaca     120
   caaaacgttg tccttactaa atacgggttt gacaaagatg ttactgccat tgatcgtgcg     180
   actgatcaaa tttggaccgg cgatggtgct aagcctttac aaggcgttga tttcaccatt     240
   tacaacgtga cagccaatta ttgggcatcg cctaaggatt ataaaggcag ttttgatagt     300
   gctccggttg ccgcaaccgg tacgactaat gacaaggggc aactaaccca agcattacct     360
   atccaatcaa aagatgccag tggtaagact cgtgctgctg tctatctttt ccatgaaacc     420
   aatccgcgag ctggttataa cacgtctgcc gatttctggt taaccttacc agccaaggca     480
   gcagccgacg ggaatgtcta tgtctaccca aagaatgttc aaaagaccac ctatgagcgc     540
   acttttgtta agaaagatgc tgagactaaa gaagtgcttg aaggagccgg ctttaagatt     600
   agcaatagtg atggcaagtt tttgaagttg acagataaag atggtcaaag cgtcagcatc     660
   ggcgaaggat ttatcgatgt attggccaat aactatcgat tgacgtgggt tgctgaaagc     720
   gatgctactg ttttcacgtc tgataagagc ggtaagtttg gcttaaatgg atttgctgat     780
   aacaccacaa cttacacggc agttgaaaca aacgtgccgg atggttatga tgctgctgcc     840
   aatacagact ttaaagctga taattcgtct agcgacattc tagatgcacc aagcggtatt     900
   ctgccacaca ctggtggtac tggcacagtc atttttgcga ttttgggcgt tgccttaatt     960
   gcatttggag cagttgccta tcgcaagcgc cgcaatggtt tctaa                    1005
<210> 11
   <211> 726
   <212> DNA
   <213> Lactobacillus rhamnosus
<400> 11
   atgactaaat ccttccgtcc gttagtgatt ttgacctttt gcttggcact actagtcagt     60
   ttggcaacga caacgttgca gcagacacag gcggcaactg tgccgaccac tgttgatgtt    120
   gtgttgcata agctgttgtt taaagatacc ttgccaactc aacaagcaaa taacgggaca    180
   acaaaacccg acttttcgca ggcagatgtg ccgttaaacg gtgtgacgtt cacagtttat    240
   gacgtgaccg ctgacttttg gcagcttgtc tccaaaaatg gcggtgcgat tgaggtagca    300
   caaacgacgt tgagtcaaga tagctatcag cctgctagct ccagccttat cgcacaggtt    360
   gtgacggctg gtcagggaga agcgtacttt ggcgatttac cactccgaca ggggcagcat    420
   gctgcggttt atctttttaa agaaacggcg gcacctaaga atattgaagc cagtcagaat    480
   cttgtggttg tcatgtcaag caaccttcaa catgggaatc aatcacgcat tgatttattt    540
   cctaagaaca aaatggtaag tcgtcacacc gatgccccca aaaaagttcc aaagaaaata    600
   cgtcaattgt tgccacaaac gggtgataca gttgcagctt ggctttcagt gctcgggttg    660
   ataatcttcg cgacagtact tgcttttaac ataaaaaacc aaaaaattaa taagtgggag    720
   agataa                                                               726
<210> 12
   <211> 2688
   <212> DNA
   <213> Lactobacillus rhamnosus
<400> 12
   atgacagcta aagtggcgag aactgggcat ttgttcgcgg tcttattgat tttgatgagt     60
   atgttaacag gcttagtgac aagtggcagt tcagttgtga cagccactga taacattcgc    120
   ccaacctatc aaaccgatgc taatggtacc tatccgacaa attcgtggca ggtcacggga    180
   caacaaaatg taatcaatca acgtggcggg gatcaagttt cagggtggga taataataca    240
   atatggaatg gtgatgcgac tgataccacg aactcttacc tgaaatttgg tgaccccaat    300
   aatccggatt atcagattcg aaaatatgct aaagagacga atacccctgg attgtacgac    360
   gtttatttga acgtcaaagg caataaacag caaaatgtga agcctgtaga tattgtctta 420
   gttgttgata tgtctgggtc aatggagtca aacagatggg gcacgaatcg agctggtgct 480
   gttcgtactg gcgttaagaa tttcttgact tctattcaaa acgccggtct gggtaattac 540
   gtcaatgttg gtttaattgg gttttctagt cctggttata tcggtggcaa atcgggttat 600
   attagtgtca aattaggcaa agcaggtaat gccagccagc aacaagcgat taatggtgca 660
   ttgagtccaa ggtttcaagg gggtacgtat acgcagattg gtttgcggca aggatcagcc 720
   atgctgaatg cggacaccag tggcaataaa aaaatgatga ttttgttaac tgatggcgtg 780
   ccgacttttt ctaacgaggt gataaattca gagtggataa atggtacatt gtatggcact 840
   aattttggat ccagcagaga tgaaccaggg aacaccgcac gacttcgatg gccatacacc 900
   gatagttcag gtcattatat atatgatact tggccagcaa cattgggtga ggccaagata 960
   gcaaaggata gtggtaatga ggtgcacgcg ttaggcatcc aactggctga cgacgaccac 1020
   tacatgacga aagaaaaaat acgccaaaac atgcagctta ttaccaattc accggattta 1080
   tacgaagatg ctgatagtgc cgatgctgtt gaggcttatt tgaacaatca ggcaaaggac 1140
   attatcaaaa actttaatac tgtcaccgac ggcacgatca cagacccgat tggtacgcaa 1200
   tttcaatatg cgaacaacca ggcgaccgtt acgagtgtcg gcaagcaaac tgtgccagca 1260
   agtgagttgc caagtgcggc gatccaagat ggtcaattga cggtgaatca catgaacttg 1320
   ggtcaggatc aggaagttca aatccattat caagtacgga tcaaaacaga ggatgctggc 1380
   ttcaagcctg atttttggta ccaaatgaat ggtgaaacat tgttgacacc aaaagcgggc 1440
   gctgccgctg ttgactttgg gattccttca ggcagggcac cagcaactac agtttatgtg 1500
   cagaagcaat ggcgccagtt aagcaatcaa tcgttaccgg atacgctcaa cgtcacggtg 1560
   cagcgaaaag tggctgacgg ttcgcttgat ccaaattggc aacagacctt agtccttaaa 1620
   aaagctgata actggaaagc tagctttacg gcacctgcgt ataacaatca gggtcaaagt 1680
   ttttcatatg tcgttaagag tgaagatgcc tcgggaattg atttgagttc gtttatcagt 1740
   tctcaaaata tggatcagca aacagcaacg ttgactttga caaatcagca gtatggtttt 1800
   cagtttcaga aaaaaacaac cgatggtact gatttatcag cagatcagtt gaaggccatg 1860
   cagtttaact taacccagta cagcgataac agttttcagc aggcatccaa aaccaacgcc 1920
   atcacgtcaa cggatctgca ggcactagcg ccagggtatt acggtattca ggaagctgca 1980
   gcacctacag gttatcaact tgatgggaca acgtatcttt ttcagctaac gtctgatggg 2040
   caatggcaat accatggcac aaaggacaat gtgacatcag ggagtgttat taatggccag 2100
   cagactttga atcctgttgg tgataagtca gatgatttta cggtgaccgg ggatcaccag 2160
   caaattctga cgctaacgaa atatgatgaa ccaaagccat ccatgacttt gcgggtcatc 2220
   aaacaggata atcaaagcca atatcttgca ggtgcagcgt tcaccctgca accaagtgct 2280
   ggcgaagctg agacgataac atcatcggcg acatctgagg gacaagcgtt tgcgacaaaa 2340
   ttagttgcag atggtaccta tacgatgtca gaaacaaaag caccagatgg ctatcaaagc 2400
   aatcctgcaa agattgccat tcaggtagct acgactggta aagaggcaac cgtcacgatt 2460
   gacggtgagg cattgaagcc gggcgaaagt aagaacggat acacattagc gattgatggc 2520
   agcacgatca ctttgcaggc gattaatcag ccacttgcaa ttttgccgca tacaggtggt 2580
   cagggctatc agcgattgct tggtatcgca ctgggattga tcagcgcagc gttcctttta 2640
   ttactggttg ttttgataaa gcgacgggtg gtgaagcaac atgactaa 2688
<210> 13
   <211> 825
   <212> DNA
   <213> Lactobacillus rhamnosus
<400> 13
   atgacaaaaa aagcgtcggg gacaagtcgg ctgttacgct ggttcgtcat cttacttttt 60
   actgcgggag ccgcgtgttt ctgctatccg ttcgcggcaa cggctattaa tgaattgcta 120
   ctaaccagtc gccgagcagc agcacagcaa gaagccaagc aaaatgccgc cgcccaagat 180
   gagcaacggg cagcggagaa ccgtgcactt gcccagactg gtttgcgtcc gggacaggat 240
   ccgtttcaaa gtaggcagaa atttaaccaa gcctatgtga aacggcatct gatcgggcga 300
   gtggttatcc cgaaattagc ggttgatctg cccctttttg acaccaccaa caacacgctg 360
   ttagatcaag gggcagtggt gttaccaggt actagctatc cgcggggagg caagaacacg 420
   catacagttg tttcggcaca cggcggcttg cccaccaaac gctttttcac cgatctgagc 480
   aagttgaaac gagggcagaa gttctttctc caagtcaacg gcaaaaagat ggcgtatcag 540
   gtctttcgga tcaaaaccgt gcggccggat gaaacccaga gcttgcgcat tgaaccggga 600
   cgcgatttgg ccacattaat gacctgtacc ccgtatatga tcaactccca ccgcctgtta 660
   gtgaccggca aacgggtacc ttataccgaa tcacttgagc acgccgccga gtctgctgat 720
   cgctggcgct tgtggttaag tatcgcggtt gtcgtcggag tgctgggatt ggcattgctg 780
   agtttctatc tggctcggcg ctatcttcgc cgaccgcggg cgtaa 825
<210> 14
   <211> 1554
   <212> DNA
   <213> Lactobacillus rhamnosus
<400> 14
   atgcaagtaa cgtttaaaaa gatcgggcac agtctcttgg cagcgctgat gctcatgagc     60
   ttccttctac cactgcttag tgcgggcaaa cccgtacatg ccgcgacaac gactgtggat    120
   ttcacgctgc acaaaatcga acaaaccagt gacgaacaga ttcaaaatac cggccacgac    180
   cttggactga ccgggcgtaa accggtgcaa ggcgctcaat ttaaaatttt caacgtgacg    240
   gacgcctttt accaattact ggaaaatcat gataagacaa ccgctgcgag catgatatcg    300
   caaaacctgg gtcagtatgt gaatctccag gatcctaatg cagcaactgt cacgactgat    360
   gcagacggct tggcggcatt caaaggatta gccgccaaaa ccaatggccg gcatagcgtg    420
   tacgcatttc acgaagccgt gaccccgcaa ccgtatcaaa aagcagcaga tatgatcgtg    480
   agtctgccag tgcggcaaga cgatggatcg gatctgacca acattcatct ttatcctaaa    540
   gacagtcttg ttaccaaaaa tctgacggaa atcaatgaac aagcggtggc aacaaaagat    600
   ctccatgatg tcgcggttgg cgatgtgctc acgtatcagg ttcagttcca gattccgcat    660
   gatattggcg cgctggctga tcacagtcaa gacactttta agtacaacca atttaaagtg    720
   ctggattata tgaccaagga aggccttact tttaaggcat tgacggcaat cacggttgac    780
   ggtcaggaca ttttaaaggc attaaccgga aaaatggcct tcatgagttc taatgacgca    840
   gcttggcaac aaacacacaa ctatccattc gggtttgaac tggactttct aggcgggacc    900
   gatcccgatg cggtacgaaa cctgttgacc caatatgccg gcaaacgcgt gaccgttgcc    960
   tacaccggaa tcgtcaatga gaaaatgatc ccagaccaaa aagtcggtaa cacggctgaa   1020
   gtgagctttg atcctgacag caagattacc gtcaatggtc cggaaatcca gactggcggg   1080
   attcggttct tcaaacacga agccggatct tccaaaagtt tggccaacgc gactttcatc   1140
   ttacagcgaa tgaacggcaa tgtgcgcgaa tatgcagttc ttgaaggcgt taacggtatg   1200
   gccggaacct accaaccgac caagattacc tggacaacga atcaagacgc ggcaacgaga   1260
   ctcaaaacca gtggagccga gacagccaac ttaaccattc aagggctgtt gccagggcga   1320
   tataccttgg ttgaaaccgc ggcaccagaa ggctatgaaa tccttgatcc gacaacagat   1380
   tttgaagtca ttgccggtac ttggggtacg aaaacgattc gcatcgccaa cacgccggtg   1440
   aatcaattat tgccgatgac aggcggaatc ggactcttcg ccttcctgat gatcggggcc   1500
   atcttaatgg gtggcggtca cctaatgaag aaaaagacca gcaagaaagt ctaa          1554
<210> 15
   <211> 1356
   <212> DNA
   <213> Lactobacillus rhamnosus
<400> 15
   atgcgacgat tttattggtg gcttgtcccg ttgcttctat tgattggtat cgtgcttggc 60
   aacacaccac attgggttca cgcggctgat caaactgccg agattgtgat ccataagcga 120
   atttatcggg atattcgcca accggaagac gtttggtatg aaaatgacgg tcatcggatt 180
   gacccgaata acccggataa agatggctac aaattattaa gcaaaaccag cgggctgaat 240
   ggtgctaact ttgaggtcta tgatgccagc tccttattga aaccgaatat gacgcctgaa 300
   gcaattcggg ctttagttga tcgttatcag aatatgacgc gtaagcaagc actgaaattt 360
   gcgcgggcca acctgaaatt agccggtcaa gggaacaaag gtatcgggct gatgaataca 420
   aaaaacgatc caacactcgg tgaagatggg atcagccgaa taaccgtttc tgtcgatcaa 480
   caggcaccga ctaaagctta tctgatgatc gaggtggcac cggatccttc aaccgaactc 540
   aatgtggact tagagcgcaa aagttcgccg atgttagttg tttttccagt cacggatcct 600
   atcagtggca acccgttaca gaccatccat ctgtatccga aaaatgtcgg ttatgtccgc 660
   gatccgtatt tcttcaagtt cggcgtgcac cctgatggta cgagtaaacg gttagccggt 720
   gcgatctttg ctatttaccg aattgagaat ggtaagaagc tttatctcga tatgtcgcca 780
   gtaaccgact tgcgcaacaa atgggtgagc actactgatc cgttgcatga tgaccgcgtg 840
   aacaaatttg tttccgatca agatgggcta gttaatacag gtgaacgctt tttgcccgcc 900
   ggagaatatt tctttgaaga attgcaaggc gttcccggct atgaagtgga tgctaaaagc 960
   cgcgcgatca aaatcgagat tcctgattct tgggaagacg aagatggcaa ccggcgcttt 1020
   gtgttaattg acggccagcc gatgcaggaa aactttggcg gggtggtgac accggaaatg 1080
   atcagtagcg gctacccgcg agtttataac tatgccgata agcaggcgtc gacaaccggt 1140
   gatcaaaccg cggggccatc aacgacccag cttggcaatc acgggcagga tacgaacggc 1200
   accggaacgc gtacacctaa gcgtcaatcc ggttatttgc cggccatgtc cgattggcgc 1260
   aatttacgct ttgtcctttt agggagtctg ttactactac tggccactta cttcttcatt 1320
   aaaaataaga aagcgaggca ccacgcatgc aagtaa 1356
<210> 16
   <211> 2952
   <212> DNA
   <213> Lactobacillus rhamnosus
<400> 16
   ttgcctagga aatggattca tatgctgatg ttactgctga tgctggtcac gcaaattggc     60
   agtgccgcgg tcccggtagc caaaagcgct cagactaatc caaagcacga tgtccgggat    120
   gcgtcagtgc agccgagcac tcgtcctgcc gcatccgaag ctgctgagtt tgatctggaa    180
   gcagcggcta gtgcgccatc aaccagcgcg gccgccaagc aaactacttc aaaagctcgg    240
   cagcacatca agctagaagc agggaagtct tggcacggcg atggtcatac gttaacttac    300
   aacgttgaca ttcagcggtc tgaaattcag gttaagttga ttttagccaa gccacaggat    360
   caaacggggc agcaagtcgt caagttcgct aatgcccaag gattcacgtc ccagcctgca    420
   catactaacg gtgaaataac gcgccggctt gcagagaaaa cggcagaaaa aggtgaatac    480
   cttttaacca aaaagctgcc tgatacaaag cagcaagcag ctagtgtgaa actcagtctg    540
   gatggattta atgacgctgc tcaggtatta gcgcttgatg ttgacttgca gctgcctgcg    600
   cgcctggcta acgatgatgt gcaggaaccg gctgcactca gcaaagatgc gcacagtctg    660
   attttaccgc cgtctgcact tgggacgatt aaaattcacg caactaaagc tgatggcgcc    720
   gcactttcgg atgaagaggc gcaaatttat cgcaaaccaa atagtagtac ccgttcaaaa    780
   tacggatcac gttgggcgat ggagaatggc gtttcatcag attatgtgtc gcgttctgat    840
   gccaccgcca ttatttttaa agatgcggtt caaaatccca gtggtccttc taacctgcta 900
   gatgccaaga tcaaagtgga tattgatcat gttggctcag caagtgatct tgatggaaac 960
   cgttttgaga ttggtgctta tgttgaacta acaggtattc gcgtccgtcc agtcgaatgg 1020
   ggcactaccc ctcaggatgt cggcattgat tttccaacaa acttcttttc cgggatgtca 1080
   tttgccaatg ttttgtacta tgactggcgc gtgatttttt atgacaaggc aacgagacag 1140
   cgattgaact ttattccgca aagtgaagcc aatcaaaatt cgaccctgac gtttacttcg 1200
   ttgaatcccg gtgagtttgt ctggacggag caggcgggga tgacgcccac ttatgacgat 1260
   cggtttatca cggattggca atttgaggaa ggaacctgga ttacctcaga taaagcgaca 1320
   ttcgaaaccg aaaaactggg cgctcgcggg aaggaacaac gtgggtacac ctcacagacc 1380
   tggggaaact gggtcgatcc aattgatcac gagaatatga cggaatggga agatcgacta 1440
   ggtgcgccaa catttgggcg tggtgccgtt gcgtttactt taaacggtac cagtcatacc 1500
   tttagacgcg gcacttattc caacggcggc ggtacttggg ttgccaatgg gagtggacaa 1560
   atcgagttga ttgacccaaa tgtcaccaac aacaaaagcg tgagcgcaaa tgccgaagcc 1620
   ggtggcggag ccgaggaaga taaaaccggc accatctgga ccgcaaatga tttagacgat 1680
   caggtggtca atcagcatta caacggcgag ccattttact actacatcaa ccaggaagta 1740
   tacagtatgg gcgattacgt ggtgaagccg accaaaattg ttgtgacgga cctgctaccg 1800
   gagcatgtcg agttgattcc ggacaataac aacagtcccc cgacttatca aaaagcgttc 1860
   cagctcttta atgcaactga tccggatgcg gttggccaag atcggaaaat gacgctgact 1920
   gaggacgtgt cggattttgt cgtgacgcaa gaaggcgatc ggcagcgaat cacgctgaca 1980
   atcggacgtg aagatgtgca gaaaattcat tttcatagcg gctttttctc acttcgattg 2040
   aaggtgcggc caacaaagga tccggacacc ctgacaaaac gacttacgct ggtcaataaa 2100
   gcgaccgtta aatttttcga cactgaggaa cgttacagta aggaaaccaa cgcagtgcag 2160
   gttcatcttg atccggcagg cagatttcca gctgaattta ccaagaaaaa ccagtatggc 2220
   gcagtgctgc cgggtagtcg gtttgtcttg aagcaaggag acactcaact gcaaacagca 2280
   actgccgatt cgcagggtaa agtctcattt ggaacgctaa aacccggcga ctatcaggta 2340
   agcgaaattg ccgctgccgg tcacgagttg caggctgaat ttgatttaaa agtggcagct 2400
   gacggtactg tgacagtcgg ccgcaacggc gagatttggc cagacaccac ggtgatcaac 2460
   caactgaaac ccaccgaact tgagttgatc aaaattgaaa aaggtaaaaa caaactcgcc 2520
   aatgcaagtt ttgccttata ccgtggcgat caaaccaccc ctgttgctca aggaacgact 2580
   gatgaaaatg gccagttgcg attcacacat cagttgaccc cgggaactta tcgcttaacg 2640
   gaaaccaaag cgcctgccgg atttgatcgg ctgaacggat cgtttacctt caagattaac 2700
   gcgcatggca caatggtaga tcttgcgtat agtggcagcg atttaagcag tgatgagtat 2760
   gggtttgaat ttatccctga tgcagaggat aagttgaatc ggattcgctt cacactgacg 2820
   aaccattcgt tggaaacact cctaccgaaa actggtggta gcggtatctt gctgtttctc   2880
   atggtcgcaa tcagtgcgtg tggcggcggc tggctgcttt acctgtatct gaagcgaaag   2940
   gaggcccgtt aa                                                       2952
<210> 17
   <211> 6894
   <212> DNA
   <213> Lactobacillus rhamnosus
<400> 17
   acgatggttc agtgcggatt ggactgcttg aatttcaacc agggtaactg ctctgaggga 60
   cttccctttc gggaagaatt ccctaagcag tccattggcg ttctcgtttg tgccacgctc 120
   ccaaggcgag tacggatgtg cgaagtaaat ctgggttcca acaatctctg ataacttggc 180
   aaactcggaa ccattgtcaa aagtgatact ctcaaattcc ttggccccgt agtcgtcgat 240
   cgtgtcctgc aaggctttaa ggcaggtgtc cgcatgatag tcaggaatct tgacgatgat 300
   ctcagtccgg ctgtaccgtt ctgtgagcgt cattaatgct ggctcatcag ctaagcgaat 360
   acctttgacc aagtcgcctt cccaatgtcc cacgcctgtg cggtcattca cggccgcagg 420
   acgcaactcg attgagtcgc cgtatatctt cttattcttg cgcttgtggg cgttcttata 480
   gcctttgatg cggcgtcgga gcttcttggg aagtgtcatg ttgtctagcg caagcagccc 540
   ggcgtcgatg tagcgataca cagttgtcgt tgaagggcaa gccttgccct ggtcgcgata 600
   gaagtgtacg aagctatcaa cgctgtgtac gcgcggctta cgagtaagct ccctggcgag 660
   agccttgaag aacgcacggc cggtcttaag aaaggcgtag tgaccggttc tatcgcgttt 720
   acggtcgtgc atggcttggg cagtttccgc aagatagact tgatgcgagt gacgcttcga 780
   gtcgagctga gttacagatc cacgcgtgat ttctcgtgag attgtcgctt tactgcgatg 840
   aagcttctgt gcaatcacgg tcgcggtgtc accagcagcc tgaagggcct gaattgtagc 900
   acggtcgcta aaactgagtt gttggtaatg cttgtgggtg ttagtctgag agtgggtcat 960
   gaagattcct gctttcttgt ttagctagca ctaacaagaa taggtcttca tggcctttat 1020
   ggtctagtcg tcagggtgtt gcacttgaat tgtaaactgg ggttaaaatt ttacttgaga 1080
   ggagggtaaa atttacgaag atgacagcta aagtggcgag aactgggcat ttgttcgcgg 1140
   tcttattgat tttgatgagt atgttaacag gcttagtgac aagtggcagt tcagttgtga 1200
   cagccactga taacattcgc ccaacctatc aaaccgatgc taatggtacc tatccgacaa 1260
   attcgtggca ggtcacggga caacaaaatg taatcaatca acgtggcggg gatcaagttt 1320
   cagggtggga taataataca atatggaatg gtgatgcgac tgataccacg aactcttacc 1380
   tgaaatttgg tgaccccaat aatccggatt atcagattcg aaaatatgct aaagagacga 1440
   atacccctgg attgtacgac gtttatttga acgtcaaagg caataaacag caaaatgtga 1500
   agcctgtaga tattgtctta gttgttgata tgtctgggtc aatggagtca aacagatggg 1560
   gcacgaatcg agctggtgct gttcgtactg gcgttaagaa tttcttgact tctattcaaa 1620
   acgccggtct gggtaattac gtcaatgttg gtttaattgg gttttctagt cctggttata 1680
   tcggtggcaa atcgggttat attagtgtca aattaggcaa agcaggtaat gccagccagc 1740
   aacaagcgat taatggtgca ttgagtccaa ggtttcaagg gggtacgtat acgcagattg 1800
   gtttgcggca aggatcagcc atgctgaatg cggacaccag tggcaataaa aaaatgatga 1860
   ttttgttaac tgatggcgtg ccgacttttt ctaacgaggt gataaattca gagtggataa 1920
   atggtacatt gtatggcact aattttggat ccagcagaga tgaaccaggg aacaccgcac 1980
   gacttcgatg gccatacacc gatagttcag gtcattatat atatgatact tggccagcaa 2040
   cattgggtga ggccaagata gcaaaggata gtggtaatga ggtgcacgcg ttaggcatcc 2100
   aactggctga cgacgaccac tacatgacga aagaaaaaat acgccaaaac atgcagctta 2160
   ttaccaattc accggattta tacgaagatg ctgatagtgc cgatgctgtt gaggcttatt 2220
   tgaacaatca ggcaaaggac attatcaaaa actttaatac tgtcaccgac ggcacgatca 2280
   cagacccgat tggtacgcaa tttcaatatg cgaacaacca ggcgaccgtt acgagtgtcg 2340
   gcaagcaaac tgtgccagca agtgagttgc caagtgcggc gatccaagat ggtcaattga 2400
   cggtgaatca catgaacttg ggtcaggatc aggaagttca aatccattat caagtacgga 2460
   tcaaaacaga ggatgctggc ttcaagcctg atttttggta ccaaatgaat ggtgaaacat 2520
   tgttgacacc aaaagcgggc gctgccgctg ttgactttgg gattccttca ggcagggcac 2580
   cagcaactac agtttatgtg cagaagcaat ggcgccagtt aagcaatcaa tcgttaccgg 2640
   atacgctcaa cgtcacggtg cagcgaaaag tggctgacgg ttcgcttgat ccaaattggc 2700
   aacagacctt agtccttaaa aaagctgata actggaaagc tagctttacg gcacctgcgt 2760
   ataacaatca gggtcaaagt ttttcatatg tcgttaagag tgaagatgcc tcgggaattg 2820
   atttgagttc gtttatcagt tctcaaaata tggatcagca aacagcaacg ttgactttga 2880
   caaatcagca gtatggtttt cagtttcaga aaaaaacaac cgatggtact gatttatcag 2940
   cagatcagtt gaaggccatg cagtttaact taacccagta cagcgataac agttttcagc 3000
   aggcatccaa aaccaacgcc atcacgtcaa cggatctgca ggcactagcg ccagggtatt 3060
   acggtattca ggaagctgca gcacctacag gttatcaact tgatgggaca acgtatcttt 3120
   ttcagctaac gtctgatggg caatggcaat accatggcac aaaggacaat gtgacatcag 3180
   ggagtgttat taatggccag cagactttga atcctgttgg tgataagtca gatgatttta 3240
   cggtgaccgg ggatcaccag caaattctga cgctaacgaa atatgatgaa ccaaagccat 3300
   ccatgacttt gcgggtcatc aaacaggata atcaaagcca atatcttgca ggtgcagcgt 3360
   tcaccctgca accaagtgct ggcgaagctg agacgataac atcatcggcg acatctgagg 3420
   gacaagcgtt tgcgacaaaa ttagttgcag atggtaccta tacgatgtca gaaacaaaag 3480
   caccagatgg ctatcaaagc aatcctgcaa agattgccat tcaggtagct acgactggta 3540
   aagaggcaac cgtcacgatt gacggtgagg cattgaagcc gggcgaaagt aagaacggat 3600
   acacattagc gattgatggc agcacgatca ctttgcaggc gattaatcag ccacttgcaa 3660
   ttttgccgca tacaggtggt cagggctatc agcgattgct tggtatcgca ctgggattga 3720
   tcagcgcagc gttcctttta ttactggttg ttttgataaa gcgacgggtg gtgaagcaac 3780
   atgactaaat ccttccgtcc gttagtgatt ttgacctttt gcttggcact actagtcagt 3840
   ttggcaacga caacgttgca gcagacacag gcggcaactg tgccgaccac tgttgatgtt 3900
   gtgttgcata agctgttgtt taaagatacc ttgccaactc aacaagcaaa taacgggaca 3960
   acaaaacccg acttttcgca ggcagatgtg ccgttaaacg gtgtgacgtt cacagtttat 4020
   gacgtgaccg ctgacttttg gcagcttgtc tccaaaaatg gcggtgcgat tgaggtagca 4080
   caaacgacgt tgagtcaaga tagctatcag cctgctagct ccagccttat cgcacaggtt 4140
   gtgacggctg gtcagggaga agcgtacttt ggcgatttac cactccgaca ggggcagcat 4200
   gctgcggttt atctttttaa agaaacggcg gcacctaaga atattgaagc cagtcagaat 4260
   cttgtggttg tcatgtcaag caaccttcaa catgggaatc aatcacgcat tgatttattt 4320
   cctaagaaca aaatggtaag tcgtcacacc gatgccccca aaaaagttcc aaagaaaata 4380
   cgtcaattgt tgccacaaac gggtgataca gttgcagctt ggctttcagt gctcgggttg 4440
   ataatcttcg cgacagtact tgcttttaac ataaaaaacc aaaaaattaa taagtgggag 4500
   agataagaat gaaaaagaca attgccaaga aagtgctgac attaaccagc acgatcctaa 4560
   tgacattact gatggttctc gggtttaatg gcactcgggt tcaagcagat acgaatgata 4620
   cgacaacaca aaacgttgtc cttactaaat acgggtttga caaagatgtt actgccattg 4680
   atcgtgcgac tgatcaaatt tggaccggcg atggtgctaa gcctttacaa ggcgttgatt 4740
   tcaccattta caacgtgaca gccaattatt gggcatcgcc taaggattat aaaggcagtt 4800
   ttgatagtgc tccggttgcc gcaaccggta cgactaatga caaggggcaa ctaacccaag 4860
   cattacctat ccaatcaaaa gatgccagtg gtaagactcg tgctgctgtc tatcttttcc 4920
   atgaaaccaa tccgcgagct ggttataaca cgtctgccga tttctggtta accttaccag 4980
   ccaaggcagc agccgacggg aatgtctatg tctacccaaa gaatgttcaa aagaccacct 5040
   atgagcgcac ttttgttaag aaagatgctg agactaaaga agtgcttgaa ggagccggct 5100
   ttaagattag caatagtgat ggcaagtttt tgaagttgac agataaagat ggtcaaagcg 5160
   tcagcatcgg cgaaggattt atcgatgtat tggccaataa ctatcgattg acgtgggttg 5220
   ctgaaagcga tgctactgtt ttcacgtctg ataagagcgg taagtttggc ttaaatggat 5280
   ttgctgataa caccacaact tacacggcag ttgaaacaaa cgtgccggat ggttatgatg 5340
   ctgctgccaa tacagacttt aaagctgata attcgtctag cgacattcta gatgcaccaa 5400
   gcggtattct gccacacact ggtggtactg gcacagtcat tttgcgattt ttgggcgttg 5460
   ccttaattgc atttggagca gttgcctatc gcaagcgccg caatggtttc taaaaagtta   5520
   ataagataaa tgagtcaagc aagagcgtcg atggcgctct tgttttgata tggcgaggta   5580
   atcagagtga caaaacgaac acgtcgacct ttagacttga ttgatattgt gattggatgt   5640
   cttcttttag cgggttttgg tgttttatgc tatccatttg caagtgatgc ttacgtttct   5700
   taccaaaatc agcaagtcat cgacaggtat cgacaacaag aagcgcggaa gaatcagatg   5760
   gtgttgcggc gggaatataa cgactatcag caaaaaaata aacagttggc agcaagtcaa   5820
   caagtgcccg gcgttgccag ttttaatcat gctgttaatg atcaaggaac cgcaaaaaca   5880
   gcagccaaac gcaatcaaca aatcttgact cggcagacag ttgctcagtt gacgattccc   5940
   aaaattggcc ttagtctgcc ggtttttgat catacaagcg attggcttct acaatttggc   6000
   gcctgtttat tggatggtac aagttatcca actggtggta aaaataccca tgctgtcatt   6060
   tcagcgcatc gtggtgtgcc aaacgctgaa ctttttaccc gagtaccagc gttaaaaaaa   6120
   ggcgacaagt tttttattag cataggcaat cataaattgg cttaccaagt ctttaagcgc   6180
   caggttattg agccaagtga tacccggcag ctaagaattg tgccgggaca ggatcttgtg   6240
   accttaatga cctgcacgcc ttatatgatc aattctcatc gattgttgat aacgggtcgc   6300
   cgaattcctt acgttaaggc agatgaagag gcttcaagtt gggcggtttg gtggaacaaa   6360
   ttaaagctaa tagtcgcact tttaggcgcg gtgatcattt taggcgtgat cggtttcgta   6420
   atgcgcagtt tgatgcttgg ccgaaagcat tatttgctgg aagtaccggc tgaagccaca   6480
   caagtcgtgg tgaaacgagg tcgacatata cattctttta aatcagatca aactggggtg   6540
   actgacatca gcctgcctgg taatcattat cgagtcgcaa ttgtcacacc gcttggccgg   6600
   actaagtaca aggcttatgt caaaaaaatt cgggataaaa gctttcaatt aaaagaatat   6660
   cattaagatc ttaaaatttg tttaatatcc ttttgggtta atttaaatga ggaaaggatc   6720
   aatattttaa gactgatatt gagtttaata aactaaaaac gaccaactta ttaaaacact   6780
   atgtctgtat atttcaagct tttgaagtag gacgatgcaa catgaatgag tattagaaaa   6840
   ccggttcatc aaagatgact ttccatagtg gaagccgtct tttttgatat ttaa         6894
<210> 18
   <211> 7540
   <212> DNA
   <213> Lactobacillus rhamnosus
<400> 18
   gcccatggta ctgccgagtt cactgttgac ggggcgcagt tgcgtattca tattgagatg    60
   tttgatacac ctgcaaacgt ccagcattgg gaacacttcc atggttttcc ggatggcaag   120
   ccagctgaga tagccaccgc ggcccaagat gctaatggtg acggctttgt tgatttacct   180
   gaaacggaac cggtttccgg tacaacgatg gtcccgtttg atgccgagcc tgctaaaatg   240
   cacgtaccca atgacagcta tccagtagcc gatgctgacg ggcactatgc ttatgacaag   300
   ctcgtggatt taaaagagct gcagaaggcg ttcaaggcag catttggtag tgaagatttg 360
   caattagata aacgggttgt ttacattcat ggtgttccgg acagtttgga acttccagat 420
   acggttaaag gaaccgtcat gaactatgat gcacacgtca cactgccaat tgccgtgggt 480
   aaaattgtcc gcgcttagta gcaaaatata atttaataac gtaggttgtt cccgatccgt 540
   ctgagcgttt ccagcttagt caggtcggga ttttttgtgc gccaaatcct aaaacttata 600
   aatactggaa tgatcattca tatattcgct gctaattata atttaagaag taatataccg 660
   aagaaaactt tatttataaa ataacaatta ttatagttcc gtttacgcag ttcatcttgt 720
   tacgcttaat tcagcaacaa gtgaaaaatg taacatgaaa ggaggcaccc gattgcctag 780
   gaaatggatt catatgctga tgttactgct gatgctggtc acgcaaattg gcagtgccgc 840
   ggtcccggta gccaaaagcg ctcagactaa tccaaagcac gatgtccggg atgcgtcagt 900
   gcagccgagc actcgtcctg ccgcatccga agctgctgag tttgatctgg aagcagcggc 960
   tagtgcgcca tcaaccagcg cggccgccaa gcaaactact tcaaaagctc ggcagcacat 1020
   caagctagaa gcagggaagt cttggcacgg cgatggtcat acgttaactt acaacgttga 1080
   cattcagcgg tctgaaattc aggttaagtt gattttagcc aagccacagg atcaaacggg 1140
   gcagcaagtc gtcaagttcg ctaatgccca aggattcacg tcccagcctg cacatactaa 1200
   cggtgaaata acgcgccggc ttgcagagaa aacggcagaa aaaggtgaat accttttaac 1260
   caaaaagctg cctgatacaa agcagcaagc agctagtgtg aaactcagtc tggatggatt 1320
   taatgacgct gctcaggtat tagcgcttga tgttgacttg cagctgcctg cgcgcctggc 1380
   taacgatgat gtgcaggaac cggctgcact cagcaaagat gcgcacagtc tgattttacc 1440
   gccgtctgca cttgggacga ttaaaattca cgcaactaaa gctgatggcg ccgcactttc 1500
   ggatgaagag gcgcaaattt atcgcaaacc aaatagtagt acccgttcaa aatacggatc 1560
   acgttgggcg atggagaatg gcgtttcatc agattatgtg tcgcgttctg atgccaccgc 1620
   cattattttt aaagatgcgg ttcaaaatcc cagtggtcct tctaacctgc tagatgccaa 1680
   gatcaaagtg gatattgatc atgttggctc agcaagtgat cttgatggaa accgttttga 1740
   gattggtgct tatgttgaac taacaggtat tcgcgtccgt ccagtcgaat ggggcactac 1800
   ccctcaggat gtcggcattg atttttccaa caacttcttt tccgggatgt catttgccaa 1860
   tgttttgtac tatgactggc gcgtgatttt ttatgacaag gcaacgagac agcgattgaa 1920
   ctttattccg caaagtgaag ccaatcaaaa ttcgaccctg acgtttactt cgttgaatcc 1980
   cggtgagttt gtctggacgg agcaggcggg gatgacgccc acttatgacg atcggtttat 2040
   cacggattgg caatttgagg aaggaacctg gattacctca gataaagcga cattcgaaac 2100
   cgaaaaactg ggcgctcgcg ggaaggaaca acgtgggtac acctcacaga cctggggaaa 2160
   ctgggtcgat ccaattgatc acgagaatat gacggaatgg gaagatcgac taggtgcgcc 2220
   aacatttggg cgtggtgccg ttgcgtttac tttaaacggt accagtcata cctttagacg   2280
   cggcacttat tccaacggcg gcggtacttg ggttgccaat gggagtggac aaatcgagtt   2340
   gattgaccca aatgtcacca acaacaaaag cgtgagcgca aatgccgaag ccggtggcgg   2400
   agccgaggaa gataaaaccg gcaccatctg gaccgcaaat gatttagacg atcaggtggt   2460
   caatcagcat tacaacggcg agccatttta ctactacatc aaccaggaag tatacagtat   2520
   gggcgattac gtggtgaagc cgaccaaaat tgttgtgacg gacctgctac cggagcatgt   2580
   cgagttgatt ccggacaata acaacagtcc cccgacttat caaaaagcgt tccagctctt   2640
   taatgcaact gatccggatg cggttggcca agatcggaaa atgacgctga ctgaggacgt   2700
   gtcggatttt gtcgtgacgc aagaaggcga tcggcagcga atcacgctga caatcggacg   2760
   tgaagatgtg cagaaaattc attttcatag cggctttttc tcacttcgat tgaaggtgcg   2820
   gccaacaaag gatccggaca ccctgacaaa acgacttacg ctggtcaata aagcgaccgt   2880
   taaatttttc gacactgagg aacgttacag taaggaaacc aacgcagtgc aggttcatct   2940
   tgatccggca ggcagatttc cagctgaatt taccaagaaa aaccagtatg gcgcagtgct   3000
   gccgggtagt cggtttgtct tgaagcaagg agacactcaa ctgcaaacag caactgccga   3060
   ttcgcagggt aaagtctcat ttggaacgct aaaacccggc gactatcagg taagcgaaat   3120
   tgccgctgcc ggtcacgagt tgcaggctga atttgattta aaagtggcag ctgacggtac   3180
   tgtgacagtc ggccgcaacg gcgagatttg gccagacacc acggtgatca accaactgaa   3240
   acccaccgaa cttgagttga tcaaaattga aaaaggtaaa aacaaactcg ccaatgcaag   3300
   ttttgcctta taccgtggcg atcaaaccac ccctgttgct caaggaacga ctgatgaaaa   3360
   tggccagttg cgattcacac atcagttgac cccgggaact tatcgcttaa cggaaaccaa   3420
   agcgcctgcc ggatttgatc ggctgaacgg atcgtttacc ttcaagatta acgcgcatgg   3480
   cacaatggta gatcttgcgt atagtggcag cgatttaagc agtgatgagt atgggtttga   3540
   atttatccct gatgcagagg ataagttgaa tcggattcgc ttcacactga cgaaccattc   3600
   gttggaaaca ctcctaccga aaactggtgg tagcggtatc ttgctgtttc tcatggtcgc   3660
   aatcagtgcg tgtggcggcg gctggctgct ttacctgtat ctgaagcgaa aggaggcccg   3720
   ttaagatgcg acgattttat tggtggcttg tcccgttgct tctattgatt ggtatcgtgc   3780
   ttggcaacac accacattgg gttcacgcgg ctgatcaaac tgccgagatt gtgatccata   3840
   agcgaattta tcgggatatt cgccaaccgg aagacgtttg gtatgaaaat gacggtcatc   3900
   ggattgaccc gaataacccg gataaagatg gctacaaatt attaagcaaa accagcgggc   3960
   tgaatggtgc taactttgag gtctatgatg ccagctcctt attgaaaccg aatatgacgc   4020
   ctgaagcaat tcgggcttta gttgatcgtt atcagaatat gacgcgtaag caagcactga   4080
   aatttgcgcg ggccaacctg aaattagccg gtcaagggaa caaaggtatc gggctgatga   4140
   atacaaaaaa cgatccaaca ctcggtgaag atgggatcag ccgaataacc gtttctgtcg   4200
   atcaacaggc accgactaaa gcttatctga tgatcgaggt ggcaccggat ccttcaaccg 4260
   aactcaatgt ggacttagag cgcaaaagtt cgccgatgtt agttgttttt ccagtcacgg 4320
   atcctatcag tggcaacccg ttacagacca tccatctgta tccgaaaaat gtcggttatg 4380
   tccgcgatcc gtatttcttc aagttcggcg tgcaccctga tggtacgagt aaacggttag 4440
   ccggtgcgat ctttgctatt taccgaattg agaatggtaa gaagctttat ctcgatatgt 4500
   cgccagtaac cgacttgcgc aacaaatggg tgagcactac tgatccgttg catgatgacc 4560
   gcgtgaacaa atttgtttcc gatcaagatg ggctagttaa tacaggtgaa cgctttttgc 4620
   ccgccggaga atatttcttt gaagaattgc aaggcgttcc cggctatgaa gtggatgcta 4680
   aaagccgcgc gatcaaaatc gagattcctg attcttggga agacgaagat ggcaaccggc 4740
   gctttgtgtt aattgacggc cagccgatgc aggaaaactt tggcggggtg gtgacaccgg 4800
   aaatgatcag tagcggctac ccgcgagttt ataactatgc cgataagcag gcgtcgacaa 4860
   ccggtgatca aaccgcgggg ccatcaacga cccagcttgg caatcacggg caggatacga 4920
   acggcaccgg aacgcgtaca cctaagcgtc aatccggtta tttgccggcc atgtccgatt 4980
   ggcgcaattt acgctttgtc cttttaggga gtctgttact actactggcc acttacttct 5040
   tcattaaaaa taagaaagcg aggcaccacg catgcaagta acgtttaaaa agatcgggca 5100
   cagtctcttg gcagcgctga tgctcatgag cttccttcta ccactgctta gtgcgggcaa 5160
   acccgtacat gccgcgacaa cgactgtgga tttcacgctg cacaaaatcg aacaaaccag 5220
   tgacgaacag attcaaaata ccggccacga ccttggactg accgggcgta aaccggtgca 5280
   aggcgctcaa tttaaaattt tcaacgtgac ggacgccttt taccaattac tggaaaatca 5340
   tgataagaca accgctgcga gcatgatatc gcaaaacctg ggtcagtatg tgaatctcca 5400
   ggatcctaat gcagcaactg tcacgactga tgcagacggc ttggcggcat tcaaaggatt 5460
   agccgccaaa accaatggcc ggcatagcgt gtacgcattt cacgaagccg tgaccccgca 5520
   accgtatcaa aaagcagcag atatgatcgt gagtctgcca gtgcggcaag acgatggatc 5580
   ggatctgacc aacattcatc tttatcctaa agacagtctt gttaccaaaa atctgacgga 5640
   aatcaatgaa caagcggtgg caacaaaaga tctccatgat gtcgcggttg gcgatgtgct 5700
   cacgtatcag gttcagttcc agattccgca tgatattggc gcgctggctg atcacagtca 5760
   agacactttt aagtacaacc aatttaaagt gctggattat atgaccaagg aaggccttac 5820
   ttttaaggca ttgacggcaa tcacggttga cggtcaggac attttaaagg cattaaccgg 5880
   aaaaatggcc ttcatgagtt ctaatgacgc agcttggcaa caaacacaca actatccatt 5940
   cgggtttgaa ctggactttc taggcgggac cgatcccgat gcggtacgaa acctgttgac 6000
   ccaatatgcc ggcaaacgcg tgaccgttgc ctacaccgga atcgtcaatg agaaaatgat 6060
   cccagaccaa aaagtcggta acacggctga agtgagcttt gatcctgaca gcaagattac 6120
   cgtcaatggt ccggaaatcc agactggcgg gattcggttc ttcaaacacg aagccggatc 6180
   ttccaaaagt ttggccaacg cgactttcat cttacagcga atgaacggca atgtgcgcga 6240
   atatgcagtt cttgaaggcg ttaacggtat ggccggaacc taccaaccga ccaagattac 6300
   ctggacaacg aatcaagacg cggcaacgag actcaaaacc agtggagccg agacagccaa 6360
   cttaaccatt caagggctgt tgccagggcg atataccttg gttgaaaccg cggcaccaga 6420
   aggctatgaa atccttgatc cgacaacaga ttttgaagtc attgccggta cttggggtac 6480
   gaaaacgatt cgcatcgcca acacgccggt gaatcaatta ttgccgatga caggcggaat 6540
   cggactcttc gccttcctga tgatcggggc catcttaatg ggtggcggtc acctaatgaa 6600
   gaaaaagacc agcaagaaag tctaatggcc tatgacaaaa aaagcgtcgg ggacaagtcg 6660
   gctgttacgc tggttcgtca tcttactttt tactgcggga gccgcgtgtt tctgctatcc 6720
   gttcgcggca acggctatta atgaattgct actaaccagt cgccgagcag cagcacagca 6780
   agaagccaag caaaatgccg ccgcccaaga tgagcaacgg gcagcggaga accgtgcact 6840
   tgcccagact ggtttgcgtc cgggacagga tccgtttcaa agtaggcaga aatttaacca 6900
   agcctatgtg aaacggcatc tgatcgggcg agtggttatc ccgaaattag cggttgatct 6960
   gccccttttt gacaccacca acaacacgct gttagatcaa ggggcagtgg tgttaccagg 7020
   tactagctat ccgcggggag gcaagaacac gcatacagtt gtttcggcac acggcggctt 7080
   gcccaccaaa cgctttttca ccgatctgag caagttgaaa cgagggcaga agttctttct 7140
   ccaagtcaac ggcaaaaaga tggcgtatca ggtctttcgg atcaaaaccg tgcggccgga 7200
   tgaaacccag agcttgcgca ttgaaccggg acgcgatttg gccacattaa tgacctgtac 7260
   cccgtatatg atcaactccc accgcctgtt agtgaccggc aaacgggtac cttataccga 7320
   atcacttgag cacgccgccg agtctgctga tcgctggcgc ttgtggttaa gtatcgcggt 7380
   tgtcgtcgga gtgctgggat tggcattgct gagtttctat ctggctcggc gctatcttcg 7440
   ccgaccgcgg gcgtaatctg gaaagagaat gttagaaagt aagaaagttc gccgttgtgc 7500
   agggataggt ctgtggacgg cgggcttttt tgtgtttcga 7540
<210> 19
   <211> 30
   <212> DNA
   <213> Artificial sequence
<220>
   <223> Oligonucleotide
<400> 19
   tcgggttcag aattctacga atgatacgac 30
<210> 20
   <211> 30
   <212> DNA
   <213> Artificial sequence
<220>
   <223> Oligonucleotide
<400> 20
   tgccagtacc accctcgagt ggcagaatac 30
<210> 21
   <211> 28
   <212> DNA
   <213> Artificial sequence
<220>
   <223> Oligonucleotide
<400> 21
   gcagacacag aattcaactg tgccgacc 28
<210> 22
   <211> 34
   <212> DNA
   <213> Artificial sequence
<220>
   <223> Oligonucleotide
<400> 22
   caactgtatc accctcgagt ggcaacaatt gacg 34
<210> 23
   <211> 33
   <212> DNA
   <213> Artificial sequence
<220>
   <223> Oligonucleotide
<400> 23
   cagttcagtt gtgaattcca ctgataacat tcg 33
<210> 24
   <211> 31
   <212> DNA
   <213> Artificial sequence
<220>
   <223> Oligonucleotide
<400> 24
   agccctgacc accctcgagc ggcaaaattg c 31
<210> 25
   <211> 28
   <212> DNA
   <213> Artificial sequence
<220>
   <223> Oligonucleotide
<400> 25
   acccgtacag aattcgacaa cgactgtg 28
<210> 26
   <211> 31
   <212> DNA
   <213> Artificial sequence
<220>
   <223> Oligonucleotide
<400> 26
   gtccgattcc gccctcgagc ggcaataatt g 31
<210> 27
   <211> 31
   <212> DNA
   <213> Artificial sequence
<220>
   <223> Oligonucleotide
<400> 27
   ccacattggg ttcagaattc tgatcaaact g 31
<210> 28
   <211> 30
   <212> DNA
   <213> Artificial sequence
<220>
   <223> Oligonucleotide
<400> 28
   tgcgccaatc ggactcgagc ggcaaataac 30
<210> 29
   <211> 29
   <212> DNA
   <213> Artificial sequence
<220>
   <223> Oligonucleotide
<400> 29
   gcaaattggc aggagctcgg tcccggtag 29
<210> 30
   <211> 28
   <212> DNA
   <213> Artificial sequence
<220>
   <223> Oligonucleotide
<400> 30
   ccgctaccac cctcgagcgg taggagtg 28
<210> 31
   <211> 20
   <212> DNA
   <213> Artificial sequence
<220>
   <223> Oligonucleotide
<400> 31
   tctcgggttt aatggcactc 20
<210> 32
   <211> 20
   <212> DNA
   <213> Artificial sequence
<220>
   <223> Oligonucleotide
<400> 32
   tctgtattgg cagcagcatc 20
<210> 33
   <211> 20
   <212> DNA
   <213> Artificial sequence
<220>
   <223> Oligonucleotide
<400> 33
   tccttccgtc cgttagtgat 20
<210> 34
   <211> 20
   <212> DNA
   <213> Artificial sequence
<220>
   <223> Oligonucleotide
<400> 34
   cgtttgtggc aacaattgac 20
<210> 35
   <211> 20
   <212> DNA
   <213> Artificial sequence
<220>
   <223> Oligonucleotide
<400> 35
   ccaaattggc aacagacctt 20
<210> 36
   <211> 20
   <212> DNA
   <213> Artificial sequence
<220>
   <223> Oligonucleotide
<400> 36
   gccatctggt gcttttgttt 20
<210> 37
   <211> 20
   <212> DNA
   <213> Artificial sequence
<220>
   <223> Oligonucleotide
<400> 37
   cggacgcctt ttaccaatta 20
<210> 38
   <211> 20
   <212> DNA
   <213> Artificial sequence
<220>
   <223> Oligonucleotide
<400> 38
   aacaggtttc gtaccgcatc 20
<210> 39
   <211> 20
   <212> DNA
   <213> Artificial sequence
<220>
   <223> Oligonucleotide
<400> 39
   tatgacgcgt aagcaagcac 20
<210> 40
   <211> 20
   <212> DNA
   <213> Artificial sequence
<220>
   <223> Oligonucleotide
<400> 40
   tggccgtcaa ttaacacaaa 20
<210> 41
   <211> 20
   <212> DNA
   <213> Artificial sequence
<220>
   <223> Oligonucleotide
<400> 41
   ctaccggagc atgtcgagtt 20
<210> 42
   <211> 20
   <212> DNA
   <213> Artificial sequence
<220>
   <223> Oligonucleotide
<400> 42
   ggccattttc atcagtcgtt 20